(19) Europäisches Patentamt — European Patent Office — Office européen des brevets

(11) **EP 3 549 947 B1**

(12) **EUROPEAN PATENT SPECIFICATION**

(45) Date of publication and mention of the grant of the patent:
**08.05.2024 Bulletin 2024/19**

(21) Application number: **18000319.6**

(22) Date of filing: **04.04.2018**

(51) International Patent Classification (IPC):
**C07K 7/06** (2006.01)    **C07K 14/78** (2006.01)
**C07K 7/02** (2006.01)

(52) Cooperative Patent Classification (CPC):
**C07K 7/06; C07K 7/02; C07K 14/78;**
C07K 14/70546

(54) **POLYRGD PEPTIDES OBTAINED BY CHEMICAL AND BIOTECHNOLOGY METHODS WITH INJURY REDUCING AND/OR NEUROPROTECTIVE EFFECTS AND A KIT FOR THEIR ACTIVITY EVALUATION**

DURCH CHEMISCHE UND BIOTECHNOLOGIEVERFAHREN ERHALTENE POLY(RGD)PEPTIDE MIT VERLETZUNGSREDUZIERENDEN UND/ODER NEUROPROTEKTIVEN EFFEKTEN UND KIT ZU DEREN AKTIVITÄTSAUSWERTUNG

PEPTIDES POLYRGD OBTENUS PAR PROCÉDÉS CHIMIQUES ET BIOTECHNOLOGIQUES AVEC RÉDUCTION DE LÉSIONS ET/OU DES EFFETS NEUROPROTECTEURS ET KIT D'ÉVALUATION DE LEUR ACTIVITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**09.10.2019 Bulletin 2019/41**

(73) Proprietors:
- **Uniwersytet Gdanski**
  **80-309 Gdansk (PL)**
- **Politechnika Gdanska**
  **80-233 Gdansk (PL)**
- **Gdanski Uniwersytet Medyczny**
  **80-210 Gdansk (PL)**
- **Instytut Biologii Doswiadczalnej im. Marcelego Nenckiego Polskiej Akademii Nauk**
  **02-093 Warszawa (PL)**
- **Skowron, Piotr**
  **Gdansk (PL)**
- **Janus, Lukasz**
  **Poznan (PL)**
- **PRO SCIENCE Polska sp. z o.o.**
  **80-302 Gdansk (PL)**

(72) Inventors:
- **Czupryn, Artur**
  **02-747 Warszawa (PL)**
- **Mucha, Piotr**
  **83 - 110 Tczew (PL)**

- **Skowron, Piotr**
  **80-303 Gdansk (PL)**
- **Mazuryk, Jaroslaw**
  **56-200 Góra (PL)**
- **Wisniewska, Marta**
  **03-948 Warszawa (PL)**
- **Kozinski, Kamil**
  **02-390 Warszawa (PL)**
- **Beresewicz, Malgorzata**
  **00-380 Warszawa (PL)**
- **Krupska, Olga**
  **20-130 Lublin (PL)**
- **Zaluska, Izabela**
  **82-300 Elblag (PL)**
- **Palczewska - Groves, Malgorzata**
  **80-358 Gdansk (PL)**
- **Zylicz - Stachula, Agnieszka**
  **80-306 Gdansk (PL)**
- **Zebrowska, Joanna**
  **84-230 Rumia (PL)**
- **Piotrowski, Arkadiusz**
  **83-031 Rózyny (PL)**
- **Pikula, Michal**
  **80-170 Gdansk (PL)**
- **Sachadyn, Pawel**
  **81-646 Gdynia (PL)**
- **Janus, Lukasz**
  **60-694 Poznan (PL)**
- **Rodziewicz-Motowidlo, Sylwia**
  **83-010 Rotmanka (PL)**

(74) Representative: **Pawlowska-Bajerska, Justyna et al**
**Kancelaria Patentowa Justyna Pawlowska**
**Wzgorze Bernardowo 263B/6**
**81-583 Gdynia (PL)**

(56) References cited:
**US-B1- 6 291 428**

- **Murata ET AL: "Molecular properties of poly(RGD) and its binding capacities to metastatic melanoma cells.", , 1 January 1991 (1991-01-01), pages 212-217, XP055560873, Retrieved from the Internet: URL:http://www.ncbi.nlm.nih.gov/pubmed/176 1368 [retrieved on 2019-02-22]**
- **NIKOLAOS BIRIS ET AL: "The Ac-RGD-NH2 peptide as a probe of slow conformational exchange of short linear peptides in DMSO", BIOPOLYMERS, vol. 69, no. 1, 1 May 2003 (2003-05-01), pages 72-86, XP055561006, US ISSN: 0006-3525, DOI: 10.1002/bip.10335**
- **C.-Y. LIN ET AL: "Integrin regulation of cytoplasmic calcium in excitatory neurons depends upon glutamate receptors and release from intracellular stores", MOLECULAR AND CELLULAR NEUROSCIENCES., vol. 37, no. 4, 1 April 2008 (2008-04-01), pages 770-780, XP055561013, US ISSN: 1044-7431, DOI: 10.1016/j.mcn.2008.01.001**
- **HUGO PELUFFO ET AL: "RGD domains neuroprotect the immature brain by a glial-dependent mechanism", ANNALS OF NEUROLOGY., vol. 62, no. 3, 1 September 2007 (2007-09-01), pages 251-261, XP055561173, BOSTON, US ISSN: 0364-5134, DOI: 10.1002/ana.21170**
- **YUNPENG YE ET AL: "Design, Synthesis, and Evaluation of Near Infrared Fluorescent Multimeric RGD Peptides for Targeting Tumors", JOURNAL OF MEDICINAL CHEMISTRY, vol. 49, no. 7, 1 April 2006 (2006-04-01), pages 2268-2275, XP055612962, US ISSN: 0022-2623, DOI: 10.1021/jm050947h**

**Description**

The subject of the invention is new peptidse derivatives of platelet derived growth factor, for use of these compounds as a therapeutic agent for the regeneration and/or treatment of neurological wounds caused by mechanical, chemical, thermal and/or radiative injuries, surgical operations and/or other pathological conditions.

Neurological injuries include all types of traumatic and rapid pathological changes within the nervous system and associated neurovascular system. Injuries within the central nervous system (CNS) i.e. in the brain and a spinal cord are exceptional life- and health-threatening. In these cases, an injury causes structural changes, dysfunctions of electrochemical signaling, cellular death (affecting not only neurons, but also all types of glial cells), resulting in physiological, cognitive changes as well as in these regarding to feelings, emotions, and memory. Such damages, so-called traumas, are mostly consequences of mechanical injuries (wounds) and ischemic or hemorrhagic strokes that in majority of cases lead to chronic or irreversible encephalopathy. A vast number of the cases lead to the patient's death for direct reasons of the damage or secondary pathological processes that are still beyond of modern medicine. In the CNS, mechanical (physical) injuries are the most common clinical cases of damages of the brain injury and the spinal cord injury. They include traumatic brain injury (compression injuries) as a result of a heavy impact or mechanical brain injury (wounds) with a direct appearance of deep brain wounds. Both types of injuries occur as consequences of traffic accidents, falls, and other damages leading to concussion and physical defects and finally to the death or to transient or chronic epileptic seizures, migraines, or malfunctions of specific brain regions. In turn, the spinal cord injury, as an effect of backbone fracture (partial or complete transsection of the spinal cord), or crush (compression damage), leads to temporary or permanent disability of muscle functions, loss of sensation or autonomic responsiveness of those body parts that are controlled by nerves below a site of the spinal cord lesion.

Strokes (brain hypoxia) are the other group of the neurological injuries, and they generally consist of two types of dysfunctions: ischemic stroke, as a result of the blood vessels blockage in the brain, and hemorrhagic stroke, as a result of disintegration of blood vessel walls within the brain. The former type includes global and local (focal) strokes. Whereas both subtypes of the ischemic stroke concern lack of nutrients delivered to neurons and glial cell by the blood, however both types significantly differ with reason of the vascular dysfunction. *The global ischemia* is caused by the systemic hypoxia and circulatory arrest resulting often by loss of consciousness, whereas *the local ischemia* is an effect of blood vessel blockage by a blood clot or atherosclerotic deposits (atherosclerosis). Clinical symptom of this case displays tissue necrosis in the vicinity of the ischemic site, in the stroke center (ischemic core) and in the penumbra that is a site in which blood reperfusion is possible, if a medical treatment is applied shortly after the stroke. Brain hypoxia can also be induced in infants by improper childbirth. Brain hemorrhage, on the other hand, is an effect of the rapid vessel breaking usually originating from the global blood hypertension or blood leakage from aneurysm, and leads to a decrease of global blood tension and to damages of parenchyma/brain tissue within the affected region of the CNS.

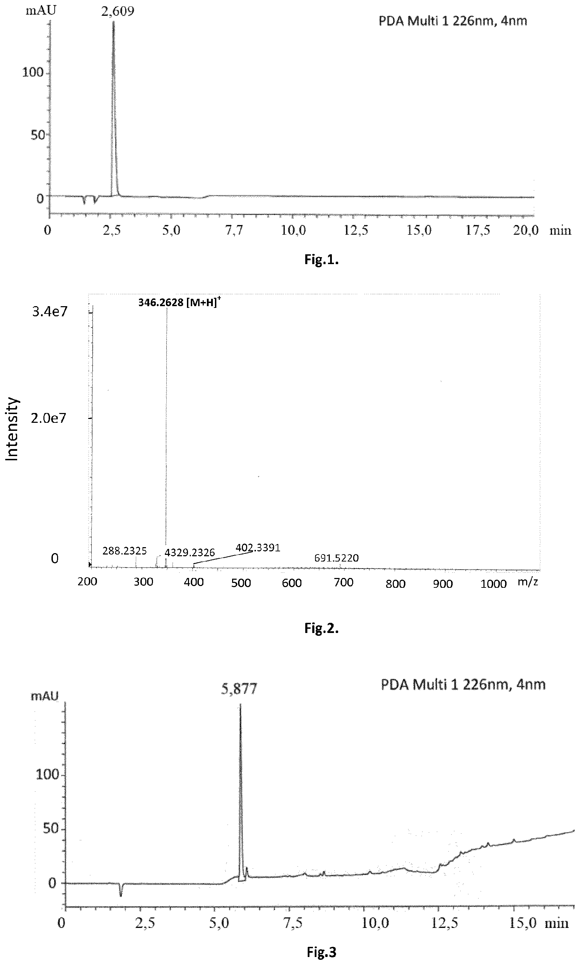

As a consequence of the aforementioned injuries of brain and spinal cord (both compression/post-strike injuries or and mechanical wound to the brain/spinal cord), and the strokes (both ischemic and hemorrhagic), a series of interweaving and self-stimulating events are induced, such as: 1) over-stimulation of neurons (excitotoxicity), 2) inflammation stimulated by microglial cells present in CNS or by lymphatic cells infiltrating from the circulatory system, and 3) phenomena of cellular death (occurring as an effect of an intracellular program, called apoptosis, as well as these effecting from necrosis) (Fig. 1).

Fig. 1. Diagram of the course of changes after damage to the central nervous system.

These three group of processes appear in different time windows and with different intensity after the injury initiation. A common feature of the neurological injuries mentioned above is a characteristic cascade of secondary, physiological events (Fig. 2). The most important clinical factor underlying these mechanisms is loss of integrity of the blood vessels and the disruption of the blood-brain barrier (BBB). In general, the blockage of blood flow in the brain results in loss of oxygen, glucose and nutrients, i.e. so-called respiratory-energetic deprivation and that leads to the immediate neural cell death (necrosis) and, in the further perspective, to the programmed cell death (apoptosis) within the insulted brain region, and to global inflammation within the tissue. The energy deprivation is a basic metabolic process causing a self-stimulating series of biochemical and neurochemical reactions. Taking into account the time period of these reactions, it is necessary to distinguish short-term (minutes) depolarization processes leading to interconnected events of an increase of intracellular calcium, pH decrease due to tissue acidosis caused by accumulation of lactate as an anaerobic metabolite, and above the others, dangerous, self-arousing neuronal excitation, i.e. excitotoxicity, followed by long-term (hours, days) processes dominated by inflammation and apoptosis. A key pathological symptom is the excitotoxicity itself as a result of uncontrolled neuronal depolarization and of the calcium-dependent activation of glutamate receptors causing an excessive release of excitatory neurotransmitters (glutamate). Eventually, accumulation of glutamate in the synaptic cleft results in chain stimulation of neighbouring neurons. Such dysregulation of an electrochemical equilibrium in the synapses, accompanied by oxygen deficiency, directly affects cellular respiration, a so called oxidative stress. Neurological injuries, especially post-stroke complications, lead to many alterations of the brain vascularity and abnormal angiogenesis. The angiogenesis is a process of forming new blood vessel and plays a key role during tissue renewal and regeneration. These processes are mainly governed by cell-secreted extracellular matrix molecules (ECM) that are responsible for continuous proliferation, migration, adhesion, maturation, and cell-to-cell communication of the endothelial cells. The ECMs are primarily fibrous proteins (e.g. collagens, elastins), laminins, fibronectins and glycosaminoglycans, heparan sulfates or non-proteoglycan polysaccharides (e.g. hyaluronic acid). In particular, a role of glycoprotein fibronectin in angiogenesis and regeneration has been established both *in vitro* and *in vivo.*

**Oxygen and glucose deprivation**

**Oxidative stress** ⟵--⟶ **Excitotoxicity**

**Acidosis within the tissue**

**Accumulation of lactate**

Fig. 2

Fibronectin interacts with collagen and cell-surface integrins, by which contributes to cytoskeletal rearrangement, facilitating fibrin adhesion to fibroblasts during blood clotting (trombogenesis). The integrins are $\alpha\beta$ heterodimers involved in formation of cell-to-cell and cell-to-ECM connections, providing a fundamental for transmembrane signaling, proliferation, differentiation, growth and migration. Native ligands of these receptors display distinct activity and structural selectivity. The examples are: fibronectin and vitronectin preferably binding to $\alpha_V\beta_3$ integrin; fibrinogen - to $\alpha IIb\beta_3$, whereas tenascin to $\alpha_V\beta_1$ and $\alpha_V\beta_6$; there are also interactions of specific ligands with different integrin types, e.g. these of laminin and osteopontin. All ECM proteins pointed above share the presence of RGD (Arg-Gly-Asp) amino acid sequence flanked by different neighbouring sequences. Activity of the RGD sequence exhibits mainly during the extracellular signal transduction and proliferation. Moreover, RGD-recognizing integrins are considered oncomarkers of certain tumors with high potential to metastasis. Besides, RGD-conjugates display positive properties in regeneration stimulation in bones, cornea and heart. RGD-dependent integrins play a crucial role in synaptic plasticity and neurological diseases. In general, this activity involves maintenance of the neuron-glial cell interactions in a form of the mesh-like connections between cytoskeletons of these cells and the ECM. Such interactions are indispensable for proper nervous system development. Additionally, integrins participate in neurotransmitter receptors coupling contributing to synaptic plasticity, that is mechanistic CNS capability to adaptive changes, such as memory and learning as well as to functional-structural alterations following neurological diseases, such as epilepsy, trauma, injuries or neurodegeneration. Although many experimental evidences supporting aforementioned reports for a role of the integrins and ECM in neuropathologies such as multiple sclerosis, glioblastomas, brain tumors, neurodegenerative diseases, epilepsy, strokes, neurological injuries and peripheral nerves lesions, still little is known about molecular mechanisms of these processes. Specificity of the RGD sequence binding to a wide spectrum of receptors depends on its conformation and allosteric specificity towards respective integrins. Structural modifications of the RGD sequence, such as C-amidation or N-acetylation, yield improved resistance against hydrolytic proteolysis in physiological conditions. In turn, multiplication of the active RGD sequence in a single oligopeptide chain enhances the activity and provides controlled release of the RGD tripeptide in a living organism. In consistence with these data, it is worth to mention *in vivo* studies concerning a vital and positive role of the RGD motif-containing proteins/peptides in therapy of neurological diseases and injuries. These data show the involvement of the RGD sequence in allosteric blockage of integrin-ECM protein interaction by a decreasing in the number of available integrin receptors. Such activity of RGD-containing peptides results in therapeutic attenuation of epileptic seizures and cognitive improvement of learning deficits in an Alzheimer disease model. A positive effect of the RGD sequence-containing peptides has been also demonstrated in *in vivo* regeneration of peripheral nerves, in which peptides stimulated the outgrowth of nerve fibers. Furthermore, implantation of RGD sequence-incorporated collagen tubes to injured rat peripheral nerves improved secretion of trophic factors, enhanced proliferation of Schwann cells, increased axonal myelination and fiber formation in early days of regeneration. In a rat model of spinal cord injury, RGD sequence-grafted polymers provide a stable scaffold enabling the outgrowth of axons and nerves. RGD-containing materials have been also exploited as conjugates with cell-penetrating peptides, developed for the BBB crossing. In a rat model of focal ischemia, the intraperitoneally-delivered RGD-penetratin conjugate has been shown to cross the BBB and enter ischemic brain hemisphere and leads to a reduction of penumbra area. Analysis of molecular mechanisms underlying those activities showed crucial influence of the RGD-containing materials on cellular signaling pathway and angiogenesis stimulation.

RGD-dependent integrins play a key role in signal transduction across the synaptic cleft. Nevertheless, despite vast experimental evidences, details regarding neuroprotective and neuoregenerative mechanisms of integrins and the RGD-containing proteins, remain unknown. For the reason of integrin contribution to long-term physiological processes, such as inflammation and apoptosis, it seems that it is not to sufficient to consider receptors blockage for satisfactory therapeutic effect. Hence, wide-spread basic research focused on explaining these molecular mechanisms seems obligatory. In recent decades a lot of attention has been paid to beneficial influence of the RGD motif on stimulation, proliferation and migration of neural stem cells (NSCs). It has been shown that the RGD peptide-functionalized biomaterials effectively support the NSC attachment and maturation. Similarly, polymeric scaffolds combined with neurotrophic growth factors and the RGD motifs, can cause immobilization of human NSCs, facilitating their differentiation.

As aforementioned, a tangible outcome of any neurological injury is a complex series of primary or secondary events, that lead directly or indirectly to death of neural, glial and other cells - fig. 2. Damage factors: glucose deprivation, reduction of cellular respiration ($NaN_3$), excitotoxicity, glutamic acid, kainic acid , NMDA, acidosis / acidification (pH = 6.35, lactate)

Dysregulation of biochemical and electrochemical processes caused by the injury happen as energetic deprivation, oxidative stress and acidosis, that generally lead to excitotoxicity. This process is a climax of uncontrolled calcium-dependent activation of glutamate receptors, occurring as accumulation of glutamate in a synaptic cleft and as a self-driven neuronal excitation. *In vitro* techniques that model these processes are well described in subject literature. Application of the setup of all of these techniques or their modifications within a all-purpose, multifactorial methodology, is suggested to provide additional/extra economical and factual advantages. In our approach, we render the use and complex analysis of six injury factors, namely: glucose deprivation, partial inhibition of cellular respiration and oxidative stress (*via* sodium azide); and excitotoxity [induced by glutamic acid (including an intracellular increase of calcium ions concentration), NMDA and kainic acid], and acidification (acidosis, pH 6.35, caused by increased sodium lactate concentration). A combination of all mentioned techniques yields more efficacy than each technique applied separately, and allows omitting disturbances between experimental sessions. In turn, application of such a combination in a specific session of six parallel measurement of the key physiological responses in the CNS injury to one particular cell culture constitute both a simplified, multifactorial *in vitro* model of the neurological injury, and a well-performing, high-throughput experimental method. The application of six injury factors constitute a true improvement of technological merit and novelty.

An object of the invention is to provide a method for obtaining concatameric polysignaling proteins composed of monomeric peptide units, for use in regenerative medicine.

The invention are five chemical compounds :

| | |
|---|---|
| H$_2$N-RGD-amide | (referred in the text as **RGD1**), |
| H$_2$N-RGDRGD-amide | (referred in the text as **RGD2**), |
| H$_2$N-RGDRGDRGD-amide | (referred in the text as **RGD3**), |
| acetyl-RGDRGDRGD-amide | (referred in the text as **Ac-RGD3**) |
| H$_2$N-RGDRGDRGDRGD-amide | (referred in the text as **RGD4**) |

for medical use as an agent for regeneration and/or treatment of neuroligical injury Some chemical compounds are used within this patent application as a reference: acetyl-RGD-amide (referred in the text as **Ac-RGD1**),

H$_2$N - RGD-RGD-RGD-RGD-RGD-RGD-RGD-RGD-RGD-RGD -amide (referred in the text as **RGD10-PM**),

IMSRSSP- RGD-RGD-RGD-RGD-RGD-RGD-RGD-RGD-RGD-RGD -PRRA (referred in the text as **RGD10-PS**).

A subject of the invention is also a method for the preparation of the chemical compound RGD10-PS and its derivatives by a biological method through the use of chemical synthesis of the encoding gene and, optionally, the technology of amplifying the DNA segment of the vectors indicated in the patent PL228341 B.

A subject of the invention is also *in vitro* application of a novel method for the evaluation of

## Figure legend:

**Fig. 1** - shows a HPLC chromatogram of peptide RGD 1

**Fig. 2** - shows a ESI MS spectrum of peptide RGD 1

**Fig. 3** - shows a HPLC chromatogram of peptide RGD2

**Fig. 4** - shows a ESI MS spectrum of peptide RGD2

**Fig. 5** - shows a chromatogram HPLC of peptide RGD3

**Fig. 6** - shows a ESI MS spectrum of peptide RGD3

**Fig. 7** - shows a chromatogram HPLC of peptide RGD4

**Fig. 8** - shows a ESI MS spectrum of peptide RGD4

**Fig. 9** - shows a chromatogram HPLC of peptide RGD10-PM

**Fig. 10** - shows a ESI MS spectrum of peptide RGD10-PM

**Fig. 11** - shows slab gel electrophoresis (A) and capillary electrophoresis (B) of peptide RGD10-PS. Legend of the gel: A: M-mass marker, RGD10-PS-Ubq (a fusion protein of RGD10-PS peptide conjugated with ubiquitin), RGD10-PS - peptide IMSRSSPRGDRGDRGDRGDRGDRGDRGDRGDRGDRGDPRRA cleaved from ubiquitin. Gel staining - Coomassie

**Fig. 12** - shows a chromatogram HPLC of peptide AcRGD 1

**Fig. 13** - shows a ESI MS spectrum of peptide AcRGD 1

**Fig. 14** - shows a chromatogram HPLC of peptide AcRGD3

**Fig. 15** - shows a ESI MS spectrum of peptide AcRGD3

**Fig. 16** - displays the nucleotide sequence of the expression DNA cassette, along with the selected functional areas, containing genes encoding for the [His6-c-Myc-WYY-ubiquitinpoly-epitope- RGD10] protein and the amino acid sequence of the expressed fusion protein.

**Fig. 17** - displays the nucleotide sequence of the expression DNA cassette, along with the selected functional areas, containing genes encoding for the [His6-c-Myc-WYY-ubiquitin-poly-epitope- RGDGG10] protein and the amino acid sequence of the expressed fusion protein.

**Fig. 18** - displays the genetic map of the expressed construct along with the nucleotide and amino acid sequences of plasmid pET28amutSapIKASETARGD, together with marked functional modules

**Fig. 19** - displays the genetic map of the expressed construct along with the nucleotide and amino acid sequences of plasmid pET28amutSapIKASETARGD, together with marked functional modules

**Fig. 20 -** displays the genetic map of the expressed construct along with the nucleotide and amino acid sequences of plasmid pET28amutSapIKASETARGDGG, together with marked functional modules

**Fig. 21** - displays the genetic map of the expressed construct along with the nucleotide and amino acid sequences of plasmid pET28amutSapIKASETARGDGG, together with marked functional modules

**Fig. 22** - shows the proteins: [His6-c-Myc-WYY-ubiquitin-poly-epitope RGD10] protein and His6-c-Myc-WYY-ubiquitin - poly-epitope RGDGG10 protein.

22a) shows the result of the protein electrophoresis under denaturing conditions (SDS-PAGE gel stained with Coomassie Brilliant Blue), samples were prepared from cell extracts and the chromatographic fraction of [His6-c-Myc-WYY-ubiquitin-poly-epitope RGD10] protein purification, with a predicted molecular mass of 16.2 kDa in accordance with the disclosed amino acid sequence (SEQ. 2). The line M contains standards for molecular weights of the following proteins: bovine serum albumin 66 kDa, ovalbumin 40 kDa, carbonic anhydrase 29 kDa, soybean trypsin inhibitor 20/21 kDa, cytochrome C 12 kDa, aprotinin 6.5 kDa; the line unb represents unbound cellular proteins; W2 represents proteins present in the column wash; and E3-6 represents fractions eluted from the column.

22b) shows the result of the protein electrophoresis under denaturing conditions in 12.5% Tris-Tricine buffered SDS-PAGE. Samples were obtained from cell extracts and chromatographic fraction of [His6-c-Myc-WYY-ubiquitin-poly-epitope RGDGG10] protein purification, with a predicted molecular mass of 17.3 kDa. The line unb represents unbound cell extract proteins, W2 represents proteins present in the buffer after column wash, and E3-6 represent fractions eluted from the column.

22c) from sample preparation of [His6-c-Myc-WYY-ubiquitin-poly-epitope RGDGG10] protein, done under control conditions, i.e. no IPTG induction of the bacterial culture. Line M contains molecular weight standards, line unb represents unbound cell extract proteins, W2 represents proteins present in the buffer after column wash, and E3-6 represents fractions eluted from the column.

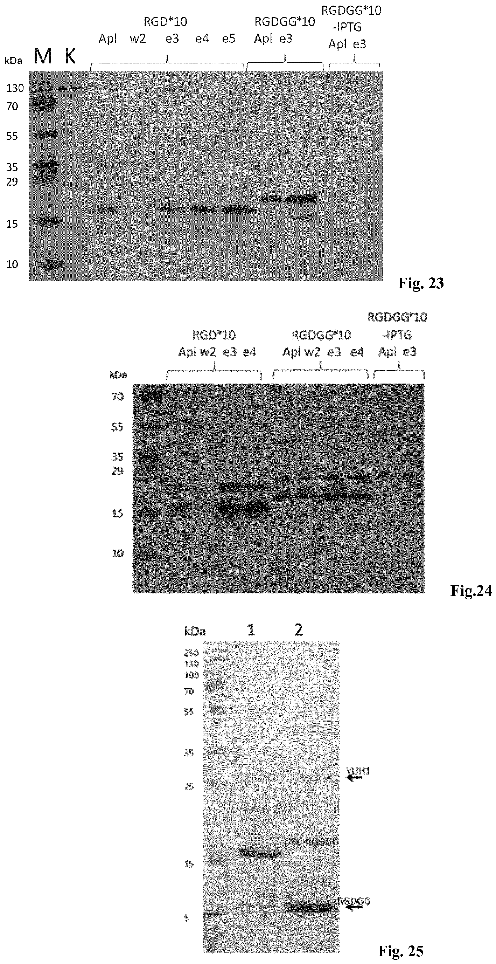

**Fig. 23-** shows [His6-c-Myc-WYY-ubiquitin-poly-epitope RGD10] protein

**Fig. 24**-shows [His6-c-Myc-WYY-ubiquitinpoly-epitope RGDGG10] protein

**Fig. 25**-shows the results of the digestion of [His6-c-Myc-WYY-ubiquitin-poly-epitope RGDGG10] protein by the YUH1 enzyme

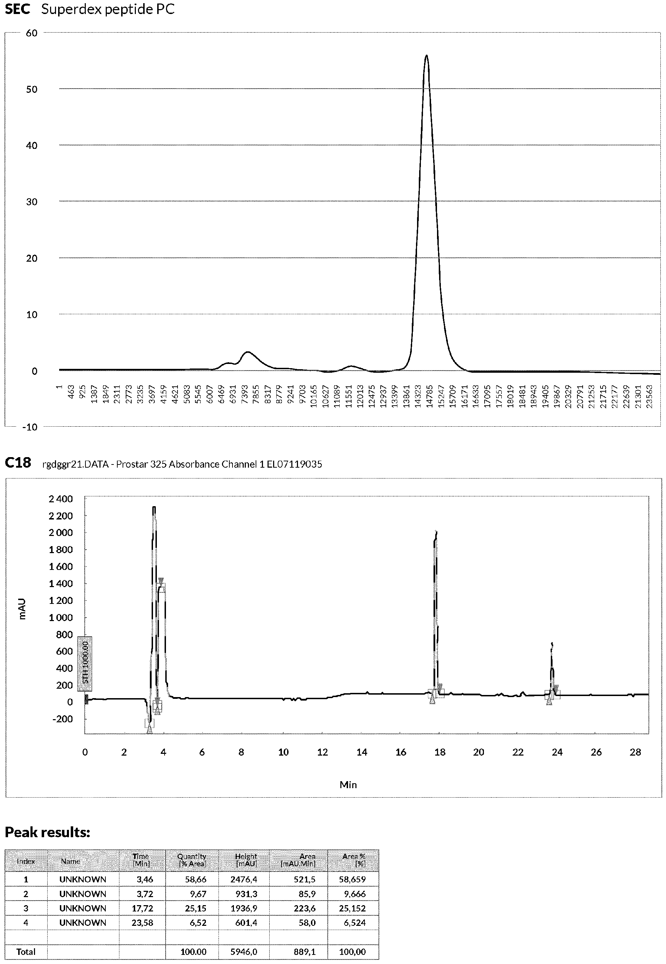

**Fig. 26** - shows two techniques for quantitative assessment of the protein

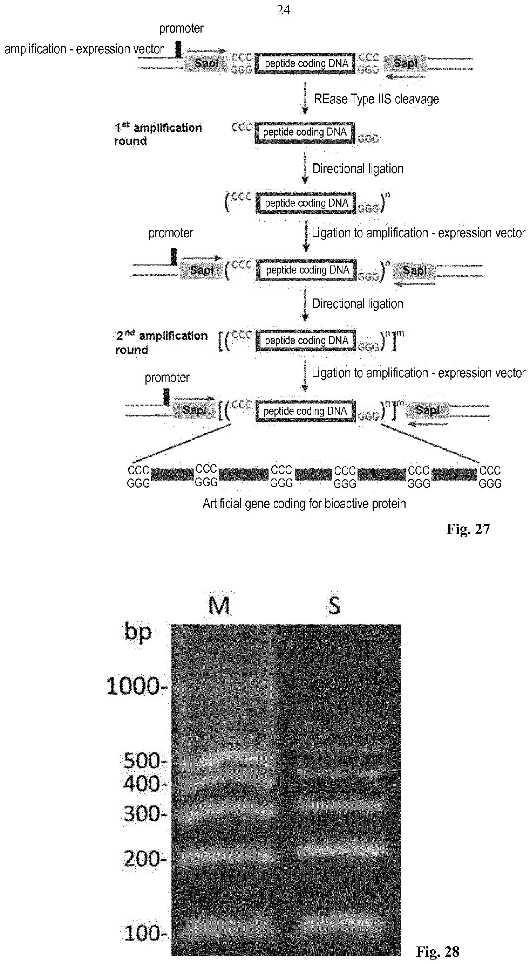

**Fig. 27** - shows a diagram of the duplication of a synthetic DNA segment encoding the 10-mer of RGD and the 10-mer of RGDGG.

**Fig. 28** - displays the directional amplification of a DNA section encoding for the RGD module* 10, using an enzymatic-vector system.

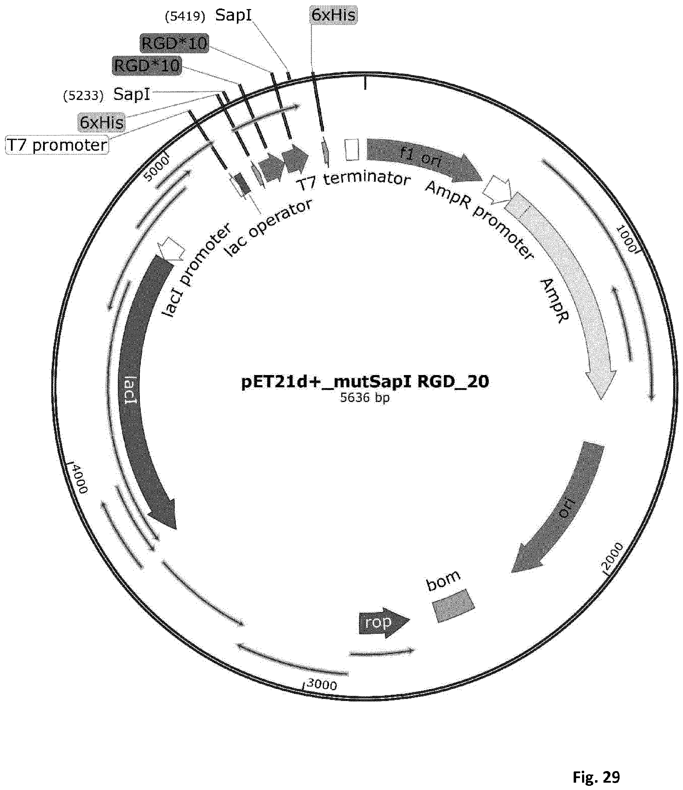

**Fig. 29** - shows genetic maps of the amplification vector constructs marked as pET21d+ _mutSapIRGD_20, containing cloned poly-10-mer-RGD, with 20 copies of the RGD peptide coding module, with molecular mass of 8.6 kDa.

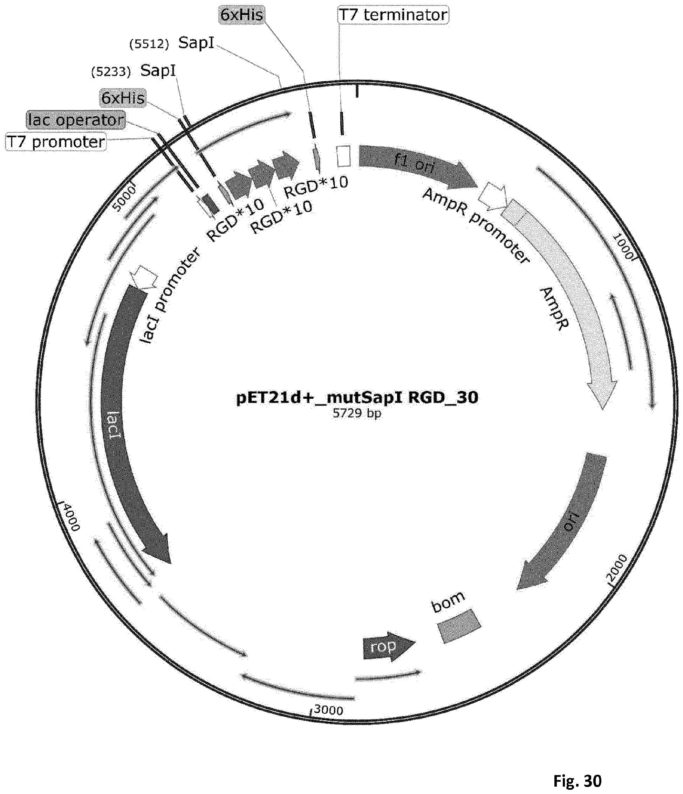

**Fig. 30** - shows genetic maps of the amplification vector constructs marked as pET21d+ _mutSapIRGD_30, containing cloned poly-10-mer-RGD, carrying 30copies of the RGD peptide coding module, with molecular mass of, 12.0 kDa.

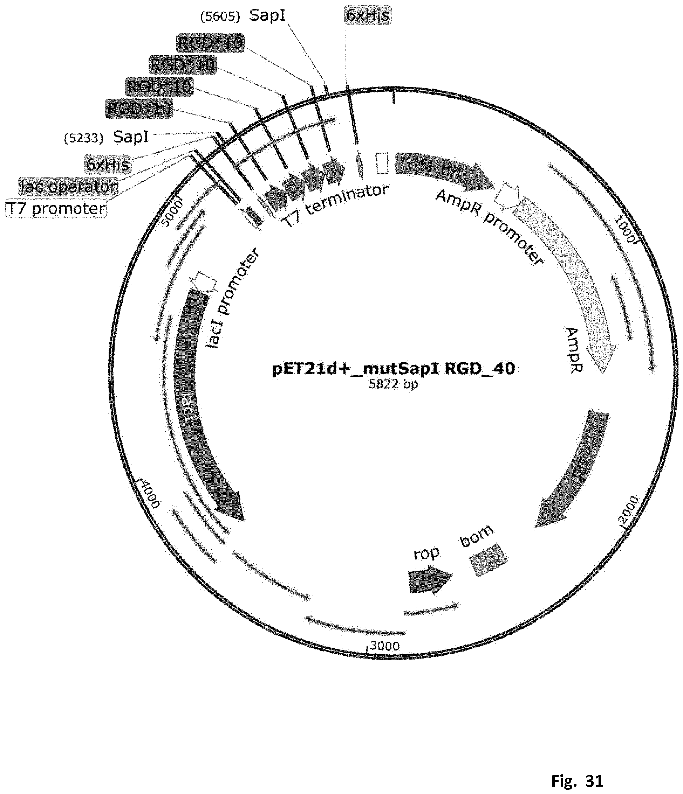

**Fig. 31** - shows genetic maps of the amplification vector constructs marked as pET21d+ _mutSapIRGD_40, containing cloned poly-10-mer-RGD, carrying 40copies of the RGD peptide coding module, with molecular mass of 15.4 kDa.

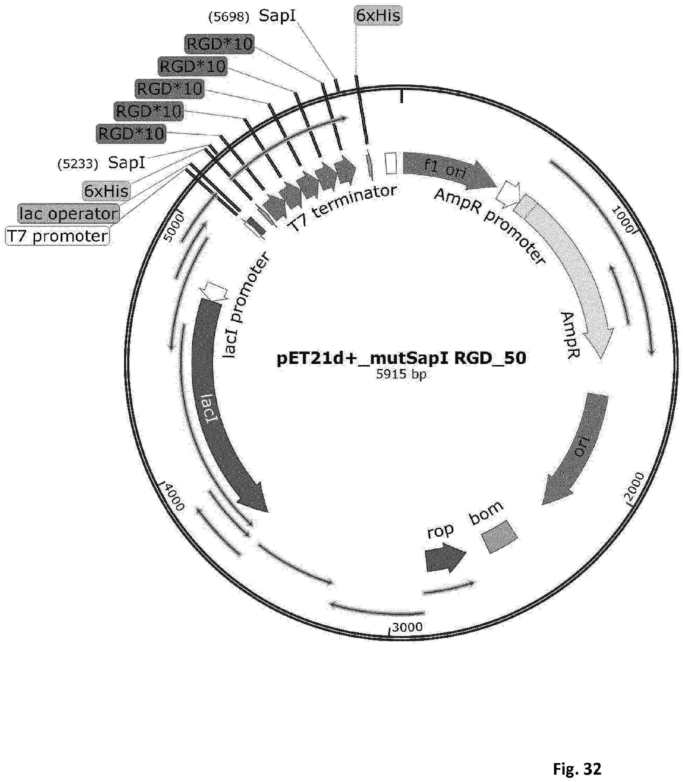

**Fig. 32** - shows genetic maps of the amplification vector constructs marked as pET21d+ _mutSapIRGD_50, containing cloned poly-10-mer-RGD, carrying 50copies of the RGD peptide coding module, with molecular mass of 18.7 kDa.

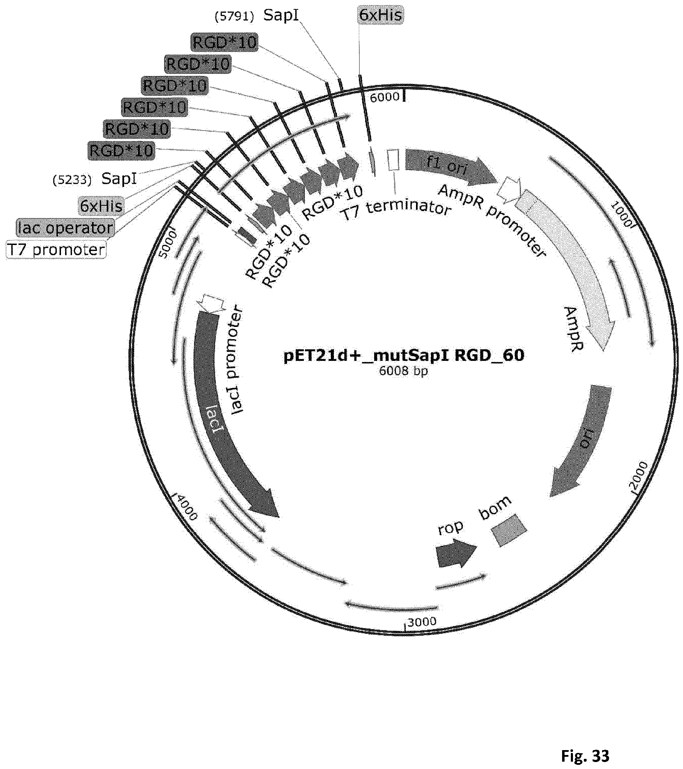

**Fig. 33** - shows genetic maps of the amplification vector constructs marked as pET21d+ _mutSapIRGD_60, containing cloned poly-10-mer-RGD, carrying 60, copies of the RGD peptide coding module, with molecular mass of 22.1 kDa.

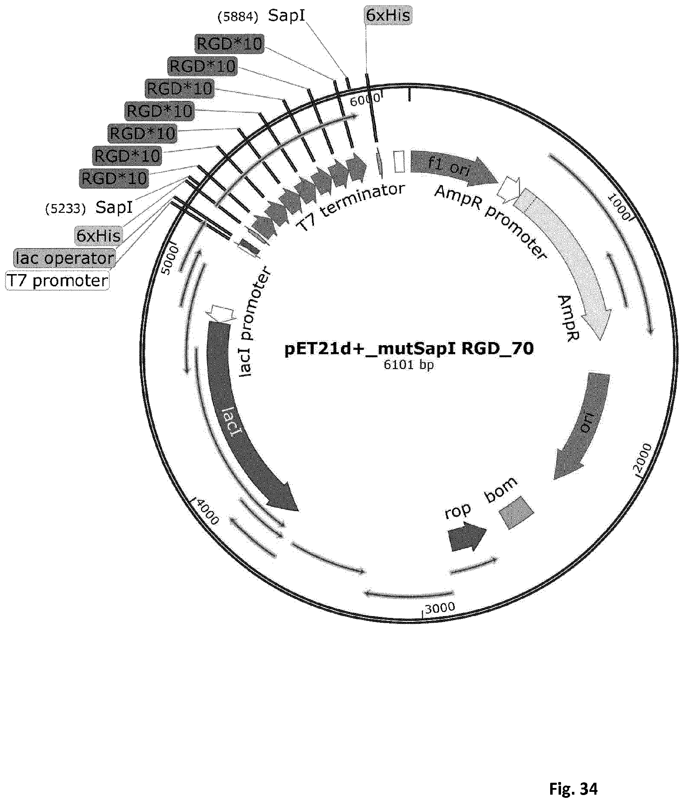

**Fig. 34** - shows genetic maps of the amplification vector constructs marked as pET21d+ _mutSapIRGD_70, containing cloned poly-10-mer-RGD, carrying 70copies of the RGD peptide coding module, with molecular mass of 25.5 kDa.

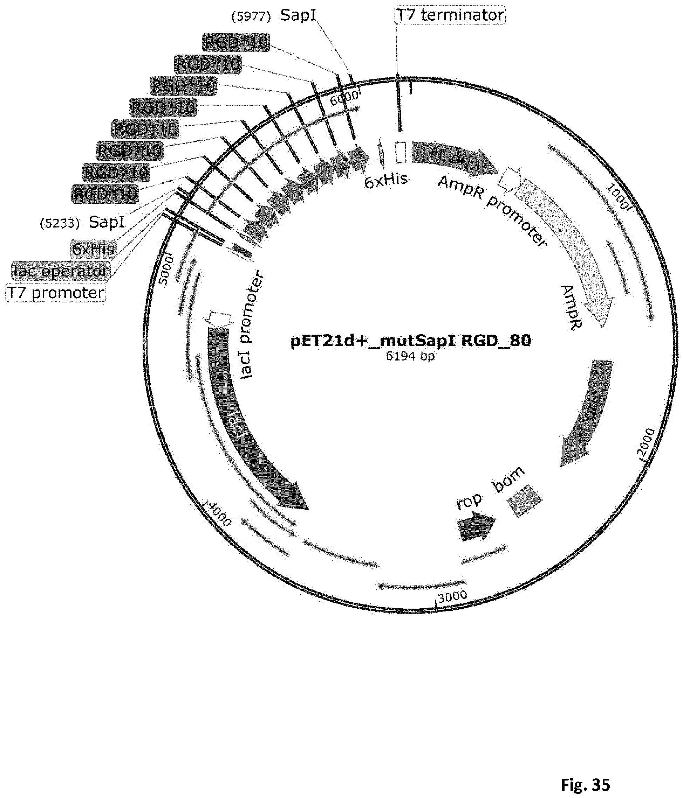

**Fig. 35** - shows genetic maps of the amplification vector constructs marked as pET21d+ _mutSapIRGD_80, containing cloned poly-10-mer-RGD, carrying 80copies of the RGD peptide coding module, with molecular mass of 28.9 kDa.

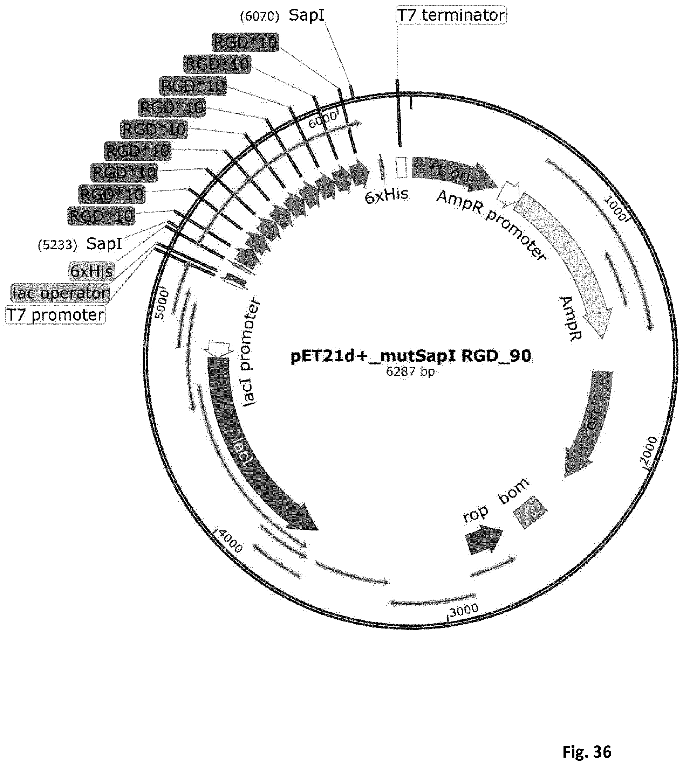

**Fig. 36** - shows genetic maps of the amplification vector constructs marked as pET21d+ _mutSapIRGD_90, containing cloned poly-10-mer-RGD, carrying 90 copies of the RGD peptide coding module, with molecular mass of 32.3 kDa.

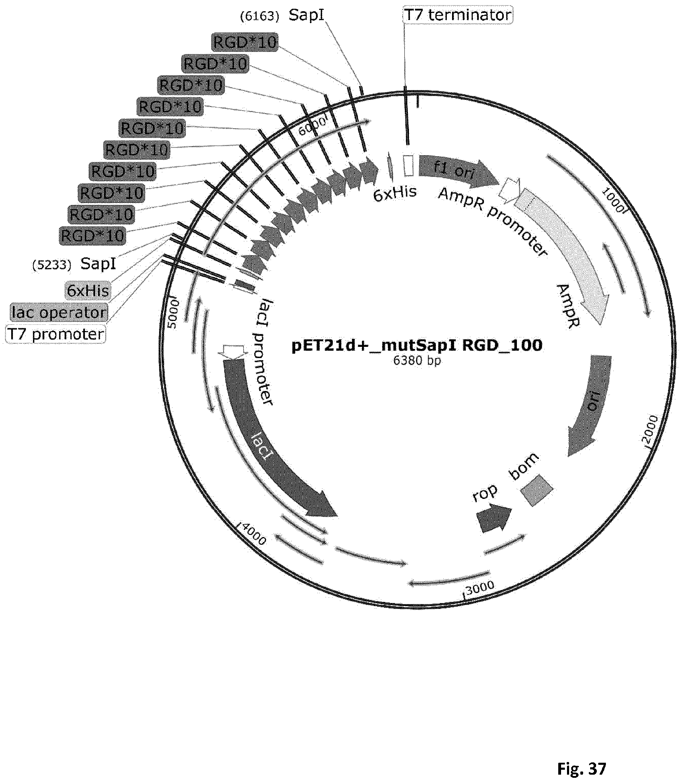

**Fig. 37** - shows genetic maps of the amplification vector constructs marked as pET21d+ _mutSapIRGD_100, containing cloned poly-10-mer-RGD, carrying 100 copies of the RGD peptide coding module, with molecular masses of 35.6 kDa.

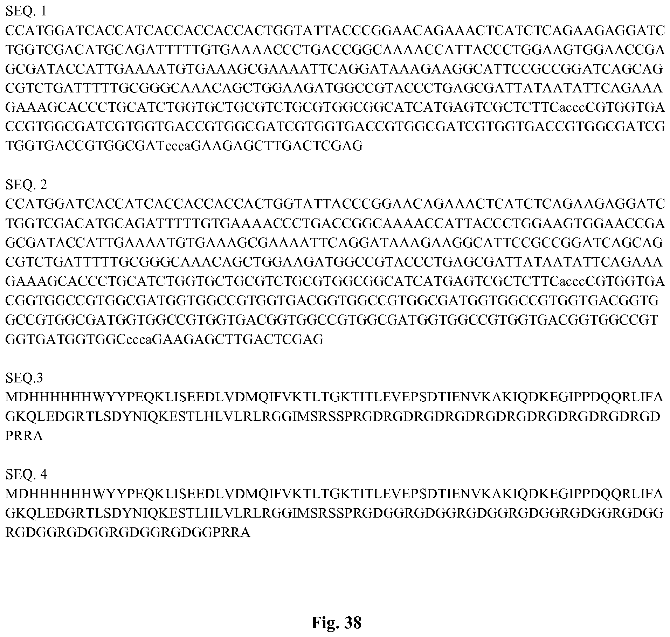

**Fig. 38** - displays the sequences Seq 1; Seq 2, Seq 3 and Seq 4.

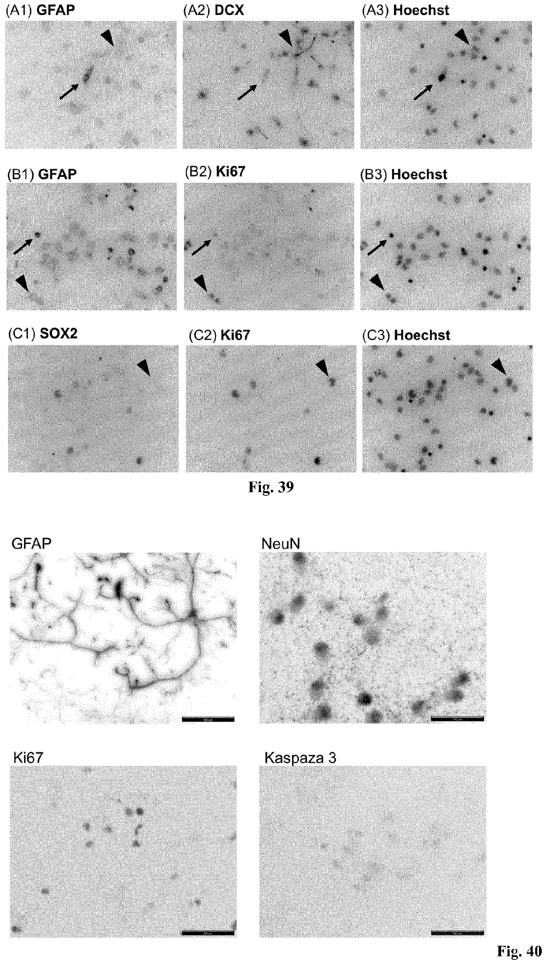

**Fig. 39** - presents representative microscopic images of the marker proteins (epitopes) detected by immunocytochemical methods, in primary rat brain neural cells, after 1 day of *in vitro* culturing. In the upper panel (A series): a small arrow indicates the cell is positive for GFAP protein (image A1) characteristic of astrocytes (glial cells), whereas a big arrowhead indicates cells containing DCX protein (doublecortin) (image A2), a marker of adolescent neurons (neuroblasts) Hoechst staining (image A3) visualizes all cells observed in a field of vision. The central panel (B series) presents cells, indicated by a big arrowhead, that undergo cellular division, visualized by the presence of Ki67 protein during a period of proliferation (image B2), and also presents selected astrocyte cells, indicated by a small arrow, that do not undergo any division in the same period (image B2). The lower panel (C series) presents neural cells displaying immunocytochemical reactivity for precursor cells (as determined by the presence of the marker protein SOX2), among which are dividing cells, which are shown as cells on the bottom of the C1 image. The figure also shows other cells (non-precursor, non-reactive to SOX2), displaying cellular divisions (indicated by a big arrowhead on the C2 image).

Our conclusion based on this observation is that a one day-old culture of primary neural cells, isolated from the developing brain cortex, exhibits a diversity of cells displaying distinct proliferation activity.

**Fig. 40** - presents immunocytochemical detection of characteristic marker proteins of different, co-occurring cell populations, on the fourteenth day of *in vitro* culturing (14 DIV), such as: mature astrocytes of proper morphology, displaying the presence of self-typical GFAP protein, as well as mature neurons, displaying the presence of self-characteristic NeuN protein. The culture contains both cells undergoing cellular division, which exhibit immunoreactivity for Ki67 protein, and non-dividing cells which do not show such immunoreactivity. The no injury-stimulated 14 DIV culture exhibits no cells that undergo cellular death (the cells are non-reactive to caspase 3 protein).

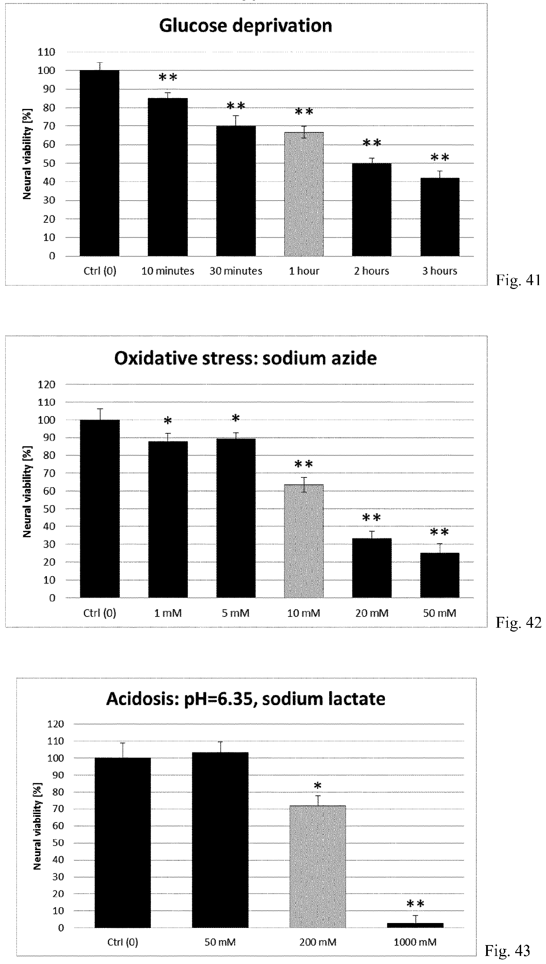

**Fig. 41 -** presents responses of a neural culture to different intensities of an injury factor in the form of inhibition of glucose deprivation; on this basis a one-hour long deprivation period (grey bar) was selected for further experiments of the neuroprotective activity evaluation of the analyzed peptides, as one maintaining around 70% neural viability.

**Fig. 42** - presents responses of a neural culture to different intensities of an injury factor in the form of inhibition of cellular respiration (oxidative stress); on this basis a 10 mM sodium azide concentration (grey bar) was selected for further experiments of the neuroprotective activity evaluation of analyzed peptides, as one maintaining around 65% neural viability.

**Fig. 43** - presents responses of a neural culture to different intensities of an injury factor in the form of medium acidification (acidosis); on this basis a 200 mM sodium lactate concentration (grey bar) was selected for further experiments of the neuroprotective activity evaluation of analyzed peptides, as one maintaining around 70% neural viability.

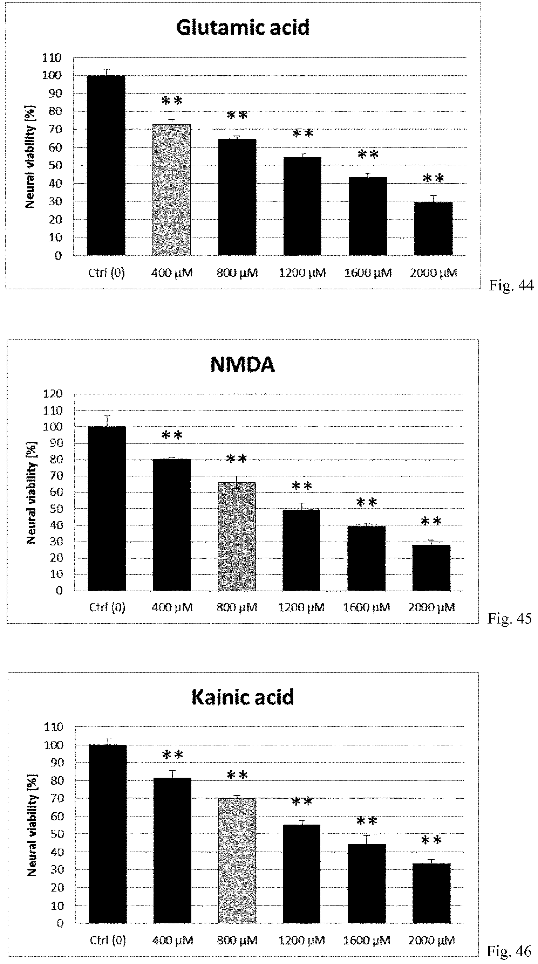

**Fig. 44** - presents responses of a neural culture to different intensities of an injury factor in the form of excessive neuronal excitation (excitotoxicity) induced by glutamic acid; on this basis a 400 $\mu$M glutamic acid concentration (grey bar) was selected for further experiments of the neuroprotective activity evaluation of the analyzed peptides, as one maintaining around 70% neural viability.

**Fig. 45** - presents responses of a neural culture to different intensities of an injury factor in the form of excessive neuronal excitation (excitotoxicity) induced by NMDA; on this basis a 800 $\mu$M NMDA concentration (grey bar) was selected for further experiments of the neuroprotective activity evaluation of the analyzed peptides, as one maintaining around 70% neural viability.

**Fig. 46** - presents responses of a neural culture to different intensities of an injury factor in form of excessive neuronal excitation (excitotoxicity) induced by kainic acid; on this basis a 800 $\mu$M kainic acid concentration (grey bar) was selected for further experiments of the neuroprotective activity evaluation of analyzed peptides, as one maintaining around 70% neural viability.

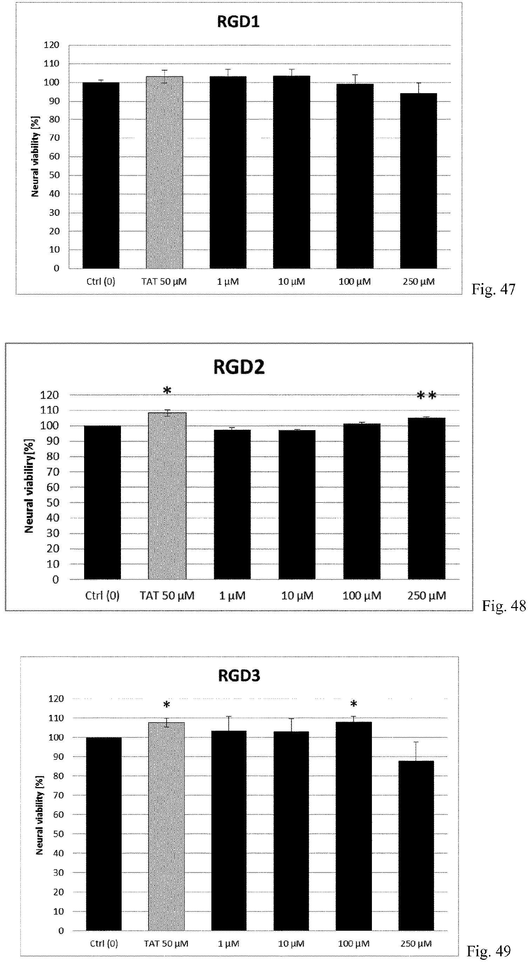

**Fig. 47** - presents the influence of peptide RGD1 on a neural culture. No cytotoxic activity of the peptide RGD1 was observed at all concentrations. TAT peptide at 50 $\mu$M concentration was used as a reference, indicated by a grey bar.

**Fig. 48** - presents the influence of peptide RGD2 on a neural culture. Slight cytotoxicity of the peptide RGD2 was observed at all concentrations. TAT peptide at 50 $\mu$M concentration was used as a reference, indicated by a grey bar.

**Fig. 49 -** presents the influence of peptide RGD3 on a neural culture. Slight cytotoxicity of the peptide RGD3 was observed at all concentrations. TAT peptide at 50 $\mu$M concentration was used as a reference, indicated by a grey bar.

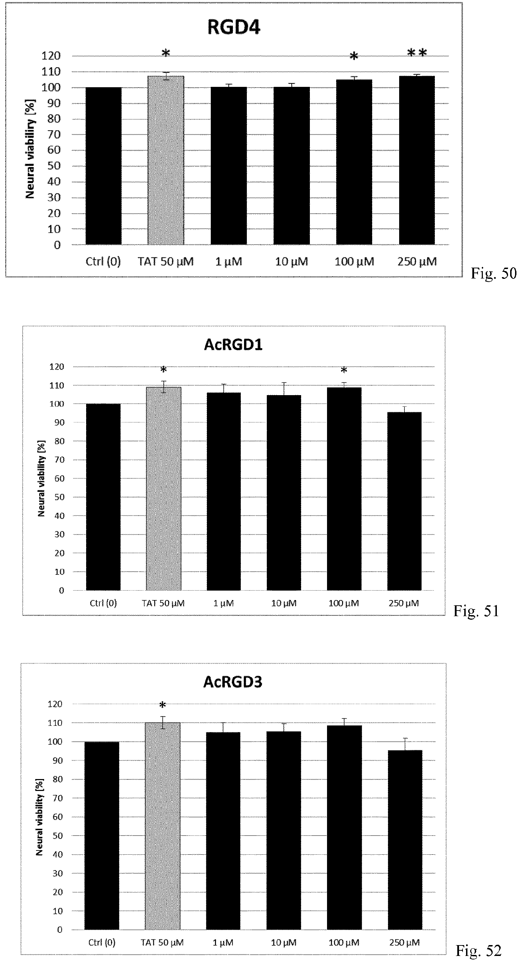

**Fig. 50** - presents the influence of peptide RGD4 on a neural culture. TAT peptide at 50 $\mu$M concentration was used as a reference, indicated by a grey bar.

**Fig. 51-** presents the influence of peptide AcRGD 1 on a neural culture. TAT peptide at 50 $\mu$M concentration was used as a reference, indicated by a grey bar.

**Fig. 52** - presents the influence of peptide AcRGD3 on a neural culture. TAT peptide at 50 $\mu$M concentration was used as a reference, indicated by grey bar.

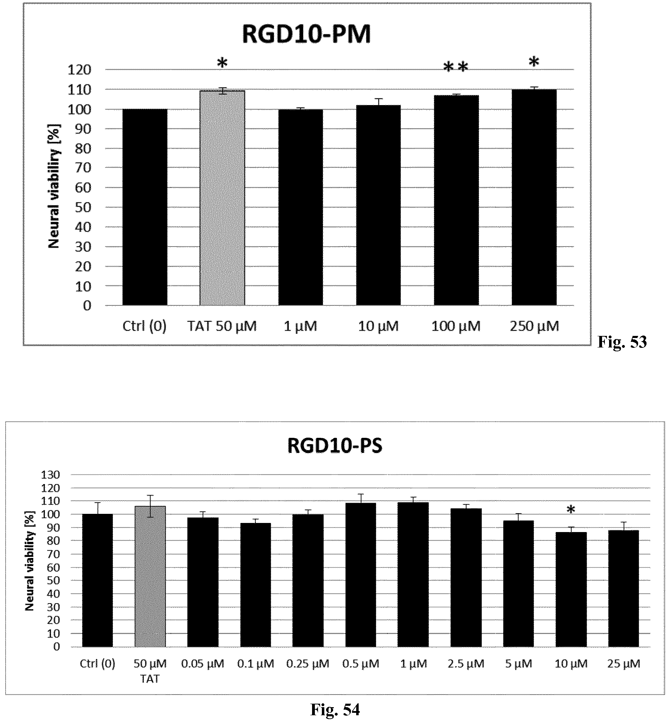

**Fig. 53** - presents the influence of the chemically synthesized peptide RGD10-PM on a neural culture. TAT peptide at 50 $\mu$M concentration was used as a reference, indicated by a grey bar.

**Fig. 54** - presents the influence of the biologically synthesized peptide RGD1S-PM on a neural culture. TAT peptide at 50 $\mu$M concentration was used as a reference, indicated by a grey bar.

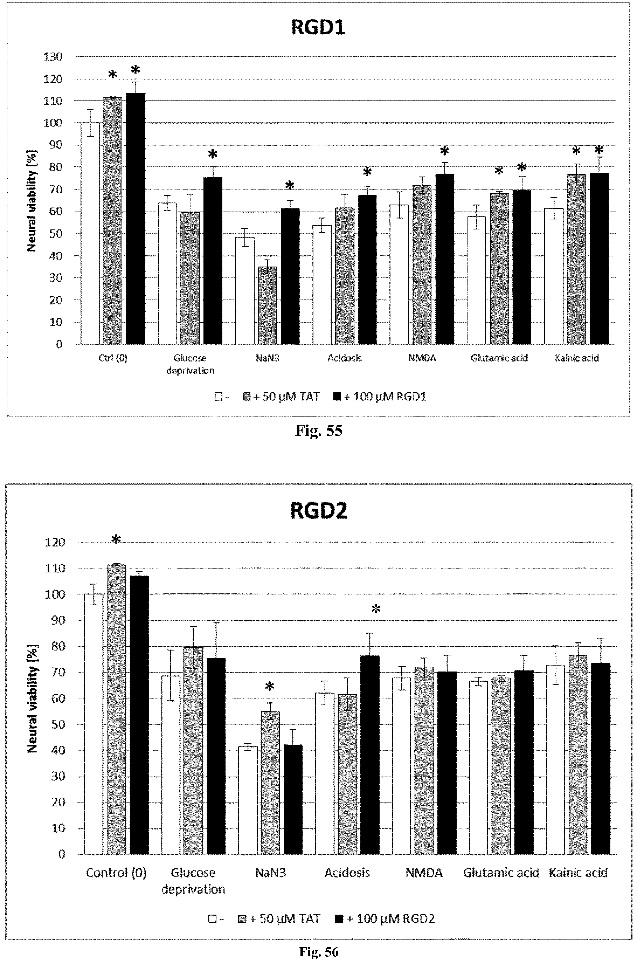

**Fig. 55** - presents activity of RGD1 in a multifactorial *in vitro* model of neurological injury.

**Fig. 56** - presents activity of RGD2 in a multifactorial *in vitro* model of neurological injury.

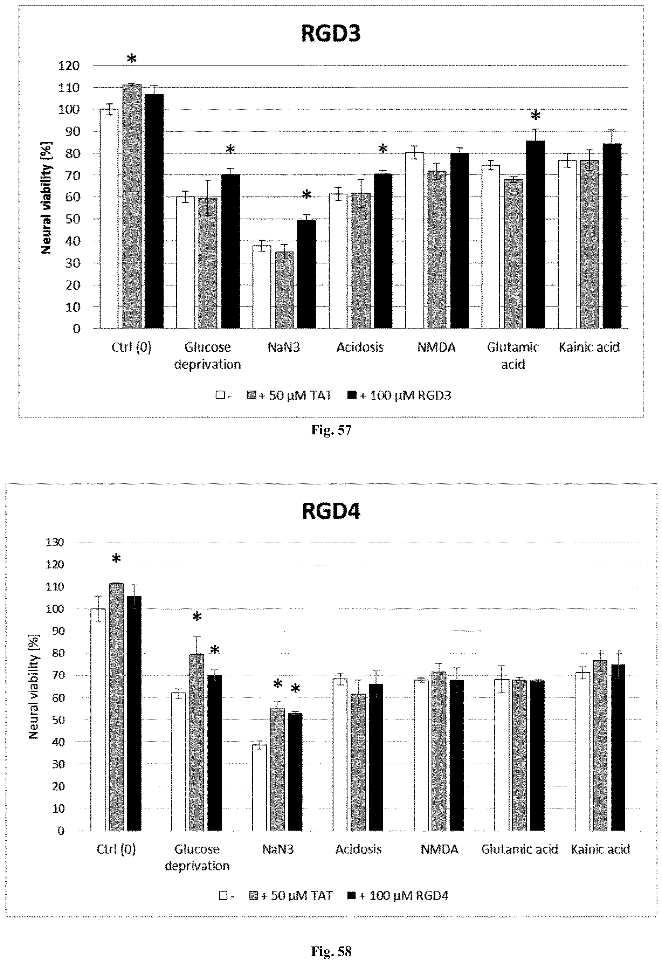

**Fig. 57** - presents activity of RGD3 in a multifactorial *in vitro* model of neurological injury.

**Fig. 58** - presents activity of RGD4 in a multifactorial *in vitro* model of neurological injury.

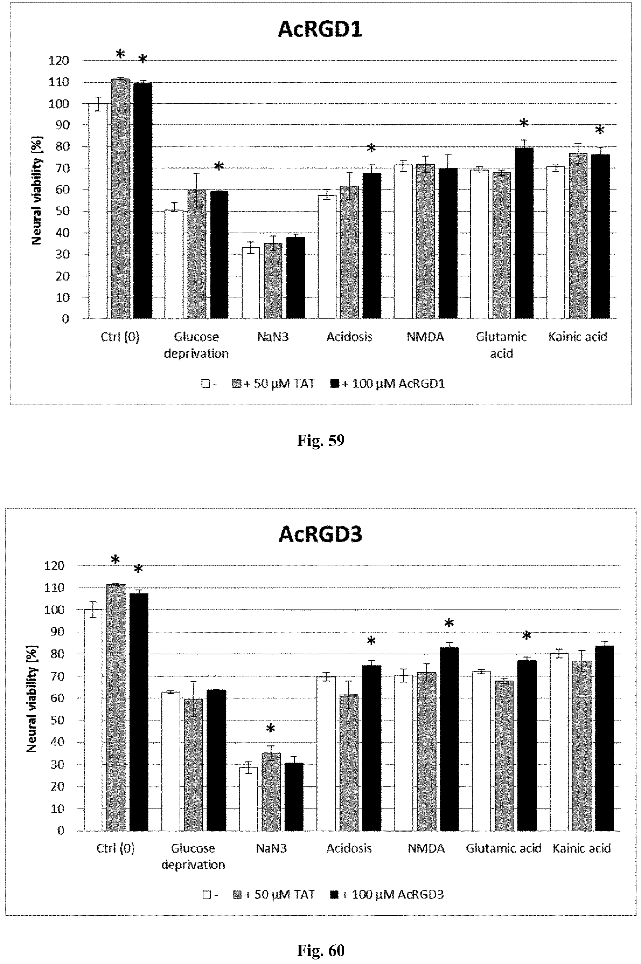

**Fig. 59** - presents activity of AcRGD1 in a multifactorial *in vitro* model of neurological injury.

**Fig. 60** - presents activity of AcRGD3 in a multifactorial *in vitro* model of neurological injury.

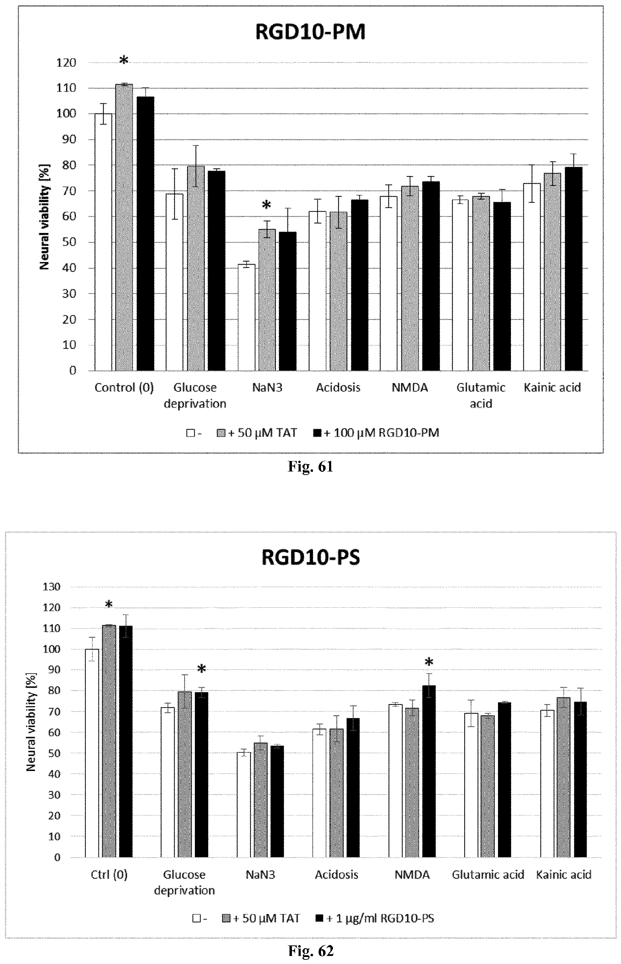

**Fig. 61** - presents activity of RGD10-PM in a multifactorial *in vitro* model of neurological injury.

**Fig. 62** - presents activity of RGD10-PS in a multifactorial *in vitro* model of neurological injury.

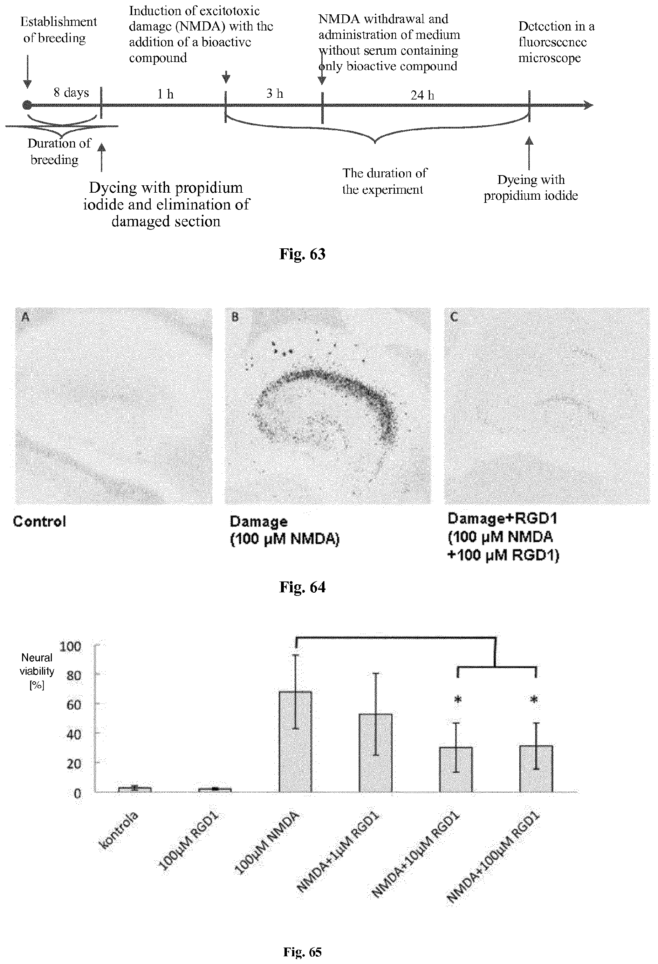

**Fig. 63** - presents a scheme of *ex vivo* organotypic hippocampal slice cultures and an experiment to evaluate neuroprotective efficacy of bioactive compounds. The 8-day-old culture was treated with propidium iodide (PI) and pre-characterized by means of fluorescence microscopy to eliminate defective slices. After PI removal, the medium was replaced by a fresh one, containing both an excitotoxicity-inducer (100 $\mu$M NMDA, which was removed after 3 hours) and a bioactive agent, which was present in the medium until the end of the experiment. The final fluorescent visualization was conducted 24 hours after the beginning of the experiment and the PI pre-treatment. The percentage of dead cells (PI staining-positive) was calculated from the formula:

$$\% \text{ PI positive cells} = (\text{fluorescence intensity of a slice [FI]/ max [FI]}) \times 100\%,$$

whereas maximal damage, i.e. max [FI], was acquired upon treatment of the hippocampal slices with 250 $\mu$M NMDA.

**Fig. 64** - presents a direct neuroprotective (injury-reducing) effect of peptide RGD1 on living *ex vivo* rat brain slices. The figure presents representative living slices containing crucial brain structure - the hippocampus - in a organotypic culture, treated with a massive neuronal death-inducer (100 $\mu$M NMDA), in which the dead neurons are shown as black dots after PI staining. Original images from a fluorescence microscope were reproduced into negative versions:

A) control slice example (incubated with saline instead of NMDA); B) an example of a slice obtained 24 hours after a 3 hour-long exposure to 100 $\mu$M NMDA injury-inducer;

C) an example of a slice exposed for 3 hours to 100 $\mu$M NMDA injury-inducer and, afterwards, incubated for 24 hours in 100 $\mu$M RGD1 solution. The image shows a strong confirmation of neuroprotective (injury-reducing) activity of the peptide RGD1 in the living rat brain slices in a model of neurological (excitotoxic) injury.

**Fig. 65** - presents quantitative, comparable analysis of cellular mortality in living *ex vivo* rat brain slices treated with an injury-inducer (100 $\mu$M NMDA), and after the treatment with neuroprotective peptide RGD1 in different concentrations. The figure also quantitatively shows no negative influence of RGD1 (100 $\mu$M) on cell mortality, in comparison to a control culture containing neither peptide nor injury-inducer. Attention should be paid to a significantly-relevant mortality difference between the NMDA-treated group and groups treated with 10 and 100 $\mu$M RGD1 (Tukey's Multiple Comparison Test, *p*-value < 0.05).

The invention is further illustrated by the following non- limiting examples.

## Example 1

### Chemical synthesis of RGD peptides

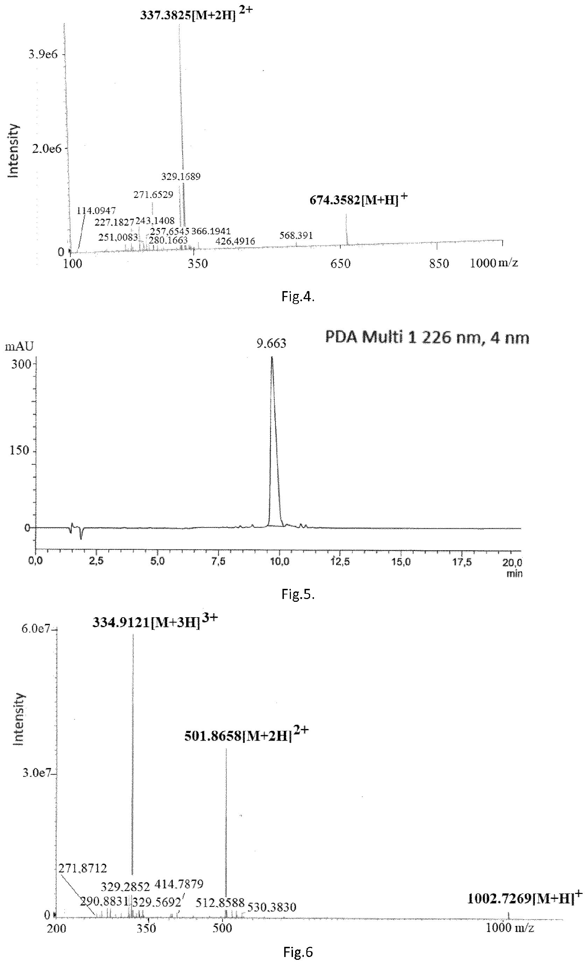

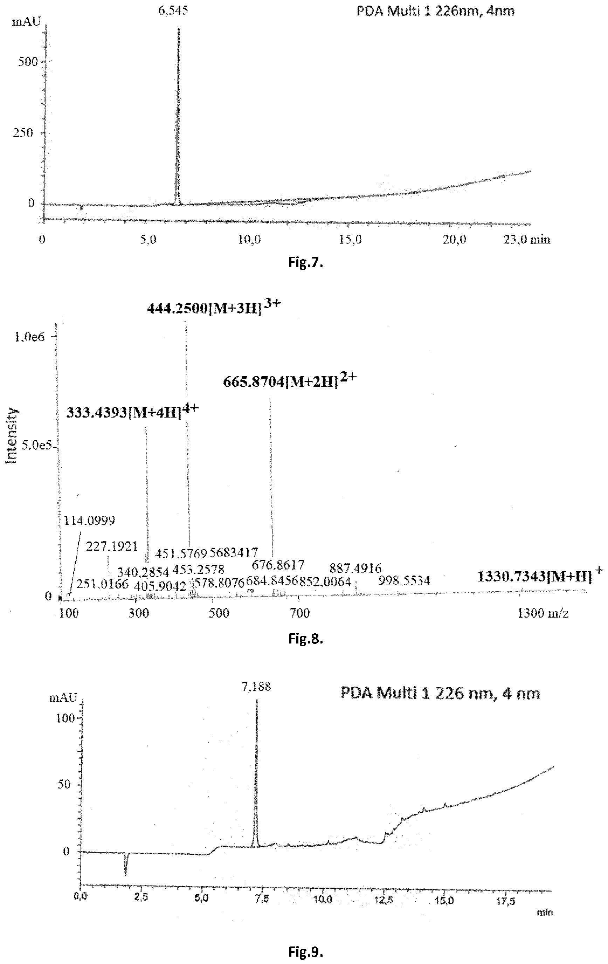

The peptides RGD1, RGD2, RGD3, RGD4, RGD10-PM, AcRGD1 and AcRGD3 were synthesized following the standard Fmoc procedure of solid phase peptide synthesis on TentaGel S RAM resin using an automatic peptide synthesizer. During the synthesis of acetylated peptide analogues suitable peptidyl resins were acetylated with N-acetylimidazole in dimethylformamide (DMF) for one hour. Peptides were cleaved from peptidyl resins with 98% trifluoroacetic acid (TFA) and precipitated with cold diethyl ether. The crude peptides were purified by preparative HPLC using a Maisch C-18 250 x 40 mm column, particle size 10 $\mu$m. A linear gradient of ACN with a 0.08% ingredient of TFA was used. Solvent flow was 25 ml/min. Fractions were analyzed by analytical HPLC using a Phenomenex Kinetex XB-C18 150 x 4.6 mm column, particle size 5 $\mu$m. For all peptides a gradient of 0-20% of ACN (with 0.08% TFA) for 30 min, with a flow of 1ml/min, was used. After purification the peptides were lyophilized. Following this they were dissolved in 0.01 M HCl to exchange trifluoroacetate ions with chloride ions and lyophilized again. The purified peptides were characterized by analytical HPLC and ESI mass spectrometry. The characteristics of the obtained peptides are shown in Figs. 1-15. HPLC chromatograms show the purity of the peptides. All peptides synthesized by solid-phase synthesis were > 95% pure. Mass spectrometry (ESI MS) shows the correct identity of the peptide structure and its homogeneity. The mass spectra show signals corresponding to pseudomolecular ions (M+H)+ or multiply charged (+2, +3, +4) ions of a given peptide.

The above process was used to produce active compounds containing the sequence of RGD (Arg-Gly-Asp) in the form of:

a. the *C*-amide of RGD tripeptide (RGD-NH$_2$, labeled as RGD1);
b. the *C*-amide of a hexapeptide in the form of a doubled RGD sequence (RGDRGD-NH$_2$, labeled as RGD2),
c. the *C*-amide of a nonapeptide in the form of a triplicated RGD sequence (RGDRGDRGD-NH$_2$, labeled as RGD3),
d. the *C*-amide of a dodecanopeptide in the form of a four-fold sequence of RGD (RGDRGDRGDRGD-NH$_2$, labeled as RGD4),
e. *N*-acetylated RGD1 (Ac-RGD-NH$_2$) and RGD3 (Ac-RGDRGDRGD-NH$_2$), labeled as AcRGD1 and AcRGD3, respectively.
f. A peptide containing a 10-fold RGD sequence-(RGDRGDRGDRGDRGDRGDRGDRGDRGDRGD-NH$_2$, labeled as RGD10-PM), obtained by the chemical synthesis method.

## Example 2

This example shows how to obtain concatemeric, poly-signal proteins of RGD peptide derivatives to be used for pro-regenerative purposes. This section also discusses DNA vectors for the implementation of this method, and the poly-signal RGD protein.

A subject of the present invention is a way to obtain a poly-epitope (=poly-signal, as the RGD epitope is a pro-regeneration signal as well) protein consisting of polymerized RGD peptides and its derivatives, with pro-regenerative properties, and DNA vectors for the implementation of this method. A subject of this invention is also biosynthesized and purified poly-epitope proteins, procedures for their isolation and monomeric peptide units used for construction of poly-peptide protein structures. Proteins obtained in accordance with the present invention can find a wide range of applications in the regeneration of cells, tissues and organs. Poly-epitope proteins may contain multimers of peptide hormones or biologically active fragments of signal proteins, and are capable of stimulating processes of tissue regeneration. Poly-epitope proteins, placed in a wound, can directly stimulate processes of regeneration or, thanks to autodegradation or activity proteases found in wounds, gradually release the biologically active peptides, thus stimulating regeneration.

Construction of poly-epitope proteins was performed by applying bioinformatic methods for the design of optimized genes, chemical synthesis of coding DNA, molecular cloning coding DNA into the bacteria *Escherichia coli* and use of proprietary technology involving a directional, vector-enzymatic amplification of DNA segment with an Open Reading Frame encoding, without interruption, for a concatemeric, poly-epitope protein. The technology also describes the design of biological nanostructures in the form of bacteriophages genetically coated with poly-epitope proteins. The above mentioned technology is the subject of the patent PL 228341 B (BioVentures Institute Sp. z o.o.).

## Example 3

This example shows a scheme for the implementation of the method according to the present invention.
A bioactive RGD peptide, selected for its injury protective and/or a neuroprotective properties, underwent further multiplication procedures at the DNA level, and as a result of the translation, the obtained concatemeric gene was also expressed at the level of *in vivo* translation in the form of an artifial, concatemeric protein. The RGD peptide was reproduced in 2 variants: (i) RGD and (ii) RGDGG. The variant RGDGG contains 2 additional G amino acid residues. It was assumed that these 2 repeated G residues form unstructured segments of the polypeptide separating the segments of RGD, thus stimulating the RGD motif to form a bioactive structure with more exposure and leading to its greater accessibility in solution. Multiplication of the peptide motifs was achieved in 2 ways: (i) at the level of chemical synthesis and (ii) using the above described, patented, DNA fragment amplification technology involving enzymatic-vector multiplication *in vitro*/*in vivo* [Skowron et.al. 2014-2017]. Using chemical synthesis it is typically only possible to generate 5-10 copies of the DNA in the concatemer segment, while the enzymatic-vector multiplication system allows the creation of as many as 500 copies of the DNA in a concatemeric segment. Construction of the poly-epitope RGD protein has been conducted using a combination of both methods, in order to obtain the spectrum of cloned concatemers with different numbers of copies of monomer units. In approach (i) we applied modular design to obtain fusion RGD and RGDGG proteins containing the following components: (a) the His6 tag - six Histidine residues on the N-terminus to be used in high-performance isolation methods of the recombinant protein using metalaffinity chromatography; (b) the c-Myc tag to be used in Western blot detection of the protein with dedicated anti-c-Myc antibodies; (c) the amino acid WYY tag, to facilitate the detection of the recombinant protein using UV light and Coomassie Blue staining of electrophoretic gels; and, finally (d) ubiquitin on the N-terminus of the proposed protein, after the His6 tag, the WYY tag and the c-Myc tag. Ubiquitin is used, in this case, as a fusion partner in order to improve the level of protein expression, as well as to decrease protein toxicity, and to improve its *in vivo* stability. Ubiquitin can optionally be proteolytically cleaved from the isolated fusion proteins using recombinant, His6 tag-carrying, Yeast Ubiquitin Hydrolase (hereinafter referred to as YUH1). This system allows for the removal of ubiquitin and the preceding amino acid sequences without leaving additional amino acid residues in the protein domain consisting of poly-epitope proteins; (e) last component of the fusion proteins was poly-epitope 10-mer RGD or 10-mer RGDGG. In approach (ii), amplification of the DNA section coding for 10-mer RGD and 10-mer RGDGG was obtained using the amplification/expression vector, SapI enzyme and DNA ligase, as disclosed in the patent and patent applications describing the technology [Skowron at.el. 2014-2017]. The obtained higher-order concatemers were cloned into: (1) the SapI site of the amplification/expression vector, creating a fusion poly-epitope protein with a His6 tag and (2) the amplification/expression vector, with a previously cloned synthetic DNA fragment encompassing a designed gene coding for a [His6-c-Myc-WYY-ubiquitin-poly-epitope] protein. This vector has internal SapI sites, the arrangement of which is designed in such a way that DNA cut with SapI releases the poly-epitope protein, leaving the cassette of His6-c-Myc-WYY-ubiquitin in the vector. After cloning of higher order concatemers, they are merged with the His6-c-Myc-WYY-ubiquitin cassette, creating a new fusion protein encompassing the cassette and an increased number of copies of the original units of the RGDGG or RGD poly-epitope protein. It needs to be noted, that the active sequence of the peptide or poly-epitope, composed often times duplicated RGD sequence contains additional N-and C-terminal flanking sequences resulting from biotechnological processes of the amplification-expression vector and protein design). This obtained poly-epitope in biotechnological process from bacterial expression system has a sequence IMSRSSPRGDRGDRGDRGDRGDRGDRGDRGDRGDPRRA and is designated as RGD10-PS.

## Example 4

This describes design and cloning of molecular fusion proteins containing ubiquitin, peptide tags and the poly-epitope proteins RGD10 and RGDGG10.

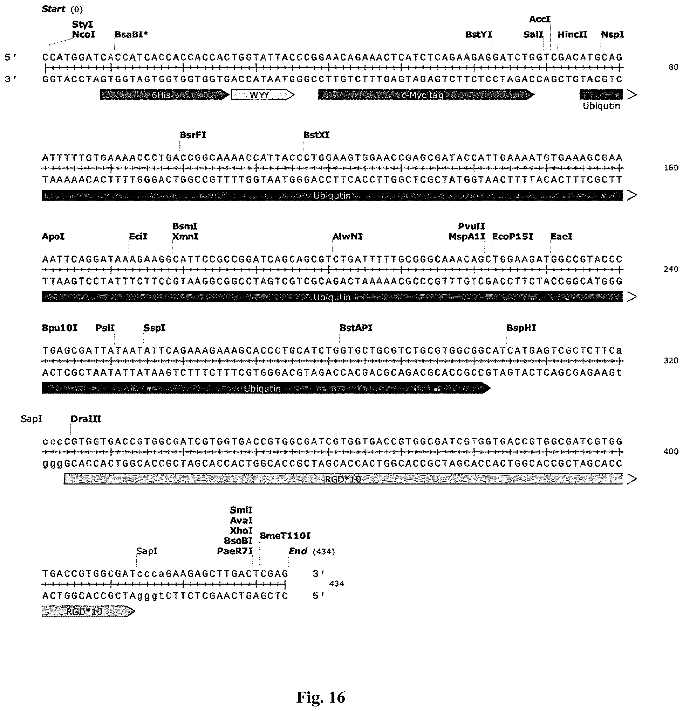

An example of the implementation of this process, presented in Figs. 16, 17, 18 and 19, shows DNA cassettes containing the amplification module SapI and optimized, in terms of codon usage, for bacteria *Escherichia coli,* includes synthetic genes encoding the following fusion proteins: [His6-c-Myc-WYY-ubiquitin-poly-epitope RGD10] (DNA revealed as Seq. 1, the encoded protein revealed as Seq.2) and [His6-c-Myc-WYY-ubiquitin-poly-epitope RGDGG10] (DNA revealed as Seq. 3, the encoded protein revealed as Seq. 4).

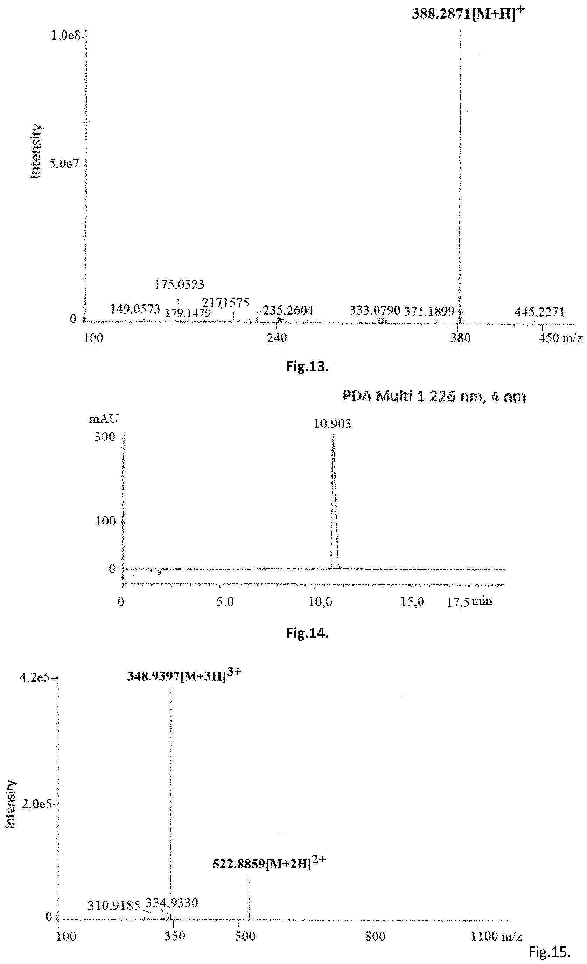

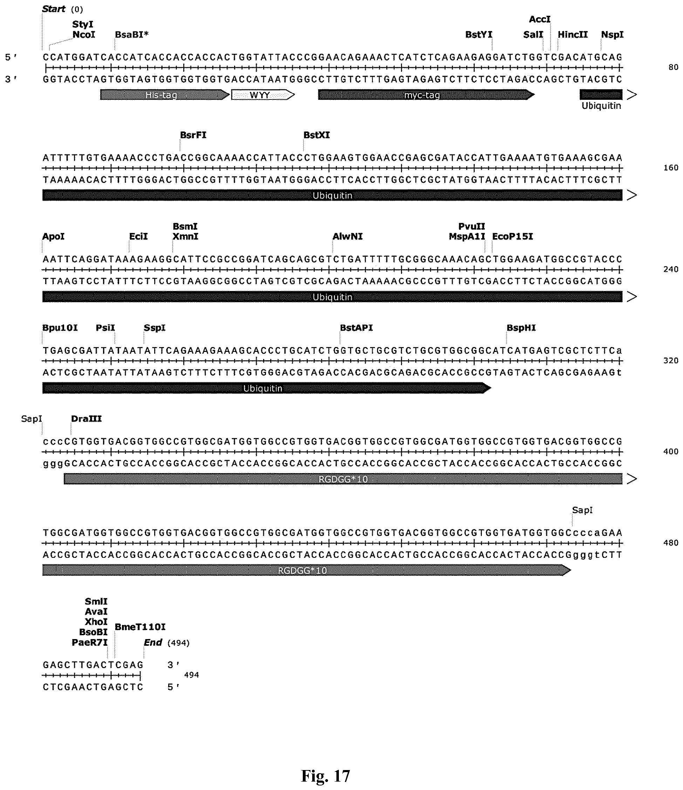

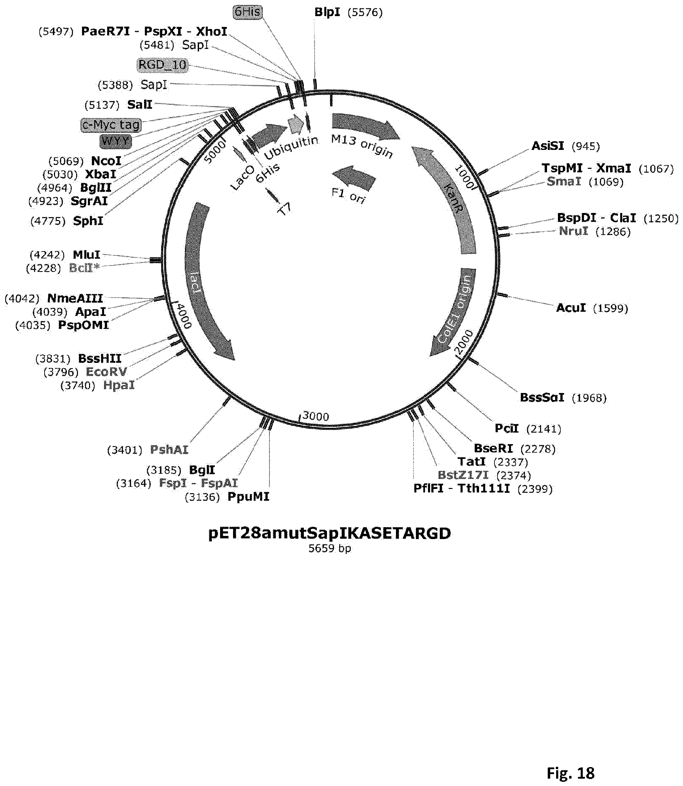

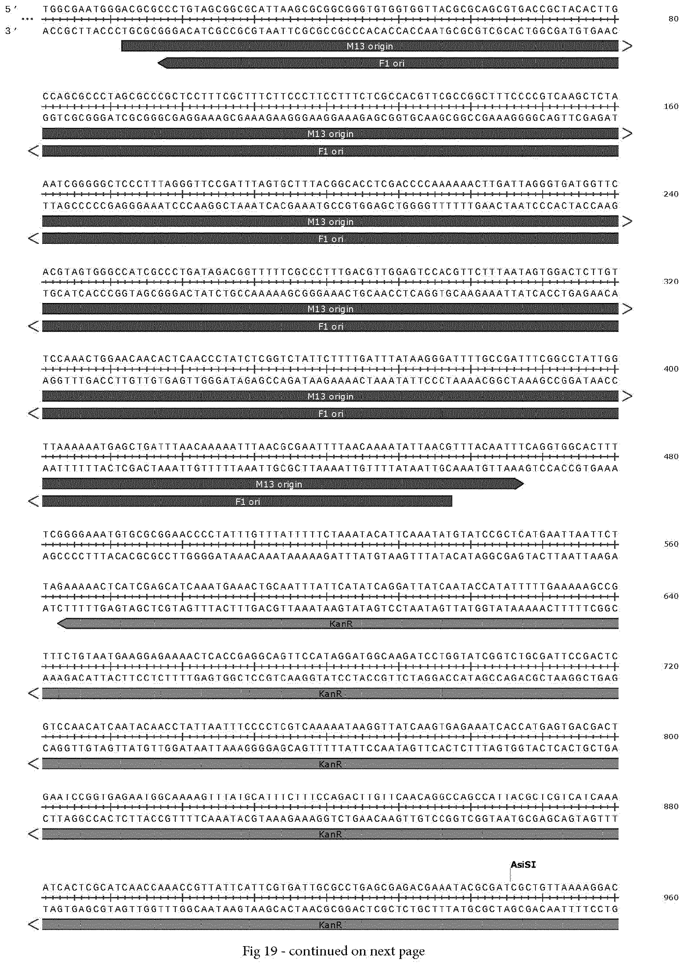

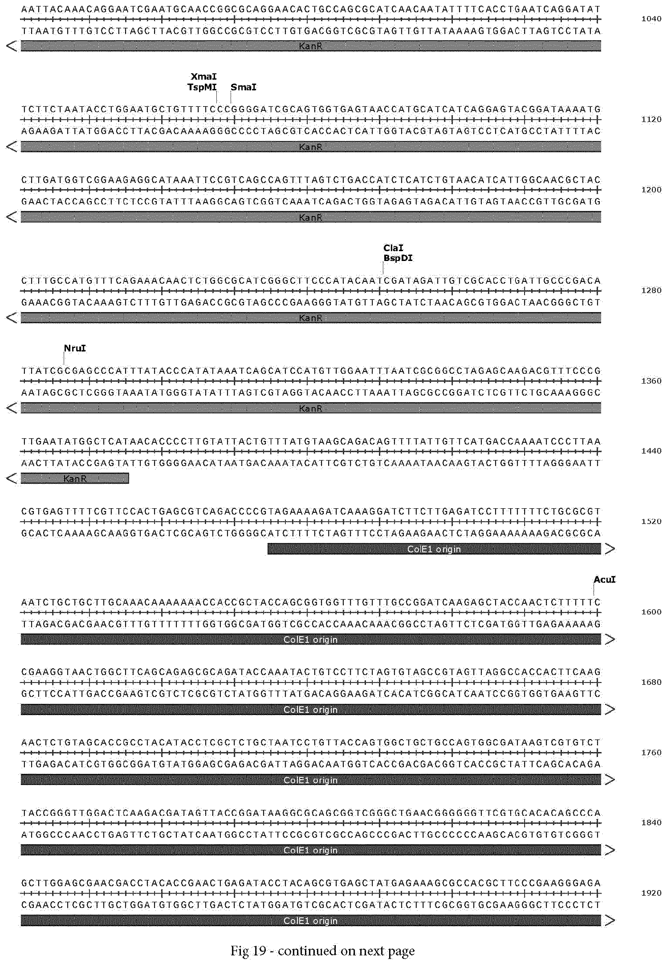

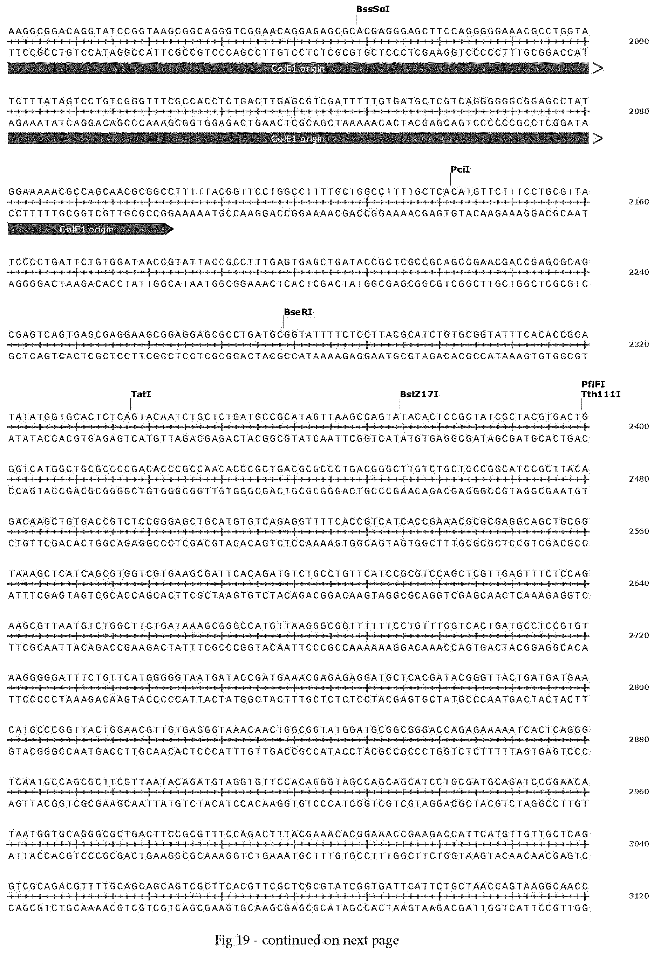

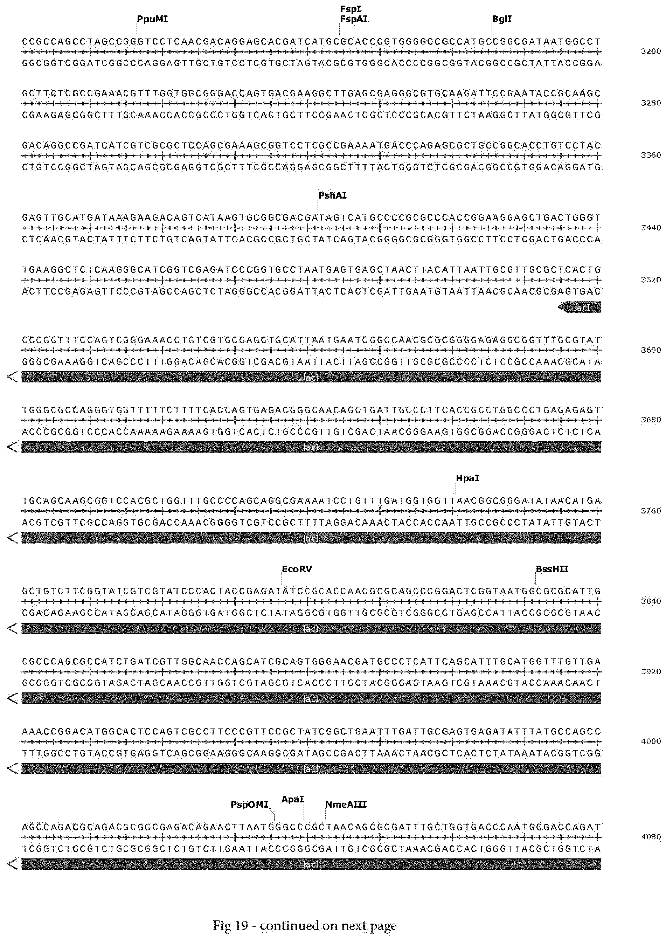

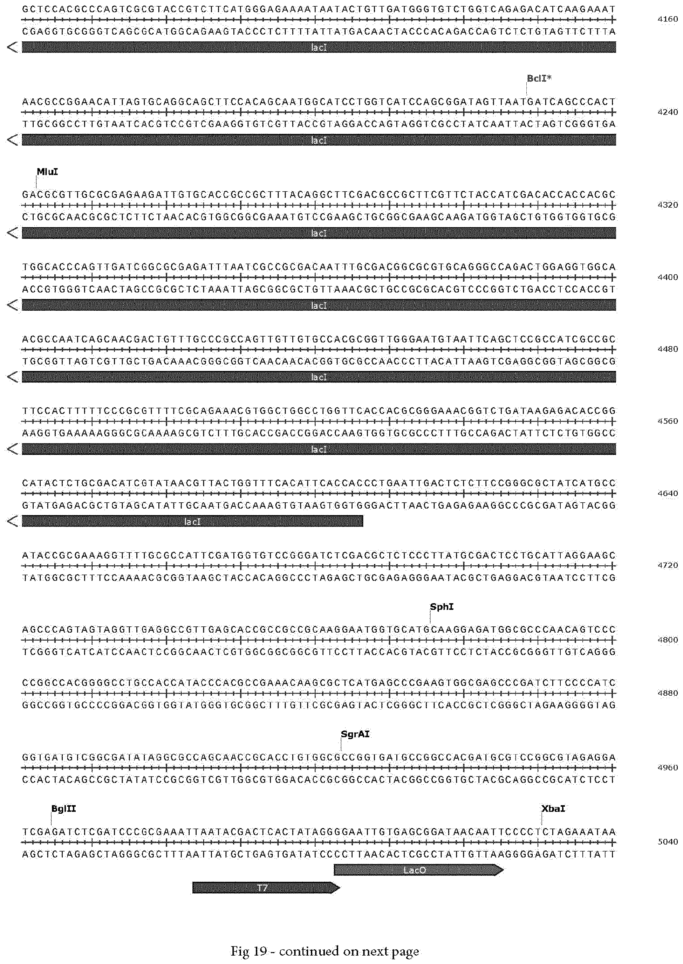

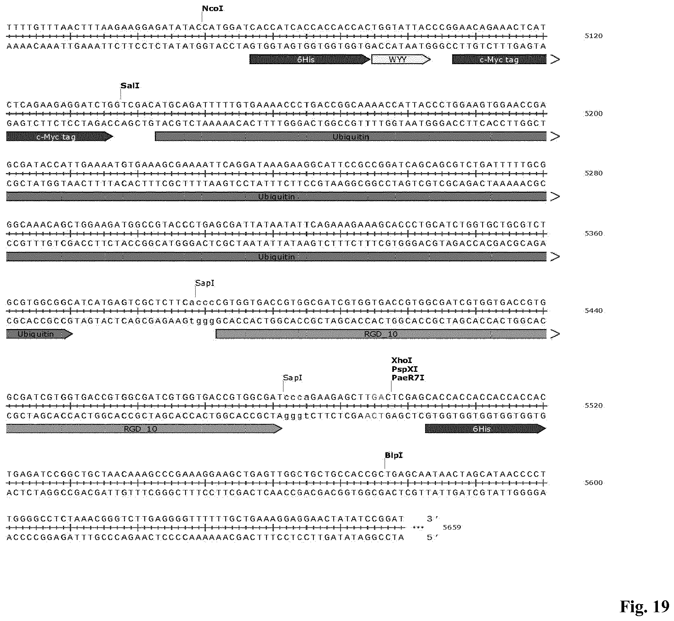

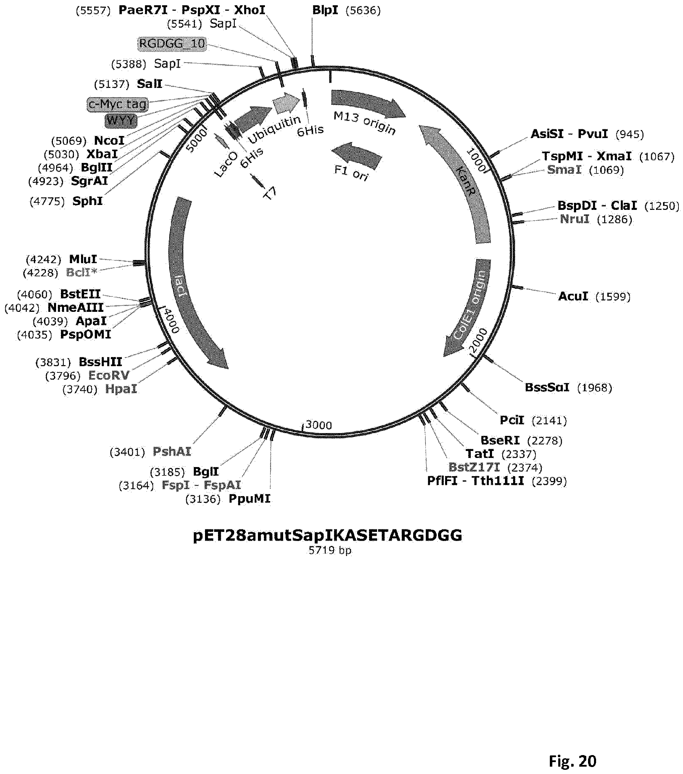

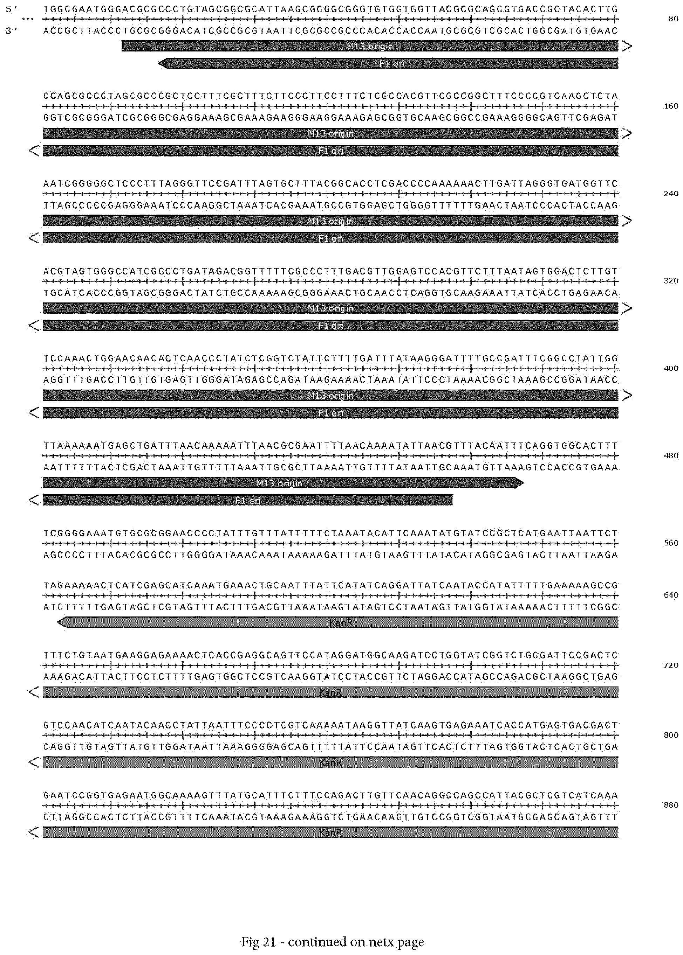

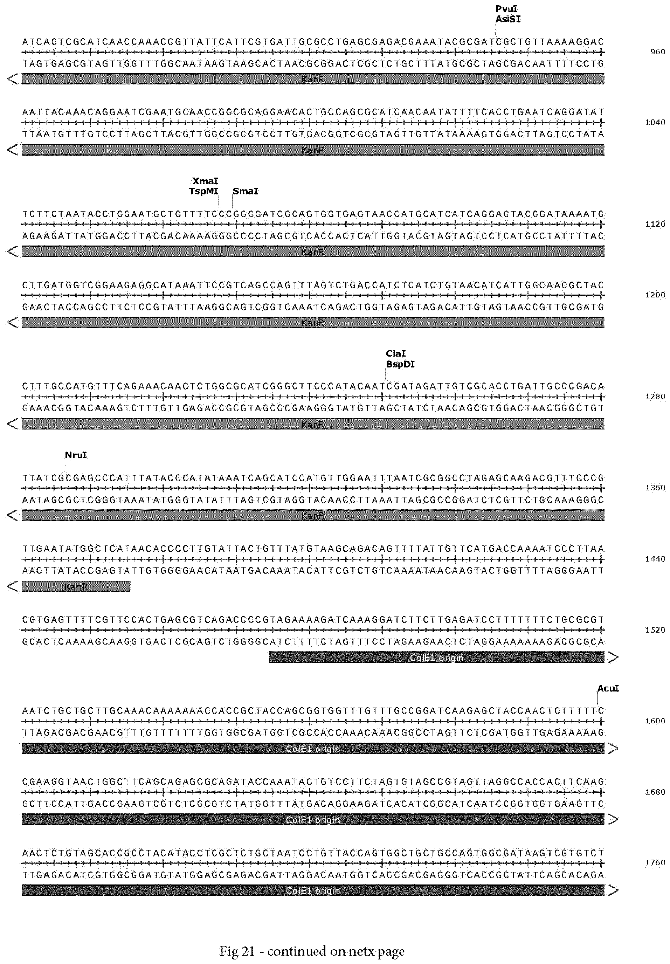

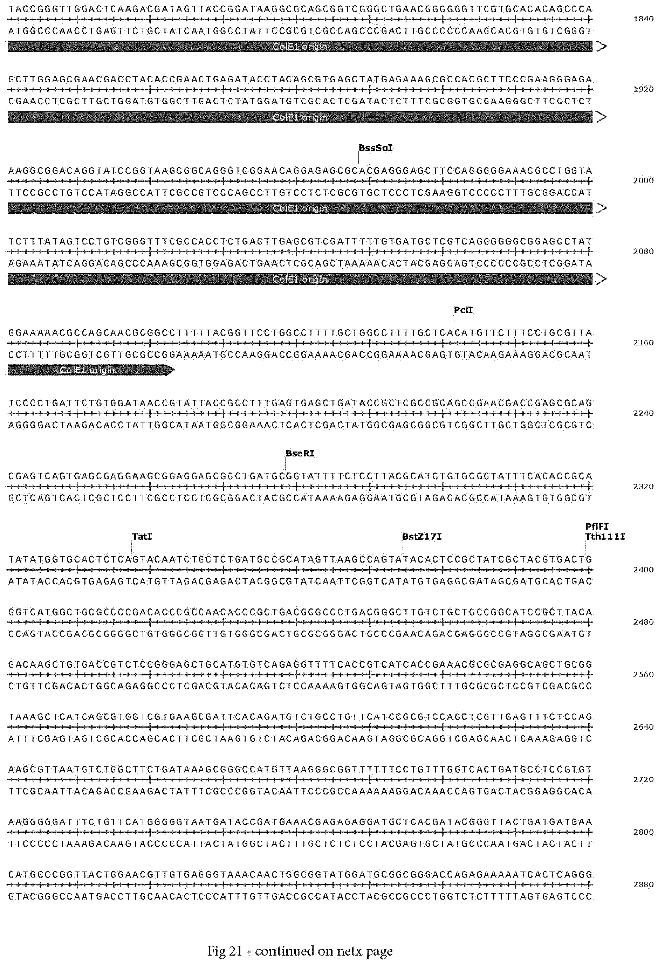

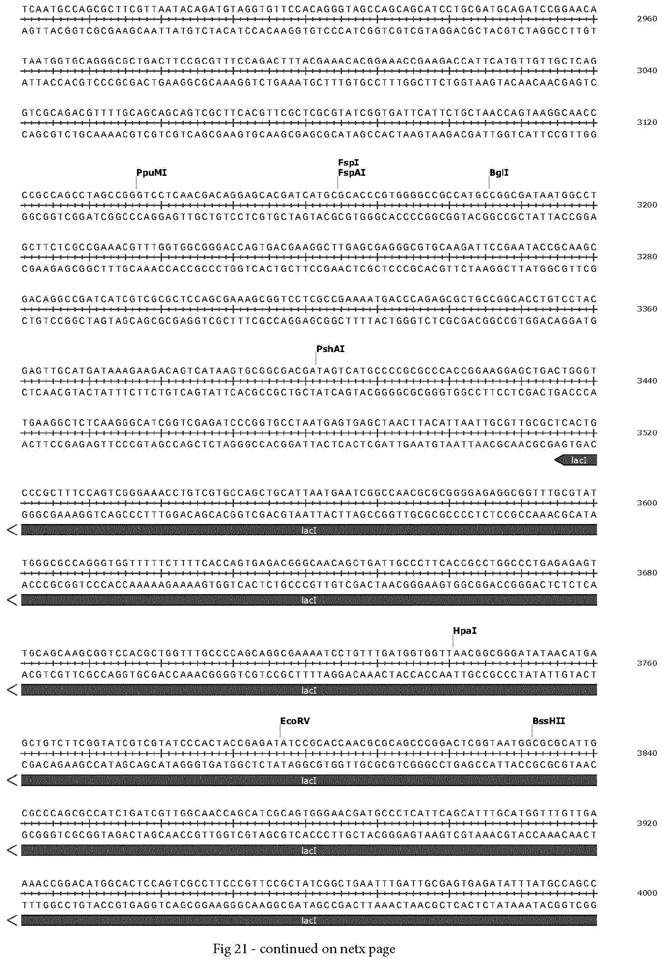

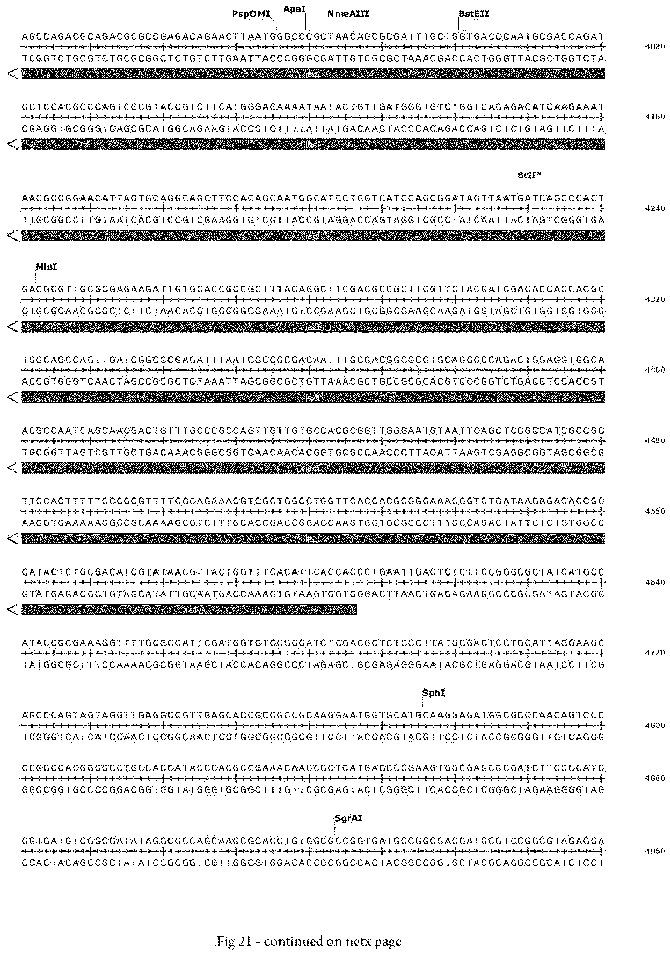

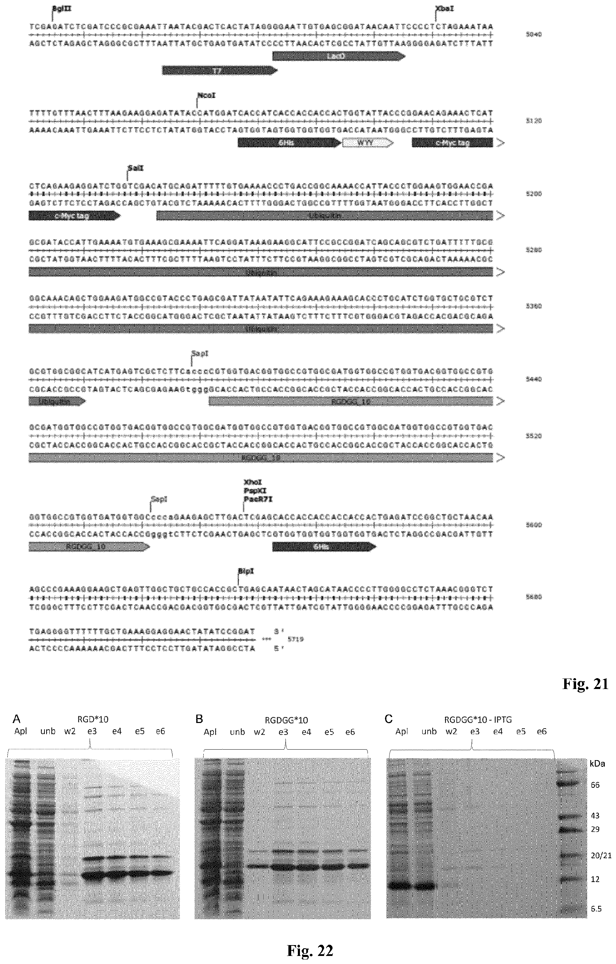

In an example of the implementation of this process, presented in Figs. 20, 21, 22 and 23, synthetic genes were cloned into the commercial vector pET28a(+), which contains a strong, hybrid transcription promoter of T7-lac, and transforming the pET28a(+) vector into an amplification/expression vector, which carries the designed synthetic genes in a configuration ensuring an optimal distance from the promoter T7-lac and the start of the translation signals, Ribosome Binding Site and codon ATG. Laboratory expression strain *Escherichia coli* BL21 (DE3) was used as a host in the biosynthesis of the synthetic constructs. Fig. 15 displays the nucleotide sequence of the expression cassette along with the selected functional areas, containing genes encoding for [His6-c-Myc-WYY-ubiquitin-poly-epitope-RGD10] and the amino acid sequence of the expressed fusion protein. Fig. 16 displays the nucleotide sequence of the expression cassette along with the selected functional areas, containing genes encoding for [His6-c-Myc-WYY-ubiquitin-poly-epitope-RGDGG10] and the amino acid sequence of the expressed fusion protein. In Fig. 17 the cassettes have undergone digestion using NcoI and XhoI enzymes to allow directional cloning of the DNA segment into the pET28a(+)vector also opened with NcoI and XhoI enzymes. Vector DNA and DNA encoding for the cassette were combined using T4 bacteriophage ligase in a manner that allowed for precise combination of the signals for the start of transcription and translation of the pET28a(+) vector with Open Frame Read (ORF) of the following fusion proteins: [His6-c-Myc-WYY-ubiquitin-poly-epitope-RGD10] and [His6-c-Myc-WYY-ubiquitin-poly-epitope-RGDGG10]. The obtained ligation mixture was used for transformation of the bacteria *Escherichia coli* DH5a. The obtained clones were used for plasmid DNA isolation. This DNA was subsequently analyzed by restriction endonuclease enzymes and DNA sequencing. Plasmids pET28amutSapIKASETARGD and pET28amutSapIKASETARGDGG, which show the correct DNA sequence, unchanged relative to the designed *in silico* cassettes (and therefore do not containing unwanted mutation), with gene sequences encoding the fusion proteins [His6-c-Myc-WYY-ubiquitin-poly-epitope-RGD10] and [His6-c-Myc-WYY-ubiquitin-poly-epitope-RGDGG10], were selected. They were subsequently transformed into a strain dedicated to pET28a(+) expression, obtaining clones of *Escherichia coli* BL21Star (DE3) [pET28amutSapIKASETARGD] and *Escherichia coli* BL21Star (DE3) [pET28amutSapIKASETARGDGG]. Figs. 18 and 19 display the genetic map of the expression construct, along with the nucleotide and amino acid sequence of plasmid pET28amutSapIKASETARGD, with functional modules marked. Figs. 20 and 21 display the genetic map of the expression construct, along with the nucleotide and amino acid sequence of plasmid pET28amutSapIKASETARGDGG, with marked functional modules.

## Example 5

Expression of the genes cloned into *Escherichia coli* BL21Star (DE3), which encode for the fusion proteins [His6-c-Myc-WYY-ubiquitin-poly-epitope RGD10] and [His6-c-Myc-WYY-ubiquitin-poly-epitope RGDGG10].

The clones *Escherichia coli* BL21Star (DE3) [pET28amutSapIKASETARGD] and *Escherichia coli* BL21Star (DE3) [pET28amutSapIKASETARGDGG] undergo test expression by means of experimental transformation into *Escherichia coli* BL21Star (DE3), which was then cultured at a temperature of 30°C on a 2xY microbiological media modified for high expression. The medium had the following composition for 1 L (distilled water): Tryptone, 16 g; yeast extract, 10 g; NaCl 5 g, pH 7.2 adjusted with NaOH. Directly before use the medium was supplemented with 20 ml of sterile 1 M glucose. After bacterial culture reaching an optical density of OD = 1, gene expression was induced using 1 mM of Isopropyl β-D-1-thiogalactopyranoside (IPTG), and bacteria cultivation was then carried out for 4 hours. Genetic expression experiments were carried out on a pilot scale of 50 ml culture medium, and then a preparative scale of 1 L of culture medium. In the example of the implementation presented in Fig. 22, the proteins [His6-c-Myc-WYY-ubiquitin-poly-epitope RGD10] and [His6-c-Myc-WYY-ubiquitin-poly-epitope RGDGG10] were isolated from the recombinant *Escherichia coli* cells, using the following steps: (i) the cells were spun-down for 20 minutes, with an angular acceleration of 12000 g, at 4°C, then the cells were re-suspended in cell lysis buffer [50 mM sodium phosphate, pH 7.4, 300 mM NaCl, 0.75 mg/ml egg lysozyme, 2.5 μg/ml DNase and 0.1 mM protease inhibitor PMSF] in an amount of 2% of the output volume of the culture, then incubated for 10 minutes at a temperature of 22°C; (ii) the cooled cell suspension was subjected to ultrasonic disintegration in an ice bath, and cell debris were separated using centrifugation; (iii) the obtained supernatant was applied to the affinity chromatography column Talon [GE Healthcare Life Sciences, USA], containing immobilized $Co^{2+}$ ions. The fusion proteins [His6-c-Myc-WYY-ubiquitin-poly-epitope RGD10] and [His6-c-Myc-WYY-ubiquitin-poly-epitope RGDGG10] contain His6 tag, which allows highly specific binding of these proteins to the Talon columns, which in turns allows for a high degree of purification. The Talon column, equilibrated with the buffer T [50 mM $Na_2HPO_4$, 1.8 mM $KH_2PO_4$, pH 7.4, 300 mM NaCl] and containing the adsorbed fusion protein was rinsed with 10-column volumes of the buffer W [50 mM sodium phosphate, pH 7.4, 300 mM NaCl; 10 mM imidazole], and then the protein was eluted with 10-column volumes of buffer E [50 mM $Na_2HPO_4$, 1.8 mM $KH_2PO_4$, pH 7.4, 300 mM NaCl; 150 mM imidazole]. See Fig. 22A, Fig. 22B and Fig. 22C. Comparison of both purifications, revealed in figure 22B and 22C shows massive expression of the recombinant protein [His6-c-Myc-WYY-ubiquitin-poly-epitope RGDGG10] with a bioinformatically predicted molecular mass of 17.3 kDa, in accordance with the disclosed amino acid sequence (Seq. 4), and that this protein was absent, or present in only trace amounts, in the control preparation. The isolated fusion protein was frozen in liquid nitrogen and then stored at -80 °C. For the final verification of the identity of the recombinant proteins [His6-c-Myc-WYY-ubiquitin-poly-epitope RGD10] and [His6-c-Myc-WYY-ubiquitini-poly-epitope RGDGG10] were analyzed using antibodies specific to the His6 tag and the c-Myc tag, using a Western Blot procedure. In the example of the implementation presented in Figs. 23 and 24, the protein [His6-c-Myc-WYY-ubiquitin-poly-epitope RGD10] and protein [His6-c-Myc-WYY-ubiquitin-poly-epitope RGDGG10], were subjected to SDS-PAGE electrophoresis, on a 12.5% Tris-Tricine polyacrylamide gel, and then transferred using an electrotransfer method in buffer TW [25 mM Tris, 192 mM glycine, pH 8.3, 20% methanol, 0.04% SDS] onto a PVDF membrane. After the completion of the transfer, the membrane was blocked in TBST buffer [50 mM Tris-HCl, pH 7.6, 150 mM NaCl, 0.05% Tween 20], with the addition of 5% of powdered skimmed milk by gentle shaking for 2 hours at a temperature of 4°C, then rinsed with TBST buffer by shaking for 10 minutes and subjected to reaction with primary, anti-His6, rabbit antibodies (VTTF SP. z o.o., Poland). The antibody was used in a dilution of 1: 5000 for 18 hours at 4°C, with shaking in TBST buffer. Unreacted antibodies were removed by washing the membrane 3 times with TBST, shaking it each time for 10 minutes. Bound rabbit antibody was revealed by secondary goat anti-rabbit antibodies fused with alkaline phosphatase, used at a dilution of 1: 10000 in TBST buffer, while shaking the membrane for 1 hour at 22°C. Unreacted anti-rabbit antibodies were removed by washing the membrane 3 times with TBST, shaking it each time for 10 minutes. In order to visualize the proteins, the membrane was subjected to a reaction with chromogenic substrates Sigma fast BCiP/NBT (Sigma) for alkaline phosphatase, developed by way of producing an insoluble violet-blue product at the site of contact with bound goat antibodies. In Fig. 23, Line M shows the pattern of the molecular weight standard PageRuler Plus Prestained Protein Ladder (ThermoFisher, USA); line K shows control protein with a molecular weight of 130 kDa, containing the His6 tag; line APL shows cell lysate applied to the Talon column; line W2 shows column wash with buffer W; and lines e3, e4 and e5 show protein elution from the Talon column with buffer E. A chromatogenic reaction can be observed for the protein bands, corresponding to the expected molecular weight of proteins [His6-c-Myc-WYY-ubiquitin-poly-epitope RGD10] and [His6-c-Myc-WYY-ubiquitin-poly-epitope RGDGG10]. In the example of the implementation presented on Fig. 24, a similar procedure as the one presented in Fig. 23 was performed, with the exception of the application of the primary anti-c-Myc rabbit antibody at a dilution of 1: 1000 (Novus Biologicals, USA). In Fig. 24 Line M shows the pattern of the molecular weight standard, APL, cell lysate applied to the Talon column; line W2 shows column wash with buffer W; lines e3 and e4 show His6 and protein elution from Talon column with buffer E. A chromatogenic reaction can be observed for the protein bands, corresponding to the expected molecular weight of protein [His6-c-Myc-WYY-ubiquitin-poly-epitope RGDGG10], and a small amount of protein dimers visible due to the higher sensitivity of the c-Myc antibody. The presence of dimers is probably caused by the large amount of G residues repeated at regular intervals in the RGD and RGDGG motifs, which probably facilitates intramolecular interactions. This property is desirable in the case of poly-epitope proteins used in regenerative medicine, because the formation of such a supra-molecular structure reduces the speed of diffusion of poly-epitope protein complexes outside of the drug application area.

## Example 6

This example describes proteolytic removal of the ubiquitin and peptide tags from the poly-epitope proteins, isolation of these proteins and analysis of the isolated poly-epitope proteins.

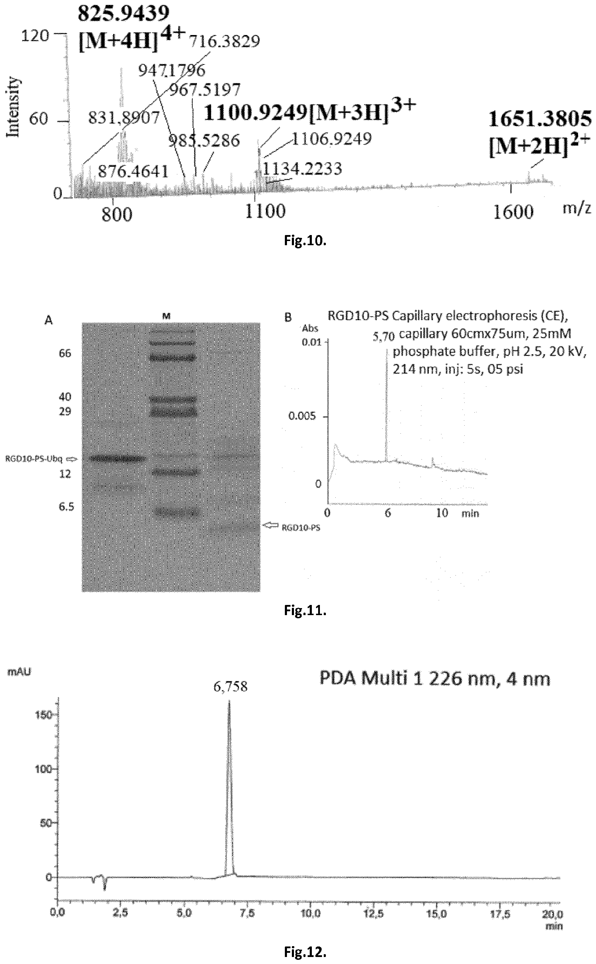

[His6-c-Myc-WYY-ubiquitin-poly-epitope RGD10] and [His6-c-Myc-WYY-ubiquitin-poly-epitope RGDGG10] proteins were purified as described in example 3 and dialyzed in the presence of theYUH1 reaction buffer [50 mM potassium phosphate, pH 7.4, 300 mM NaCl], in order to carry the cleavage of the polypeptide His6-c-Myc-WYY-ubiquitin from the poly-epitope proteins. An example of this procedure, presented in Fig. 25, reveals the results of the digestion of [His6-c-Myc-WYY-ubiquitin-poly-epitope RGDGG10] protein by the YUH1 enzyme, using an enzyme: substrate ration of 1: 10. The digestion reaction was carried out for 16 hours at 4°C. Line M shows protein molecular weight standards; line 1, the protein [His6-c-Myc-WYY-ubiquitin-poly-epitope RGDGG10]; and line 2, the protein [His6-c-Myc-WYY-ubiquitin-poly-epitope RGDGG10] digested with YUH1. Line 2 points to the efficient digestion of the fusion protein [His6-c-Myc-WYY-ubiquitin-poly-epitope RGDGG10]. This assessment is based on the disappearance of the full length form and the appearance of the N-terminal domain His6-c-Myc-WYY-ubiquitin. Quantitative assessment of the released poly-epitope RGDGG10 protein and poly-epitope RGD10 protein is impossible due to the lack of aromatic amino acid residues in the sequences of those proteins, as the presence of these is necessary for efficient staining of the proteins by Coomassie Brilliant Blue after protein separation on SDS-PAGE. Therefore, the poly-epitope proteins are not directly observable on SDS-PAGE gels. When released by YUH1, the poly-epitope RGDGG10 protein, with a molecular weight of 5.5 kDa, and the poly-epitope RGD10 protein, with a molecular weight of 4.4 kDa, were purified using the following steps: (i) affinity chromatography on Talon columns, using buffer T [50 mM sodium phosphate, pH 7.4, 300 mM NaCl], to bind the cutoff N-terminal domain His6-c-Myc-WYY and unprocessed fusion protein portion; (ii) fractionation by sequential ultrafiltration using spin columns with filter cut-off 10 kDa (Vivaspin Turbo 15, Sartorius, Germany), to remove the YUH1 enzyme with molecular weight of 26 kDa, and (iii) use of spin columns with filter cut-off 3 kDa (Amicon Ultra 0.5 mL, MWCO 3 kDa, Millipore, USA), for concentration of poly-epitope RGDGG10 protein and poly-epitope RGD10 protein. Ultrafiltration was carried out using a centrifuge with an angular acceleration of 4000 g. The isolated fusion proteins were stored: (i) by freezing in liquid nitrogen followed by long-term storage at a temperature of -80 °C, or (ii) by freeze-drying after replacing the T-buffer with buffer L [50 mM ammonium carbonate, pH 7.4]. Due to the lack of quantitative capabilities and proportional coloring of the poly-epitope protein bands of RGDGG10 and RGD10 on SDS-PAGE gels, two techniques for quantitative assessment of the proteins concentration were used, as shown in Fig.11 and Fig.26: (i) high-performance molecular filtration chromatography (SEC), carried out in PBS buffer [10 mM $Na_2HPO_4$, 1.8 mM $KH_2PO_4$, 2.7 mM KCl, 137 mM NaCl, pH 7.4] on a Superdex Peptide PC 3.2/300 column (GE Healthcare, USA) and (ii) HPLC carried out using a C18 column. Both techniques show protein purity of above 90%. The peak appearing in the 3rd minute of the elution on the C18 column (Jupiter C18, 5 mm, 300 A, Phenomenex, USA) corresponds to elution of salt present in the preparation of the poly-epitope protein.

## Example 7

This describes construction of poly-epitope proteins in the form of concatemers of a higher order: the reaction of DNA, encoding for 10mers of RGD, duplication, and molecular cloning of higher order concatemers to the amplification-expression vector.

As indicated in example 3, design of poly-epitope proteins with increased number of copies of the monomers of RGD and RGDGG requires the use of patented technology of DNA duplication and construction of concatemeric proteins (Skowron et.al 2014-2017). Fig. 27 shows a diagram of the duplication of a synthetic DNA segment encoding for a 10-mer of RGD and a 10-mer of RGDGG. DNA cassettes, coding for the poly-epitope protein revealed in Example 1, were digested by the restrictive endonuclease SapI in such a way that the poly-epitope part of the sequence was released from the vectors pET28amutSapIKASETARGD and pET28amutSapIKASETARGDGG, leaving behind a segment coding for the N-terminal domain His6-c-Myc-WYY-ubiquitin. The digestion of the DNA vectors was carried out in 50µl of buffer S [20 mM Tris-acetate, pH 7.9, 50 mM potassium acetate, magnesium acetate 10 mM, 100 µg/ml bovine albumin] at 37°C for 2 hours. The released sections encoding for 10-mer RGD and 10-mer RGDGG additionally had, on their 5' ends, single-stranded DNA sections CCC and GGG, permitting an ordered ligation of DNA fragments in only one orientation, preserving the continuity of the Open Reading Frame forming genes coding for multimers of RGDGG or RGD. Digested sections, were separated using electrophoresis in 2% agar gel, in TBE buffer (220 mM Tris, 180 mM boric acid, 5 mM EDTA, pH 8.3) and isolated from the gel using electoelution. Purified fragments were subjected to the ligation with help of the T4 bacteriophage ligase, the reaction being carried out for a time extending from 5 minutes to 2 hours, at 22°C. Ligation products were subjected to electrophoresis in 1.5 gel, in TBE buffer, and concatemers with 20 and more repeats of RGD and RGDGG were excised from the gel. DNA was isolated from the cut sections of the gel and ligated to the amplification vector pET21d+ _mutSapI, digested with endonuclease SapI. Amplification vector pET21d+_mutSapI carries a strong transcription promoter; T7-lac, translation initiation regions and an amplification module including the segment: His6 tag-SapI-SmaI-SapI.

When cloned to this vector concatemers do not form a fusion with ubiquitin and WYY, only with the His6 tag. Fig. 28 reveals the results of electrophoresis in agarose 1.5% gel, in TBE buffer, of products obtained as a result of the multiplication of DNA concatemers of poly-10-mer-RGD. The line M shows a DNA size marker of 100 bp (Thermofisher Scientific SM0321); line shows concatemers of DNA, containing the increasing number of copies of the RGD10 module.

Figs. 29, 30, 31, 32, 33, 34, 35, 36 and 37 - show genetic maps of the amplification vector constructs marked as pET21d+_mutSapIRGD_20-100, containing cloned poly-10-mer-RGD, containing 20, 30, 40, 50, 60, 70, 80, 90, 100 copies of the RGD peptide coding module, with molecular masses of 8.6 kDa, 12.0 kDa, 15.4 kDa, 18.7 kDa, 22.1 kDa, 25.5 kDa, 28.9 kDa, 32.3 kDa and 35.6 kDa.

## Example 8.

### Method of obtaining, and cultivation of, primary neural (neuronal and glial) cultures of cortical cells

Primary neural cortical cultures were obtained from Wistar rat E18.5 embryos. To this end, pregnant rat females were anesthetized and euthanized in a $CO_2$ chamber. In some cases, euthanasia was performed by isoflurane. The cortex tissue was excised from the isolated embryos and washed with HBSS buffer, supplemented with a mixture of penicillin and streptomycin, and HEPES. Afterwards, the cortex tissue was trypsinized for 10 minutes at 37 °C and homogenized mechanically in order to disintegrate the tissue to the form of a cell suspension. The cells were the centrifuged at 300 rcf for 4 minutes, resuspended once more in HBSS and quantified in a Bürker chamber in order to obtain a cell suspension of proper density. The cells were transferred to Neurobasal medium or to Neurobasal-A medium, supplemented with 1 mM sodium pyruvate, of 12.5 mM glucose concentration, supplemented with 2% B27 serum, 200 mM L-glutamine, 10 mM glutamic acid and 1% penicillin/streptomycin mixture, and seeded on 24-well poly-D-lysine plates at a concentration of 150,000-200,000 cells per well. The neural cultures (cultures of neuronal or glial cell, or a mixture thereof) were incubated for 14-15 days in a 5% $CO_2$ atmosphere (95% air, 98% humidity, 37 °C). A 14-15 day timeframe of *in vitro* culturing was maintained throughout all the experiments.

## Example 9

### Method of determination of cell characteristics in primary neural culture by means of analysis of immunocytochemical detection for the indication of cellular diversity.

In order to execute qualitative analysis of the obtained primary culture, the cells - at 1 day *in vitro* (1 DIV) and 14 days *in vitro* (14 DIV) - were fixed in 4% paraformaldehyde, washed with PBS and stained immunocytochemically using highly specific diagnostic antibodies for identification of the following populations: neurons (early neurons - DCX, and late - NeuN), astrocytes (GFAP), stem cells (SOX2), dividing cells (Ki67) and apoptotic cells (caspase 3). The following primary antibodies were used: rabbit detecting GFAP (Dako Z0334, in 1:1000 concentration), mouse detecting GFAP (Sigma G3893, in 1:300 concentration), rabbit detecting DCX (Abcam ab18723, in 1:500 concentration), mouse NeuN (Millipore MAB11144, in 1:300 concentration), goat detecting SOX2 (SantaCruz sc17320, in 1:500 concentration), rabbit detecting Ki67 (Abcam ab16667, in 1:200 concentration), rabbit detecting caspase 3 (CST 9664, in 1:125 concentration); and secondary donkey antibodies (Jackson Immunoresearch): with fluorophore Alexa488 and Alexa594 detecting rabbit antibodies, with fluorophore Alexa488 and Alexa594 detecting mouse antibodies, with fluorophore Alexa488 and Alexa594 detecting goat antibodies. Immunodetection results were imaged by epifluorescence microscope, analyzed digitally and reproduced into respective negative versions (dark sites indicating high content of a detected protein). Simultaneous detection of marker proteins exploiting the secondary antibodies labeled with different fluorophores, excited by light of specific wavelengths, enabled complex analysis of the co-occurrence or determination of the lack of marker within the cells (with respect to a non-specific cellular marker Hoechst). The representative microscope images, after 1 day *in vitro,* are presented in Fig. 39, whereas the analysis of marker localization in the mature culture, on day 14 *in vitro,* are presented in Fig. 40. On the basis of the microscope analysis, it can be seen that the primary neural cell culture, isolated from a developing rat brain cortex, exhibits high diversity of cell types of different proliferation activity not only on the first day of culture but also on day fourteen, which seems to show that this *in vitro* model reliably resembles the diversity of neural populations *in vivo.* The analysis outlined the presence of neuroblasts (immature neurons, immunopositive DCX-staining), neurons (NeuN) and astrocytes (GFAP), as well as dividing cells (Ki67-positive). On day 14, in a culture non-exposed to any injury factors, no cells undergoing cellular death are observed (lack of signal after caspase 3-immunostaining).

**Example 10**

**This describes a method of obtaining a multifactorial model of neurological injury for *in vitro* assessment of the injury-reducing, anti-damaging and/or neuroprotective efficacy of potential pharmacologically-active substances**

A subject of the invention constitutes a diagnostic methodology for measuring the neuroprotective activity of peptides in a model of several factors co-occurring during any neurological injury. The *in vitro* model of the neurological injury was optimized for a primary culture (14-15 days old) of neural cells, isolated directly from brain cortex obtained from Wistar rat embryos, and may successfully be used for other cell types. The model depends on cells' exposition to damaging factors that model biochemical and neurochemical dysfunctions present in neurological diseases and injuries. The model includes simultaneous application of severe injury factors, such as acidification, i.e. acidosis, energetic-respiratory deprivation (glucose deprivation and sodium azide-induced oxidative stress) and excitotoxity induced independently by glutamic acid, NMDA and kainic acid. These injury conditions were optimized so that the strength of each injury factor causes a 30-40% decrease in response in a CellTiterBlue-based evaluation (a CTB fluorophore in a modified biochemical assay of the MTT type) of cellular viability, relying on quantitative analysis of mitochondrial metabolic functions. To this end, cells were incubated with the CTB fluorescent agent for 1-4 hours at 37 °C (5% $CO_2$). The viability test itself was optimized previously in poly-D-lysine-coated 96-well plates. The molecular mechanism of the assay is based on conversion of resazurin (of dark blue color) to resorufin (of pink color) in the mitochondria of living cells. The fluorescence intensity of the CTB agent is measured spectrofluorimetically ($560_{ex}/590_{em}$) and is proportional to the number of living cells. Control cells (without treatment) were considered 100% viable, while signals from other wells provided a control measurement and were presented as mean $\pm$ SD. Statistical analysis relied on the ANOVA test with a *p*-value < 0.05 considered as statistically significant. Each experiment was conducted at least in triplicate.

*1) Optimization of the intensity of glucose deprivation as a neurodamaging factor for the method developed for evaluation of injury-reducing and/or neuroprotective efficacies of potentially pharmacologically-active substances*

For optimization of glucose deprivation conditions, culture media were removed from cell-seeded wells and cells were immediately washed with 300 $\mu$l PBS (pH 7.4) and incubated with 500 $\mu$l of glucose- and sodium pyruvate-free medium (Neurobasal-A) for 10 minutes, 30 minutes and 1, 2 and 3 hours. Each plate contained control cells incubated in a standard culture medium (with 12.5 mM glucose concentration). After an appropriate time period, media in all wells were replaced with fresh, standard medium (500 $\mu$l). The cells were cultivated for 20 hours under standard conditions and then tested with the viability assay. As presented on Fig. 41, this assessment allowed selection the one hour deprivation period (shown as a gray bar) as a condition resulting in around 70% viability and, as such, successively useful for further testing of neuroprotective activity in evaluation of analyzed peptides.

*2) Optimization of the intensity of the sodium azide-induced inhibition of cellular respiration as a neurodamaging factor for evaluation of injury-reducing and/or neuroprotective efficacies of potentially pharmacologically-active substances*

Inhibition of cellular respiration (oxidative stress) was modeled by the incubation of cells with different concentration of sodium azide. To this end, the culture media were removed and cells were immediately washed with 300 $\mu$l of HBSS, and subsequently incubated with culture medium containing 1, 5, 10, 20 or 50 mM sodium azide for 1 hour, under standard conditions. After incubation, medium was exchanged for a standard one (500 $\mu$l) and the cells were incubated under standard conditions for 20 hours before the viability assay was performed. As presented in Fig. 42, the 10 mM sodium azide concentration (grey bar) was selected for further experiments of neuroprotective activity in evaluation of analyzed peptides, as this concentration enabled around 65-70% cellular viability.

*3) Optimization of acidosis pH 6.35 and sodium lactate action as a neurodamaging factor for obtaining injury-reducing and/or neuroprotective efficacies of potentially pharmacologically-active substances*

In order to establish acidosis conditions, it was necessary to optimize both pH value and concentration of sodium lactate. After removal of culture media and a wash with 300 $\mu$l PBS (pH 7.4), the cells were incubated with 500 $\mu$l of a Neurobasal medium of pH 7.4 (control), 6.5, 6.35, 5.55, 5.2 or 4.6 (adjusted with 0.1 M HCl). The sodium lactate concentration was optimized in the same manner, by incubating cells in medium containing 50, 200 or 1000 mM sodium lactate. Finally, the synergistic effect of both factors was measured. After one hour incubation the medium was replaced with a standard one and the cells were incubated for 20 hours. As presented in Fig. 43, on this basis the 200 mM sodium lactate concentration (grey bar) was selected for further experiments of the evaluation of the neuroprotective activity of the analyzed peptides, as this maintained around 70% cellular viability.

*4) Optimization of excitotoxicity induced by glutamic acid, NMDA and kainic acid as a neurodamaging factor for obtaining injury-reducing and/or neuroprotective efficacies of potentially pharmacologically-active substances*

Excitotoxicity was optimized by using independent application of three typical models: glutamic acid, NMDA and kainic acid. All samples of excitotoxic factors were prepared prior to the experiment in culture media in concentrations of 400, 800, 1200, 1600 and 2000 $\mu$M. Before adding the factor, the culture media were removed from the plates and the cells were washed with 300 $\mu$l PBS (pH 7.4), and then incubated for an hour with excitotoxic factors under standard conditions. Cells were incubated with each factor separately. Afterwards, the media were replaced with fresh media and the cells were incubated for 20 hours under standard conditions. As presented in Figs. 44, 45 and 46, on this basis the 400 mM glutamic acid concentration, 800 mM NMDA concentration and 800 mM kainic acid concentration (grey bars) were selected for further experiments to evaluate the neuroprotective activity of the analyzed peptides, as these maintain around 70% cellular viability.

## Example 11

**This describes the method of viability assays for primary neural cultures and results of assessment of the influence of polyRGD peptides on neural culture survival**

Viability of neural cultures was measured quantitatively by the CellTiterBlue test (CTB, see Example 10). Fluorescence intensity of the CTB agent is measured spectrofluorimetrically ($560_{ex}/590_{em}$) and is proportional to the number of living cells. Control samples (untreated) cells were considered 100% viabile, whilst signals from other wells were referred to the control and presented as mean $\pm$ SD. Statistical analysis relied on the ANOVA test where a p-value < 0.05 was considered statistically significant. Each experiment was conducted at least in triplicate. The influence of polyRGD peptides on the viability of primary neural cortical cultures was assayed by 20 hour exposure of the cells to each peptide, dissolved in a culture medium at various concentrations (1, 10, 100 or 250 $\mu$M), and incubation under standard conditions (5% $CO_2$, 95% air, humidity 98%, 37 °C). Peptide RGD10-PS was an exception and was assayed in the concentrations 0.05, 0.1, 0.25, 1, 2.5, 5, 10 and 25 $\mu$M, with the remaining conditions being as above. 50 $\mu$M TAT peptide solution was used as a reference. The assessments were conducted on poly-D-lysine-coated 24-well plates with 500 $\mu$l of working volume. Respective peptide solutions were prepared so that only 50 $\mu$l of medium was replaced in each well, including wells with control cells (peptide-untreated). Each peptide concentration was tested in four wells. Measurements were conducted with respect to the reference peptide Tat(49-57)$NH_2$ (described as TAT) at a concentration of 50 $\mu$M.

In the case of measurements of control (peptide-untreated) cell viability, and for the reason of maintaining optimal conditions, only a part of the medium was replaced with fresh medium. According to the CTB assay measurements acquired for these samples, the mean referred to as 100% was established, and was used as a reference value for the measurements carried out for experiments with damage-inducing factors. It is worth to note that for each damaging factor an independent reference value was acquired, considered as 100% within a separated measurement session and respective conditions, as these in cases of RGD analogues.

On the basis of these quantitative, spectrofluorimetric analyses, no cytotoxic (or, in some cases, only slightly toxic) activities in the tested concentration range were reported for the following peptides: RGD1 (Fig. 47), RGD2 (Fig. 48), RGD3 (Fig. 49), RGD4 (Fig. 50), AcRGD1 (Fig. 51), AcRGD3 (Fig. 52), RGD10-PM (Fig. 53) and RGD10-PS (Fig. 54).

## Example 12

**Neuroprotective influence of RGD peptides *in vitro* in a model of neurological injury**

The neuroprotective influence of RGD peptides was analyzed in an *in vitro* model of neurological injury involving glucose deprivation (Neurobasal-A without glucose, which in other cases was commonly used at a concentration of 12.5 mM), acidosis (Neurobasal medium of pH 6.35 and 200 mM sodium lactate), oxidative stress (sodium at a concentration of 10 mM), and excitotoxcity (400 $\mu$M glutamic acid, 800 $\mu$M NMDA or 800 $\mu$M kainic acid), each analyzed independently. For this analysis primary neural cells (14-15 days old) were used, incubated on 24-well poly-D-lysine-coated plates in a standard medium (Neurobasal, glucose concentration 12.5 mM, 500 $\mu$l/well). The activity of each peptide was analyzed on two 24-well plates, using one for three injury factors and the other for the three remaining ones. The first was used for testing glucose deprivation, oxidative stress and acidosis, while the other was used for the three excitotoxicity inducers. Three wells of each row were predestined to a 1-hour treatment with a respective injury factor, and then with fresh medium, whereas the other three were treated with the same injury factor followed by incubation with the respective peptide.

All injury factors were prepared prior to the experiment. Sterile solutions of RGD1, RGD2, RGD3, RGD4, RGD10-PM, AcRGD1 and AcRGD3 were prepared in a culture medium at a concentration of 100 $\mu$M. The RGD10 sample was prepared at a concentration of 1 $\mu$g/ml. A reference sample of TAT peptide was prepared at a concentration of 50 $\mu$M (selected according to self-made optimization). As mentioned, prior to the peptide treatment, the cells were treated with 1-hour exposure to injury factors under standard conditions (5% $CO_2$, 37 °C). For this purpose, in wells with cells predestined to oxidative stress and excitotoxicity inducers, only 250 $\mu$l of the medium was replaced with media containing the respective injury factors. In cases of glucose deprivation and acidosis, whole volumes were properly exchanged after removal of the culture medium and a 300 $\mu$l PBS wash. Subsequently, as explained above, the cells in one half of each row were incubated with fresh medium, while the other cells were incubated in a medium containing a peptide. After 20 hour incubation under standard conditions, viability assessment was performed. All peptides were examined at least in triplicate, applying newly-isolated cells every time. Statistics was analyzed analogously to previous experiments.

The results of injury-reducing and/or neuroprotective activity of polyRGD peptides are presented in Figs. 55-62. The RGD peptides display selective protective properties with regard to specific injury factors. It must be stressed that, in case of RGD1, such activity is observed in cases of all injurious conditions. The 20 hour incubation of cells in the 100 $\mu$M RGD1 solution leads to a significant increase in neural cell viability with regard to all injury factors. In the case of the other peptides, significant neuroprotection was observed with particular injury factors.

### Example 13

**Direct confirmation of injury-reducing/neuroprotective activity of RGD1 peptide in *ex vivo* brain slices, pretreated with NMDA-induced excitotoxicity**

*An ex vivo model of neurological injury in rat hippocampal slices*

Potential neuroprotective/anti-damaging activity of RGD peptides was verified for peptide RGD1 as an example, the efficacy of which was analyzed directly in rat hippocampal slices treated with the injury factor NMDA. The use of this glutamate receptor agonist induces a cascade of injury processes in the slices of hippocampus, which is the brain structure responsible, along with other brain areas, for learning and spatial memory and the appearance of emotions in humans and other mammals. This material may be used as a sensitive model for analysis of the pharmacological effects of drugs or protective substances. The hippocampal slices were obtained from P7 Wistar rat pups and cultivated as organotypic cultures according to a modified Stoppini method. For this purpose, pups were decapitated, the hippocampi were carefully isolated from the brains and cut with a Tissue Chopper device into 400 $\mu$m thick tissue slices. The slices were cultivated on 6-well plates with culture inserts (4 slices/insert, MILLICELL CM Millipore with pores of 0.4 $\mu$m and a diameter of 30 mm), in medium (containing 50% Neurobasal, 22% HBSS supplemented with 25% horse serum and 2% B27, 1M HEPES; 5 mg/ml glucose; 1% penicillin/streptomycin). The culture was initiated in the medium with 25% horse serum, which was step-wise (day-by-day) replaced with a serum-free medium. The cultures were conducted for 8 days under conditions of 36 °C and 5% $CO_2$ atmosphere according to the scheme presented in Fig. 63. On the eighth day of culture the neuroprotective activity of the RGD peptide was analyzed (Fig. 63, 64 and 65). To this end, the slices were divided into four experimental groups:

Group 1) control - slices cultivated in serum-free, NMDA-free and tested peptide-free medium;
Group 2) control + tested peptide - slices cultivated in serum-free medium and with tested peptide (analysis of peptide cytotoxicity);
Group 3) NMDA - slices cultivated in serum-free medium and with an injury factor (100$\mu$M NMDA), which was removed after 3 hours;
Group 4) NMDA + tested peptide - slices cultivated in serum-free medium and with an injury factor (100 $\mu$M NMDA) and the tested peptide, after 3 hours of incubation with NMDA, and up to the end of the experiment with the peptide alone.

*Evaluation of neurological injury and neuroprotective activity of RGD1 peptide in the rat brain slices*

The slices within all four groups were stained with ~1 $\mu$M propidium iodide (to label dead cells) and incubated for 30 minutes for the purpose of elimination of the dead slices. The hippocampal cultures in Group 4 were subsequently exposed to a mixture of 100 $\mu$M NMDA and RGD1 in three concentrations, namely 1, 10 and 100 $\mu$M (on the experiment day, the peptides were suspended in autoclaved water, and subsequently filtrated and stored in ice 4°C), after which the slices were incubated under standard conditions for 3 hours. After this time, the medium in all groups was changed for the purpose of NMDA removal, by complete replacement of serum with the serum-free medium containing RGD in the respective concentrations. The slices were then incubated for 24 hours and then stained for 30 minutes with 6 $\mu$M

propidium iodide (for dead cells visualization) before final detection and evaluation of the neuroprotective efficacy of the peptide RGD1. This was followed by a quantitative analysis using a fluorescence microscope and digital image reproduction equipment. Cell mortality in the slices was counted as a ratio of the fluorescence intensity of the slice and maximal fluorescence, acquired in this case after pre-treatment of the control slices with 250 $\mu$M NMDA.

Exemplary images of the hippocampal slices, in Fig. 64. show a density difference of fluorescent-labeled points, referred to as low mortality of neurons under control conditions (a) and high mortality after the application of 100 $\mu$M NMDA (b). The density measurements of the dead cells prove the injury-reducing and/or neuroprotective properties of peptide RGD1 in an *ex vivo* model of excitotoxicity occurring, among other things, in stroke brain injuries. As shown in Fig. 64, the peptide RGD1 exhibits no significant toxicity in this model and, moreover, acts protectively at concentrations of 10 and 100 $\mu$M.

## Example 14

**A kit for in vitro evaluation of injury-reducing and/or neuroprotective activity of chemical compounds in neural cell cultures, which contains 6 injury factors involved in processes of rapid nervous system injury of vertebrates, including humans, and a reference compound for obtaining a method of comparison of biological and physiological responses of examined cells.**

The setup serves for in vitro evaluation of injury-reducing and/or neuroprotective activity of chemical compounds in cells obtained from both neural and non-neural cultures. In a series of experiments, the proposed diagnostic setup was successfully exploited in research on the injury-reducing/neuroprotective activity in cells other than neurons, e.g., a supplemented culture of astrocytes. The described setup enables the evaluation of injury-reducing and/or neuroprotective properties in a wide cluster of cells, potentially participating in clinically-relevant situations and in pre-clinical research into the repair of central nervous system injuries. The regenerative potential in CNS repair was demonstrated for cells of diverse origin, such as stem cells, tissue-specific stem cells (both progenitors and precursors), progenitors, precursors, adolescent (immature forms) and mature active and inactive forms of neurons, astrocytes, oligodendrocytes, microglia, olfactory endothelium glia, adipocytes and cells of hematopoietic and immune systems, and others, and may cover cells naturally-induced to pluripotent properties (including so-called very small embryonic-like stem cells (VSEL), and others), and cells induced in a human-stimulated manner (including so-called pluripotent stem cells), and not restricted to the species of origin, and not excluding mammalian and human cells. Such positive examples refer not only to primary cell cultures, but also to cell lines, for instance broadly-described human and/or animal cells (of self- or non-self-origin) used in central nervous system repair.

The setup ought to involve a reference compound (constituting also a control or a calibrating compound), that displays or provides positive responses or signals with regard to the injury factors. In particular, in the case of control cells (untreated with the injury factors), the reference ought to allow for acquisition of at least 4 significantly positive signals out of all 6 *in vitro* injury factors applied to excitatory cells (e.g. neurons). Some peptides may serve as such compounds, for instance RGD1 at a concentration of 100 $\mu$M. This compound is a claim subject of this patent (see Fig. 55) and has been reported to exhibit significant injury-reducing and/or neuroprotective efficacy with respect to all six injury factors *in vitro.* In the case of the reference compound applied to non-excitatory cells, significantly relevant changes may be assumed for at least 3 out of the six factors, especially as a common CTB assessment (a modified MTT biochemical test) has been developed for determination of the viability of non-excitatory cells. The setup for in vitro evaluation of injury-reducing and/or neuroprotective activity of chemical compounds in neural cell cultures may also be used with non-neural cell cultures, such as homogenous or mixed cultures of vertebrate-derived cells of different types and origins, including human cells.

A suspension of cells used in the setup may include: 1a) freshly isolated cells from biological structures of animals (mammals, especially) or isolated from human tissue (within a strictly-determined time regime) obtained from a biopsy or post-mortem, to enable establishment of a primary mixed cell culture, or 1b) a modifying culture, i.e after the application of selective/differentiative media to the culture in later stages, so that the culture contains only selected cell fractions (e.g. upon the use of cytostatic substance of an AraC type that, according to a respective scheme, may be deprived of dividing cells), or after the addition of supplementary substances so to differentiate the primary cells into types that were absent in a previous stage of development. It is also possible to exploit 1c) freshly and selectively isolated cells from biological structures on the basis of their physical properties (e.g. using differentiative centrifugation regarding the size) or of their biological properties (according to selective superficial or intracellular epitopes, allowing affinity chromatography or sorting by using fluorophore- or magnetic particle-conjugated immunoglobulins). One may also use 1d) cell suspensions modified on solid substrates by means of molecular biology, e.g. induced pluripotent cells (iPS) obtained in person or from commercial sources. One may also use 2a) primary cell mixtures or 2b) mixtures supplemented with specific cell subpopulations, or 2c) homogenous cultures of one type of cell. Another cell type that may be used in the setup are 3) cell lines with common immortalized characteristics of cellular division. In addition, one may use 4a) excitatory cells, characterized by electrical conductivity, e.g. mature neurons, neuroblasts (adolescent neurons with different conductivity than that of mature ones) and others that respond physiologically to excitatory factors, including excitotoxic factors, and 4b) non-excitatory cells, which do not respond to excitatory factors but do to general-type factors, such as those with relevance to energetic or metabolic processes (glucose deprivation, partial oxygen deprivation/oxidative stress, acidosis and lactate metabolism).

One of the examples, which shows the non-limiting application of the in vitro setup for the evaluation of injury-reducing and/or neuroprotective efficacy of chemical compounds, is isolation of mixture of cell types from selected structures of a developing brain or spinal cord, derived from rats, mice or other mammals, isolated in prenatal or neonatal periods or from adults. Such a procedure was employed in presented patent, in which the following cells were exploited: non-modified cells freshly isolated from selected rat brain structures (1a) or with differentiating modifications (1b), cell mixtures (2a) or a population enriched with specific subpopulations (e.g. neurons after the elimination of dividing cells) (2b), excitatory cells such as neurons and neuroblasts (4a) in a mixture of non-excitatory cells, such as astrocytes or other types of glial cells (4b)

Cells included in the setup were exploited in a form of a live primary cell suspension or in a deep frozen state and, after reconstitution (if necessary), were incubated at an appropriate density on multi-well plates, coated with poly-D-lysine or laminin-coated, as described in previous paragraphs, or without such surface coating in "Medium N" (composition below) or in other media. An optional possibility allows for isolation of neural cells from the biological structures, which exploits "Medium D", which is based on sterile aqueous solution of 10% Hank's Balanced Salt Solution, supplemented with 1% penicillin/streptomycin and 1% HEPES. Tissue fragments are exposed to "Medium E", prepared on the basis of "Medium D" with 0.08% trypsin, and incubated for a few minutes or a dozen or so minutes at 37 °C, depending on the tissue type. After cell disintegration with centrifugation and several washes with "Medium D", the cells are incubated for 7 to 14 days, or for another time period, at an appropriate density, determined by microscopic measurements. The media for the neural cells ("Medium N") are based on Neurobasal, and contain 2% B27, 200 mM L-glutamine, 10 mM glutamic acid, 1% penicillin and streptomycin, and glucose in a concentration which depends on the type of tissue from which the cells were isolated (usually 12.5 mM, with or without 1 mM sodium pyruvate). The cells are seeded on 24-well poly-D-lysine-coated plates, with 200,000 cells/well, or on 96-well poly-D-lysine-coated plates, with 15,000 cells/well.). The neural (cultures of neuronal or glial cells or a mixture of both types) or non-neural cultures are incubated for 14-15 days, usually in an atmosphere of 5% $CO_2$ (95% air, 98% humidity, 37 °C).

The setup includes viability assessments of the cell culture in response to different concentrations of a tested peptide and viability assesments applied after cell exposure to 6 injury and/or neurotoxic factors.

The model includes the parallel exposure of cells, obtained by common isolation, to strong injury factors, such as: acidification, i.e. acidosis (pH 6.35) and presence of 200 mM lactate or another optimized agent for a given cell type), energetic-respiratory deprivation (glucose deprivation and oxidative stress, induced by 10 mM sodium azide or by another optimized agent for a given cell type) and excitotoxicity (400 $\mu$M glutamic acid, 800 $\mu$M NMDA or 800 $\mu$M kainic acid, or other optimized agents for a given cell type). The injury conditions are conducted in such a manner that the strength of each injury factor causes a 30-40% decrease in response to a CellTiterBlue assay (a CTB fluorophore in a modified biochemical assay of the MTT type), or of another cellular viability assay which based on the evaluation of cellular viability by relying on measurable analysis of metabolic functions of mitochondria. In the case of CTB assessment, the cells are incubated with the CTB fluorescent agent for 1-4 hours at 37 °C (5% $CO_2$). The fluorescence intensity of the CTB agent is measured spectrofluorimtrically ($560_{ex}/590_{em}$) and is proportional to the number of living cells. Samples of control (untreated) cells were considered to be 100% viability, whilst signals from other wells were compared to the control and presented as a mean $\pm$ SD.

Control cell cultures are cultures without a tested substance, while reference cell cultures are cultures with a reference compound/agent, e.g. peptide RGD1 peptide at a concentration of 100 $\mu$M or another agent for a given type of cells.

A subject of presented invention is a methodology for efficient and multifactorial evaluation and analysis of the injury-reducing and/or neuroprotective activities of potentially pharmacologically-active agents, as well as chemical and biotechnological synthesis of polyRGD peptides and RGD peptide analogues, and demonstration of their biological (injury-reducing and/or neuroprotective) properties in response to injury factors.

The obtained peptides exhibited stability under physiological conditions. Introduction of modifications in the forms of C-amidification and N-acetylation results in enhanced resistance of the compounds to proteolysis. Despite the fact that sequence multiplication of a RGD motif commonly decreases its activity, it also enables prolonged release of the agent in an organism. Both chemical synthesis on a solid substrate and biotechnological production provided purity, specificity and effective yields of the obtained compounds.

As described above, the RGD motif is an essential element of extracellular matrix proteins, recognized by integrins and responsible, among things, for synaptic plasticity and proper CNS functionality, and, as such, may be exploited as a component of neuroprotectants. Single RGD peptides and polyRGD peptides, and their analogues, might turn out to be effective tools in treatment of neurological diseases, in the light of the expensive production cost of pharmaceuticals based on ECM protein structures. Damage to the nervous system, induced by a mechanical breakage of both blood vessel integrity/continuity and the blood-brain barrier, leads to a series of metabolic and neurochemical disorders, which finally culminate in excessive, self-stimulating neuronal excitation and inflammation. A subject of the invention covers a setup of six such injury factors as an efficient method of effective screening in the search for neuroprotective agents and for biological analysis of the activity of potential pharmacologically-active agents, at a cellular level, in treatment of central nervous system injuries. For a demonstration of the neuroprotective properties of the RGD peptides, primary neural cell cultures, obtained from the Wistar rat brain cortex, were initially exposed to the injury factors and, afterwards, to the peptide. None of the analyzed peptides displayed significant cytotoxic effects in the assessed concentration ranges, as well as a reference factor, i.e. TAT peptide in this case. Moreover, particular polyRGD peptides exhibited injury-reducing and/or neuroprotective properties with respect to all or to selected injury factors.

Peptide RGD 1 showed the broadest spectrum of activity, which may be associated with the greater availability of the active sequence, which in the cases of the multiplicated analogues is only released upon efficient enzymatic hydrolysis. On the other hand, the use of the peptides with repeated sequences is suggested to ensure stability and prolonged controlled release.

As evidenced throughout our experiments, a majority of polyRGD peptides act as injury-reducing and/or neuroprotective agents against excitotoxicity-induced symptoms. As mentioned, the excitotoxcity occurs as a consequence of energetic-respiratory deprivation and dysregulation of integrin receptor expression in cell membranes. These observations were confirmed in an animal model of ischemic stroke. The neuroprotective properties of the polyRGD analogues were also observed in cases of acidosis or oxidative stress. Nerve cells, and neurons especially, are prone to excessive environmental acidification, caused by the presence of lactic acid, a metabolite of anaerobic respiration, which occurs as a result of stroke. Pathological outcomes resulting from the dysregulation of cellular respiration and oxidative stress, which arise as a consequence of energetic disorders in mitochondria, may be attenuated by the activity of polyRGD peptides, since all the aforementioned are controlled by membrane-localized integrins which, in turn are modulated by proteins containing the RGD motif, as demonstrated in a rat model of Parkinson's disease. In order to verify the activity of RGD1 in an ex vivo model of excitotoxicity, the peptide was added to rat hippocampal slices pre-treated with NMDA. The experiment proved the injury-reducing and/or neuroprotective properties of RGD1.

Literature premises suggest that large proteins containing native RGD motifs (different to the presented patent in which a RGD peptide alone, and its repeats and modifications, are analyzed) act through integrins (activated by the RGD sequence itself), which are present in dendritic spines, during cytoskeletal reorganization in hippocampal neurons, in a manner dependent on the activation of NMDA receptors and calcium ion- and calmodulin-dependent type II kinase (CaMKII).

References for chemical compounds for Example 14

1. Brewer GJ, Cotman CW., Survival and growth of hippocampal neurons in defined medium at low density: advantages of a sandwich culture technique or low oxygen. Brain Res. 1989,494(1):65-74.
2. Brewer GJ, Torricelli JR, Evege EK, Price PJ., Optimized survival of hippocampal neurons in B27-supplemented Neurobasal, a new serum-free medium combination. J Neurosci Res. 1993;35(5):567-76.

All compounds used for the kit/setup are of chemical purity of at least 'pure for analysis' level.

## Conclusions

A subject of the presented invention is a methodology for efficient and multifactorial evaluation and analysis of the injury-reducing and/or neuroprotective activities of potentially pharmacologically-active agents, as well as the chemical and biotechnological synthesis of polyRGD peptides and RGD peptide analogues, and demonstration of their biological (injury-reducing and/or neuroprotective) properties in response to injury factors.

The obtained peptides exhibit stability under physiological conditions. Introduction of modifications in the forms of C-amidification and N-acetylation results in enhanced resistance of the compounds to proteolysis. Despite the fact that sequence multiplication of a RGD motif commonly decreases its activitiy, it also enables prolonged release of the agent in an organism. Both chemical synthesis on a solid substrate and biotechnological production provid purity, specificity and yield/efficacy of the obtained compounds.

As described above, the RGD motif is an essential element of extracellular matrix proteins, recognized by integrins and responsible, among other things, for synaptic plasticity and proper CNS functionality. As such, the compounds may be exploited as neuroprotectants. The single-RGD peptide and polyRGD compounds and their analogues might turn out to be effective tools in neurological diseases, in the light of expensive costs of production of pharmaceuticals, based on structures of the ECM proteins. Damage of the nervous system, induced by a mechanical breakage of both blood vessel integrity/continuity and the blood-brain barrier, leads to a series of metabolic and neurochemical disorders, which finally culminate in excessive, self-stimulating neuronal excitation and inflammation. A subject of the invention covers a setup of six such injury factors as an efficient method for effective screening of neuroprotective agents and for the biological analysis of the activity of potential, pharmacologically-active agents on the cellular level of central nervous system injuries. For a demonstration of the neuroprotective properties of the RGD peptides, primary neural cell cultures, obtained from the Wistar rat brain cortex, were initially exposed to the injury factors and, afterwards, to the activity of the peptide. None of the analyzed peptides displayed significantly-relevant cytotoxic effects in the assessed concentration ranges. Moreover, particular polyRGD peptides exhibited injury-reducing and/or neuroprotective properties with respect to all or to particular injury factors.

Peptide RGD1 showed the broadest spectrum of activity, which may be associated with the greater availability of the active sequence, while in the cases of the multiplicated analogues they are released upon efficient enzymatic hydrolysis. On the other hand, the use of the peptides with repeated sequences is suggested to ensure stability and prolonged controlled release.

As evidenced throughout our experiments, a majority of the polyRGD peptides act as injury-reducing and/or neuroprotective agents against excitotoxicity-induced symptoms. As mentioned, the excitotoxcity occurs as a consequence of energic-respiratory deprivation and dysregulation of integrin receptor expression in cell membranes. These observations were confirmed in an animal model of ischemic stroke. The neuroprotective properties of the polyRGD analogues were also observed in cases of acidosis or oxidative stress. Nerve cells, and especially neurons, are prone to excessive environmental acidification caused by the presence of lactic acid, a metabolite of anaerobic respiration, which occurs as a result of stroke. Pathological outcomes resulting from the dysregulation of cellular respiration and oxidative stress, which arise as a consequence of energetic disorders in mitochondria, may be attenuated by the activity of the polyRGD peptides, since all the aforementioned are are controlled, among other things, by membrane-localized integrins, which, in turn are modulated by proteins containing the RGD motif, as demonstrated in a rat model of Parkinson disease. In order to verify the activity of RGD1 in an ex vivo model of excitotoxicity, the peptide was added to rat hippocampal slices, pre-treated with NMDA. The experiment proved the injury-reducing and/or neuroprotective properties of RGD 1.

Contrary to the presented patent, in which a RGD peptide alone and its repeats and modifications are analyzed, literature premises suggest that large proteins containing native RGD motifs - act through integrins (activated by the RGD sequence itself), which are present in dendritic spines, during cytoskeletal reorganization in hippocampal neurons, in a manner depending on the activation of NMDA receptors and calcium ion- and calmodulin-dependent type II kinase (CaMKII).

### *References*

1. Miller G. Neuroscience. New guidelines aim to improve studies of traumatic brain injury. Science. 2010;328(5976):297.

2. Chen CC, Hung TH, Lee CY, Wang LF, Wu CH, Ke CH, et al. Berberine protects against neuronal damage via suppression of glia-mediated inflammation in traumatic brain injury. PloS one. 2014;9(12):e 115694.

3. Miller DM, Singh IN, Wang JA, Hall ED. Nrf2-ARE activator carnosic acid decreases mitochondrial dysfunction, oxidative damage and neuronal cytoskeletal degradation following traumatic brain injury in mice. Experimental neurology. 2015;264:103-10.

4. Russo MV, McGavern DB. Inflammatory neuroprotection following traumatic brain injury. Science. 2016;353(6301):783-5.

5. Jackson A. Spinal-cord injury: Neural interfaces take another step forward. Nature. 2016;539(7628): 177-8.

6. Owens B. Stroke. Nature. 2014;510(7506):S1.

7. Venkat P, Shen Y, Chopp M, Chen J. Cell-based and pharmacological neurorestorative therapies for ischemic stroke. Neuropharmacology. 2017.

8. Wang Q, Yu S, Simonyi A, Sun GY, Sun AY. Kainic acid-mediated excitotoxicity as a model for neurodegeneration. Molecular neurobiology. 2005;31(1-3):3-16.

9. Meloni BP, Craig AJ, Milech N, Hopkins RM, Watt PM, Knuckey NW. The neuroprotective efficacy of cell-penetrating peptides TAT, penetratin, Arg-9, and Pep-1 in glutamic acid, kainic acid, and in vitro ischemia injury models using primary cortical neuronal cultures. Cell Mol Neurobiol. 2014;34(2):173-81.

10. Meloni BP, Brookes LM, Clark VW, Cross JL, Edwards AB, Anderton RS, et al. Poly-arginine and arginine-rich peptides are neuroprotective in stroke models. Journal of cerebral blood flow and metabolism : official journal of the International Society of Cerebral Blood Flow and Metabolism. 2015;35(6):993-1004.

11. Patel S, Tsang J, Harbers GM, Healy KE, Li S. Regulation of endothelial cell function by GRGDSP peptide grafted on interpenetrating polymers. Journal of biomedical materials research Part A. 2007;83(2):423-33.

12. Mihardja SS, Gao D, Sievers RE, Fang Q, Feng J, Wang J, et al. Targeted in vivo extracellular matrix formation promotes neovascularization in a rodent model of myocardial infarction. PloS one. 2010;5(4):e10384.

13. Rubtsov MA, Syrkina MS, Aliev G. RGD-based Therapy: Principles of Selectivity. Current pharmaceutical design. 2016;22(7):932-52.

14. Aota S, Nomizu M, Yamada KM. The short amino acid sequence Pro-His-Ser-Arg-Asn in human fibronectin enhances cell-adhesive function. The Journal of biological chemistry. 1994;269(40):24756-61.

15. Wang F, Li Y, Shen Y, Wang A, Wang S, Xie T. The functions and applications of RGD in tumor therapy and tissue engineering. International journal of molecular sciences. 2013;14(7): 13447-62.

16. Isenberg WM, McEver RP, Phillips DR, Shuman MA, Bainton DF. The platelet fibrinogen receptor: an immunogold-surface replica study of agonist-induced ligand binding and receptor clustering. The Journal of cell biology. 1987;104(6):1655-63.

17. Katoh D, Nagaharu K, Shimojo N, Hanamura N, Yamashita M, Kozuka Y, et al. Binding of alphavbeta1 and alphavbeta6 integrins to tenascin-C induces epithelial-mesenchymal transition-like change of breast cancer cells. Oncogenesis. 2013;2:e65.

18. Denhardt DT, Giachelli CM, Rittling SR. Role of osteopontin in cellular signaling and toxicant injury. Annual review of pharmacology and toxicology. 2001;41:723-49.

19. Stipp CS. Laminin-binding integrins and their tetraspanin partners as potential antimetastatic targets. Expert reviews in molecular medicine. 2010;12:e3.

20. Massia SP, Hubbell JA. An RGD spacing of 440 nm is sufficient for integrin alpha V beta 3-mediated fibroblast spreading and 140 nm for focal contact and stress fiber formation. The Journal of cell biology. 1991;114(5):1089-100.

21. Clark EA, Brugge JS. Integrins and signal transduction pathways: the road taken. Science. 1995;268(5208):233-9.

22. Albelda SM, Mette SA, Elder DE, Stewart R, Damjanovich L, Herlyn M, et al. Integrin distribution in malignant melanoma: association of the beta 3 subunit with tumor progression. Cancer research. 1990;50(20):6757-64.

23. Chan BM, Matsuura N, Takada Y, Zetter BR, Hemler ME. In vitro and in vivo consequences of VLA-2 expression on rhabdomyosarcoma cells. Science. 1991;251(5001):1600-2.

24. Ferris DM, Moodie GD, Dimond PM, Gioranni CW, Ehrlich MG, Valentini RF. RGD-coated titanium implants stimulate increased bone formation in vivo. Biomaterials. 1999;20(23-24):2323-31.

25. Alkhodary MA. Laser micro-grooved, Arginine-Glycine-Apspartic acid (RGD) coated dental implants, a 5 years radiographic follow-up. International journal of health sciences. 2014;8(4):361-9.

26. Uzunalli G, Soran Z, Erkal TS, Dagdas YS, Dine E, Hondur AM, et al. Bioactive self-assembled peptide nanofibers for corneal stroma regeneration. Acta biomaterialia. 2014;10(3):1156-66.

27. Tweden KS, Harasaki H, Jones M, Blevitt JM, Craig WS, Pierschbacher M, et al. Accelerated healing of cardiovascular textiles promoted by an RGD peptide. The Journal of heart valve disease. 1995;4 Suppl 1:S90-7.

28. Wu XaR, D. Integrins as receptor targets for neurological disorders. Pharmacology & Therapeutics. 2012;134(1):68-81.

29. Becchetti A, Arcangeli A. Integrins and ion channels in cell migration: implications for neuronal development, wound healing and metastatic spread. Adv Exp Med Biol. 2010;674:107-23.

30. Burkin DJ, Kim JE, Gu M, Kaufman SJ. Laminin and alpha7beta1 integrin regulate agrin-induced clustering of acetylcholine receptors. Journal of cell science. 2000;113 ( Pt 16):2877-86.

31. Bernard-Trifilo JA, Kramar EA, Torp R, Lin CY, Pineda EA, Lynch G, et al. Integrin signaling cascades are operational in adult hippocampal synapses and modulate NMDA receptor physiology. Journal of neurochemistry. 2005;93(4):834-49.

32. van der Laan LJ, van der Goes A, Wauben MH, Ruuls SR, Dopp EA, De Groot CJ, et al. Beneficial effect of modified peptide inhibitor of alpha4 integrins on experimental allergic encephalomyelitis in Lewis rats. Journal of neuroscience research. 2002;67(2):191-9.

33. Gladson CL, Stewart JE, Olman MA, Chang PL, Schnapp LM, Grammer JR, et al. Attachment of primary neonatal rat astrocytes to vitronectin is mediated by integrins alphavbeta5 and alpha8beta1: modulation by the type 1 plasminogen activator inhibitor. Neuroscience letters. 2000;283(2):157-61.

34. Silletti S, Kessler T, Goldberg J, Boger DL, Cheresh DA. Disruption of matrix metalloproteinase 2 binding to integrin alpha vbeta 3 by an organic molecule inhibits angiogenesis and tumor growth in vivo. Proceedings of the National Academy of Sciences of the United States of America. 2001;98(1):119-24.

35. Akiyama H, Kawamata T, Dedhar S, McGeer PL. Immunohistochemical localization of vitronectin, its receptor and beta-3 integrin in Alzheimer brain tissue. Journal of neuroimmunology. 1991;32(1): 19-28.

36. Clegg DO, Wingerd KL, Hikita ST, Tolhurst EC. Integrins in the development, function and dysfunction of the nervous system. Frontiers in bioscience : a journal and virtual library. 2003;8:d723-50.

37. Dityatev A, Fellin T. Extracellular matrix in plasticity and epileptogenesis. Neuron glia biology. 2008;4(3):235-47.

38. Wagner S, Tagaya M, Koziol JA, Quaranta V, del Zoppo GJ. Rapid disruption of an astrocyte interaction with the extracellular matrix mediated by integrin alpha 6 beta 4 during focal cerebral ischemia/reperfusion. Stroke. 1997;28(4):858-65.

39. Relton JK, Sloan KE, Frew EM, Whalley ET, Adams SP, Lobb RR. Inhibition of alpha4 integrin protects against transient focal cerebral ischemia in normotensive and hypertensive rats. Stroke. 2001;32(1): 199-205.

40. Mabon PJ, Weaver LC, Dekaban GA. Inhibition of monocyte/macrophage migration to a spinal cord injury site by an antibody to the integrin alphaD: a potential new antiinflammatory treatment. Experimental neurology. 2000;166(1):52-64.

41. Weaver LC, Bao F, Dekaban GA, Hryciw T, Shultz SR, Cain DP, et al. CD11d integrin blockade reduces the systemic inflammatory response syndrome after traumatic brain injury in rats. Experimental neurology. 2015;271:409-22.

42. Vogelezang MG, Liu Z, Relvas JB, Raivich G, Scherer SS, ffrench-Constant C. Alpha4 integrin is expressed during peripheral nerve regeneration and enhances neurite outgrowth. The Journal of neuroscience : the official journal of the Society for Neuroscience. 2001;21(17):6732-44.

43. Werner A, Willem M, Jones LL, Kreutzberg GW, Mayer U, Raivich G. Impaired axonal regeneration in alpha7 integrin-deficient mice. The Journal of neuroscience : the official journal of the Society for Neuroscience. 2000;20(5): 1822-30.

44. Xiao T, Takagi J, Coller BS, Wang JH, Springer TA. Structural basis for allostery in integrins and binding to fibrinogen-mimetic therapeutics. Nature. 2004;432(7013):59-67.

45. Wang G. Post-translational Modifications of Natural Antimicrobial Peptides and Strategies for Peptide Engineering. Curr Biotechnol. 2012;1(1):72-9.

46. Soundara Manickam D, Bisht HS, Wan L, Mao G, Oupicky D. Influence of TAT-peptide polymerization on properties and transfection activity of TAT/DNA polyplexes. J Control Release. 2005;102(1):293-306.

47. Morini R, Becchetti A. Integrin receptors and ligand-gated channels. Advances in experimental medicine and biology. 2010;674:95-105.

48. Maier M, Seabrook TJ, Lazo ND, Jiang L, Das P, Janus C, et al. Short amyloid-beta (Abeta) immunogens reduce cerebral Abeta load and learning deficits in an Alzheimer's disease mouse model in the absence of an Abeta-specific cellular immune response. The Journal of neuroscience : the official journal of the Society for Neuroscience. 2006;26(18):4717-28.

49. Wu DY, Wang LC, Mason CA, Goldberg DJ. Association of beta 1 integrin with phosphotyrosine in growth cone filopodia. The Journal of neuroscience : the official journal of the Society for Neuroscience. 1996;16(4):1470-8.

50. Yip PM, Zhao X, Montgomery AM, Siu CH. The Arg-Gly-Asp motif in the cell adhesion molecule L1 promotes neurite outgrowth via interaction with the alphavbeta3 integrin. Molecular biology of the cell. 1998;9(2):277-90.

51. Rafiuddin Ahmed M, Jayakumar R. Peripheral nerve regeneration in RGD peptide incorporated collagen tubes. Brain research. 2003;993(1-2):208-16.

52. Woerly S, Pinet E, de Robertis L, Van Diep D, Bousmina M. Spinal cord repair with PHPMA hydrogel containing RGD peptides (NeuroGel). Biomaterials. 2001;22(10): 1095-111.

53. Yang H, Yang H, Lu Y, Ma S, Liu Y, Jia G, et al. Targeted delivery of extracellular matrix protected against neurologic defects after focal ischemia reperfusion in rats. Journal of stroke and cerebrovascular diseases : the official journal of National Stroke Association. 2015,24(1):154-62.

54. Ananthanarayanan B, Little L, Schaffer DV, Healy KE, Tirrell M. Neural stem cell adhesion and proliferation on phospholipid bilayers functionalized with RGD peptides. Biomaterials. 2010;31(33):8706-15.

55. Yang K, Lee JS, Kim J, Lee YB, Shin H, Um SH, et al. Poly dopamine-mediated surface modification of scaffold materials for human neural stem cell engineering. Biomaterials. 2012;33(29):6952-64.

56. Cipriani R, Chara JC, Rodriguez-Antiguedad A, Matute C. FTY720 attenuates excitotoxicity and neuroinflammation. Journal of neuroinflammation. 2015;12:86.

57. Meloni BP, Majda BT, Knuckey NW. Establishment of neuronal in vitro models of ischemia in 96-well microtiter strip-plates that result in acute, progressive and delayed neuronal death. Neuroscience. 2001;108(1):17-26.

58. Lin CY, Hilgenberg LG, Smith MA, Lynch G, Gall CM. Integrin regulation of cytoplasmic calcium in excitatory neurons depends upon glutamate receptors and release from intracellular stores. Molecular and cellular neurosciences. 2008;37(4):770-80.

59. Watson PM, Humphries MJ, Relton J, Rothwell NJ, Verkhratsky A, Gibson RM. Integrin-binding RGD peptides induce rapid intracellular calcium increases and MAPK signaling in cortical neurons. Molecular and cellular neurosciences. 2007;34(2):147-54.

60. Milner R, Hung S, Wang X, Berg GI, Spatz M, del Zoppo GJ. Responses of endothelial cell and astrocyte matrix-integrin receptors to ischemia mimic those observed in the neurovascular unit. Stroke. 2008;39(1):191-7.

61. Chen FY, Lee TJ. Arginine synthesis from citrulline in perivascular nerves of cerebral artery. J Pharmacol Exp Ther. 1995;273(2):895-901.

62. de Hemptinne A, Marrannes R, Vanheel B. Influence of organic acids on intracellular pH. The American journal of physiology. 1983;245(3):C178-83.

63. Goldman SA, Pulsinelli WA, Clarke WY, Kraig RP, Plum F. The effects of extracellular acidosis on neurons and glia in vitro. Journal of cerebral blood flow and metabolism : official journal of the International Society of Cerebral Blood Flow and Metabolism. 1989;9(4):471-7.

64. Baumann F, Leukel P, Doerfelt A, Beier CP, Dettmer K, Oefner PJ, et al. Lactate promotes glioma migration by TGF-beta2-dependent regulation of matrix metalloproteinase-2. Neuro-oncology. 2009;11(4):368-80.

65. Li X, Yu X, Dai D, Song X, Xu W. The altered glucose metabolism in tumor and a tumor acidic microenvironment associated with extracellular matrix metalloproteinase inducer and monocarboxylate transporters. Oncotarget. 2016;7(17):23141-55.

66. Saito A, Hayashi T, Okuno S, Nishi T, Chan PH. Oxidative stress affects the integrin-linked kinase signaling pathway after transient focal cerebral ischemia. Stroke. 2004;35(11):2560-5.

67. Gary DS, Mattson MP. Integrin signaling via the PI3-kinase-Akt pathway increases neuronal resistance to glutamate-induced apoptosis. Journal of neurochemistry. 2001;76(5): 1485-96.

68. Saavedra A, Baltazar G, Carvalho CM, Duarte EP. GDNF modulates HO-1 expression in substantia nigra postnatal cell cultures. Free radical biology & medicine. 2005;39(12): 1611-9.

69. Shi Y, Ethell IM. Integrins control dendritic spine plasticity in hippocampal neurons through NMDA receptor and Ca2+/calmodulin-dependent protein kinase II-mediated actin reorganization. The Journal of neuroscience : the official journal of the Society for Neuroscience. 2006;26(6):1813-22.

70. Wang Q, Yu S, Simonyi A, Sun GY, Sun AY. Kainic acid-mediated excitotoxicity as a model for neurodegeneration. Molecular neurobiology. 2005;31(1-3):3-16.

71. Meloni BP, Craig AJ, Milech N, Hopkins RM, Watt PM, Knuckey NW. The neuroprotective efficacy of cell-penetrating peptides TAT, penetratin, Arg-9, and Pep-1 in glutamic acid, kainic acid, and in vitro ischemia injury models using primary cortical neuronal cultures. Cellular and molecular neurobiology. 2014;34(2): 173-81.

72. Meloni BP, Brookes LM, Clark VW, Cross JL, Edwards AB, Anderton RS, et al. Poly-arginine and arginine-rich peptides are neuroprotective in stroke models. Journal of cerebral blood flow and metabolism : official journal of the International Society of Cerebral Blood Flow and Metabolism. 2015;35(6):993-1004.

73. Cipriani R, Chara JC, Rodriguez-Antiguedad A, Matute C. FTY720 attenuates excitotoxicity and neuroinflammation. Journal of neuroinflammation. 2015;12:86.

## Claims

1. Chemical compounds for use as an agent for regeneration of a neurological injury and/or treatment of neurological injury such as:

| | |
|---|---|
| $H_2N$-RGD-amide | (referred to as **RGD1**), |
| $H_2N$-RGDRGD-amide | (referred to as **RGD2**), |
| $H_2N$-RGDRGDRGD-amide | (referred to as **RGD3**), |
| acetyl-RGDRGDRGD-amide | (referred to as **Ac-RGD3**) |
| $H_2N$-RGDRGDRGDRGD-amide | (referred to as **RGD4**) |

## Patentansprüche

1. Chemische Verbindungen zur Verwendung agent als regeneration und/oder Behandlung neurologischer Verletzungen wie:

H2N-RGD-Amid (bezeichnet als RGD1),
H2N-RGDRGD-Amid (bezeichnet als RGD2),
H2N-RGDRGDRGD-Amid (bezeichnet als RGD3),
Acetyl-RGDRGDRGD-Amid (bezeichnet als Ac-RGD3)
H2N-RGDRGDRGDRGD-Amid (bezeichnet als RGD4)

**Revendications**

1. Composés chimiques pour utilisation comme agent de régénération et/ou de traitement de lésions neurologiques tels que :

   H2N-RGD-amide (appelé RGD1),
   H2N-RGDRGD-amide (appelé RGD2),
   H2N-RGDRGDRGa-amide (appelé RGD3),
   acétyl-RGDRGDRGD-amide (appelé Ac-RGD3)
   H2N-RGDRGDRGDRGD-amide (appelé RGD4).

**Fig.1.**

**Fig.2.**

**Fig.3**

26

Fig.4.

Fig.5.

Fig.6

Fig.7.

Fig.8.

Fig.9.

**Fig.10.**

**Fig.11.**

**Fig.12.**

388.2871[M+H]$^+$

1.0e8

Intensity

5.0e7

175.0323
217.1575
149.0573  179.1479     235.2604        333.0790 371.1899          445.2271
0
100              240                  380          450 m/z

**Fig.13.**

PDA Multi 1 226 nm, 4 nm

mAU
300                        10,903

100

0

0        5,0       10,0      15,0    17,5 min

**Fig.14.**

4.2e5   348.9397[M+3H]$^{3+}$

Intensity

2.0e5

522.8859[M+2H]$^{2+}$

310.9185  334.9330
0
100      350      500        800     1100 m/z

Fig.15.

```
Start (0)
 StyI                                                              AccI
 NcoI    BsaBI*                                       BstYI   SalI  HincII  NspI
5' CCATGGATCACCATCACCACCACCACTGGTATTACCCGGAACAGAAACTCATCTCAGAAGAGGATCTGGTCGACATGCAG
   ├─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┤   80
3' GGTACCTAGTGGTAGTGGTGGTGGTGACCATAATGGGCCTTGTCTTTGAGTAGAGTCTTCTCCTAGACCAGCTGTACGTC
        ◄═══════6His═══════►  ◄═WYY═►  ◄═══════════c-Myc tag═══════════►  ■■■■■■►
                                                                          Ubiqutin

          BsrFI                   BstXI
   ATTTTTGTGAAAACCCTGACCGGCAAAACCATTACCCTGGAAGTGGAACCGAGCGATACCATTGAAAATGTGAAAGCGAA
   ├─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┤  160
   TAAAAACACTTTTGGGACTGGCCGTTTTGGTAATGGGACCTTCACCTTGGCTCGCTATGGTAACTTTTACACTTTCGCTT
   ■■■■■■■■■■■■■■■■■■■■■■■■■■■■■■■■■■■■■■Ubiqutin■■■■■■■■■■■■■■■■■■■■■■■■■■■■■■■■■■■■►

                     BsmI                              PvuII
   ApoI         EciI  XmnI                    AlwNI     MspA1I  EcoP15I   EaeI
   AATTCAGGATAAAGAAGGCATTCCGCCGGATCAGCAGCGTCTGATTTTTGCGGGCAAACAGCTGGAAGATGGCCGTACCC
   ├─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┤  240
   TTAAGTCCTATTTCTTCCGTAAGGCGGCCTAGTCGTCGCAGACTAAAAACGCCCGTTTGTCGACCTTCTACCGGCATGGG
   ■■■■■■■■■■■■■■■■■■■■■■■■■■■■■■■■■■■■■■Ubiqutin■■■■■■■■■■■■■■■■■■■■■■■■■■■■■■■■■■■■►

   Bpu10I  PsiI   SspI                       BstAPI                  BspHI
   TGAGCGATTATAATATTCAGAAAGAAAGCACCCTGCATCTGGTGCTGCGTCTGCGTGGCGGCATCATGAGTCGCTCTTCa
   ├─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┤  320
   ACTCGCTAATATTATAAGTCTTTCTTTCGTGGGACGTAGACCACGACGCAGACGCACCGCCGTAGTACTCAGCGAGAAGt
   ■■■■■■■■■■■■■■■■■■■■■■■■■■■■■■■■Ubiqutin■■■■■■■■■■■■■■■■■■■■■■■■■■■■■■►

 SapI    DraIII
   cccCGTGGTGACCGTGGCGATCGTGGTGACCGTGGCGATCGTGGTGACCGTGGCGATCGTGGTGACCGTGGCGATCGTGG
   ├─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┼─┤  400
   gggGCACCACTGGCACCGCTAGCACCACTGGCACCGCTAGCACCACTGGCACCGCTAGCACCACTGGCACCGCTAGCACC
   ◄═══════════════════════════════════RGD*10═══════════════════════════════════►

                    SmlI
                    AvaI
                    XhoI
                    BsoBI  BmeT110I
           SapI     PaeR7I      End (434)
   TGACCGTGGCGATcccaGAAGAGCTTGACTCGAG      3'
   ├─┼─┼─┼─┼─┼─┼─┼─┼─┼─┼─┼─┼─┼─┼─┤        434
   ACTGGCACCGCTAgggtCTTCTCGAACTGAGCTC      5'
   ◄══RGD*10══►
```

**Fig. 16**

Start (0)

StyI
NcoI    BsaBI*                                                           BstYI    SalI    HincII    NspI

AccI

5'  CCATGGATCACCATCACCACCACCACTGGTATTACCCGGAACAGAAACTCATCTCAGAAGAGGATCTGGTCGACATGCAG
3'  GGTACCTAGTGGTAGTGGTGGTGGTGACCATAATGGGCCTTGTCTTTGAGTAGAGTCTTCTCCTAGACCAGCTGTACGTC          80

His-tag          WYY          myc-tag          Ubiquitin

BsrFI          BstXI

ATTTTTGTGAAAACCCTGACCGGCAAAACCATTACCCTGGAAGTGGAACCGAGCGATACCATTGAAAATGTGAAAGCGAA          160
TAAAAACACTTTTGGGACTGGCCGTTTTGGTAATGGGACCTTCACCTTGGCTCGCTATGGTAACTTTTACACTTTCGCTT

Ubiquitin

BsmI
ApoI          EciI    XmnI                          AlwNI                MspA1I EcoP15I

PvuII

AATTCAGGATAAAGAAGGCATTCCGCCGGATCAGCAGCGTCTGATTTTTGCGGGCAAACAGCTGGAAGATGGCCGTACCC          240
TTAAGTCCTATTTCTTCCGTAAGGCGGCCTAGTCGTCGCAGACTAAAAACGCCCGTTTGTCGACCTTCTACCGGCATGGG

Ubiquitin

Bpu10I    PsiI    SspI                          BstAPI                          BspHI

TGAGCGATTATAATATTCAGAAAGAAAGCACCCTGCATCTGGTGCTGCGTCTGCGTGGCGGCATCATGAGTCGCTCTTCa          320
ACTCGCTAATATTATAAGTCTTTCTTTCGTGGGACGTAGACCACGACGCAGACGCACCGCCGTAGTACTCAGCGAGAAGt

Ubiquitin

SapI    DraIII

cccCGTGGTGACGGTGGCCGTGGCGATGGTGGCCGTGGTGACGGTGGCCGTGGCGATGGTGGCCGTGGTGACGGTGGCCG          400
gggGCACCACTGCCACCGGCACCGCTACCACCGGCACCACTGCCACCGGCACCGCTACCACCGGCACCACTGCCACCGGC

RGDGG*10

SapI

TGGCGATGGTGGCCGTGGTGACGGTGGCCGTGGCGATGGTGGCCGTGGTGACGGTGGCCGTGGTGATGGTGGCcccaGAA          480
ACCGCTACCACCGGCACCACTGCCACCGGCACCGCTACCACCGGCACCACTGCCACCGGCACCACTACCACCGgggtCTT

RGDGG*10

SmlI
AvaI
XhoI
BsoBI    BmeT110I
PaeR7I    End (494)

GAGCTTGACTCGAG    3'
CTCGAACTGAGCTC    5'                                                              494

Fig. 17

pET28amutSapIKASETARGD
5659 bp

**Fig. 18**

5′
··· TGGCGAATGGGACGCGCCCTGTAGCGGCGCATTAAGCGCGGCGGGTGTGGTGGTTACGCGCAGCGTGACCGCTACACTTG
3′ ACCGCTTACCCTGCGCGGGACATCGCCGCGTAATTCGCGCCGCCCACACCACCAATGCGCGTCGCACTGGCGATGTGAAC

M13 origin
F1 ori

CCAGCGCCCTAGCGCCCGCTCCTTTCGCTTTCTTCCCTTCCTTTCTCGCCACGTTCGCCGGCTTTCCCCGTCAAGCTCTA
GGTCGCGGGATCGCGGGCGAGGAAAGCGAAAGAAGGGAAGGAAAGAGCGGTGCAAGCGGCCGAAAGGGGCAGTTCGAGAT

M13 origin
F1 ori

AATCGGGGGCTCCCTTTAGGGTTCCGATTTAGTGCTTTACGGCACCTCGACCCCAAAAAACTTGATTAGGGTGATGGTTC
TTAGCCCCCGAGGGAAATCCCAAGGCTAAATCACGAAATGCCGTGGAGCTGGGGTTTTTTGAACTAATCCCACTACCAAG

M13 origin
F1 ori

ACGTAGTGGGCCATCGCCCTGATAGACGGTTTTTCGCCCTTTGACGTTGGAGTCCACGTTCTTTAATAGTGGACTCTTGT
TGCATCACCCGGTAGCGGGACTATCTGCCAAAAAGCGGGAAACTGCAACCTCAGGTGCAAGAAATTATCACCTGAGAACA

M13 origin
F1 ori

TCCAAACTGGAACAACACTCAACCCTATCTCGGTCTATTCTTTTGATTTATAAGGGATTTTGCCGATTTCGGCCTATTGG
AGGTTTGACCTTGTTGTGAGTTGGGATAGAGCCAGATAAGAAAACTAAATATTCCCTAAAACGGCTAAAGCCGGATAACC

M13 origin
F1 ori

TTAAAAAATGAGCTGATTTAACAAAAATTTAACGCGAATTTTTAACAAAATATTAACGTTTACAATTTCAGGTGGCACTTT
AATTTTTTACTCGACTAAATTGTTTTTAAATTGCGCTTAAAATTGTTTTTATAATTGCAAATGTTAAAGTCCACCGTGAAA

M13 origin
F1 ori

TCGGGGAAATGTGCGCGGAACCCCTATTTGTTTATTTTTCTAAATACATTCAAATATGTATCCGCTCATGAATTAATTCT
AGCCCCTTTACACGCGCCTTGGGGATAAACAAATAAAAAGATTTATGTAAGTTTATACATAGGCGAGTACTTAATTAAGA

TAGAAAAACTCATCGAGCATCAAATGAAACTGCAATTTATTCATATCAGGATTATCAATACCATATTTTTGAAAAAGCCG
ATCTTTTTGAGTAGCTCGTAGTTTACTTTGACGTTAAATAAGTATAGTCCTAATAGTTATGGTATAAAAACTTTTTCGGC

KanR

TTTCTGTAATGAAGGAGAAAACTCACCGAGGCAGTTCCATAGGATGGCAAGATCCTGGTATCGGTCTGCGATTCCGACTC
AAAGACATTACTTCCTCTTTTGAGTGGCTCCGTCAAGGTATCCTACCGTTCTAGGACCATAGCCAGACGCTAAGGCTGAG

KanR

GTCCAACATCAATACAACCTATTAATTTCCCCTCGTCAAAAATAAGGTTATCAAGTGAGAAATCACCATGAGTGACGACT
CAGGTTGTAGTTATGTTGGATAATTAAAGGGGAGCAGTTTTTATTCCAATAGTTCACTCTTTAGTGGTACTCACTGCTGA

KanR

GAATCCGGTGAGAATGGCAAAAGTTTATGCATTTCTTTCCAGACTTGTTCAACAGGCCAGCCATTACGCTCGTCATCAAA
CTTAGGCCACTCTTACCGTTTTCAAATACGTAAAGAAAGGTCTGAACAAGTTGTCCGGTCGGTAATGCGAGCAGTAGTTT

KanR

AsiSI

ATCACTCGCATCAACCAAACCGTTATTCATTCGTGATTGCGCCTGAGCGAGACGAAATACGCGATCGCTGTTAAAAGGAC
TAGTGAGCGTAGTTGGTTTGGCAATAAGTAAGCACTAACGCGGACTCGCTCTGCTTTATGCGCTAGCGACAATTTTCCTG

KanR

Fig 19 - continued on next page

```
AATTACAAACAGGAATCGAATGCAACCGGCGCAGGAACACTGCCAGCGCATCAACAATATTTTCACCTGAATCAGGATAT
+-+--+--+--+--+--+--+--+--+--+--+--+--+--+--+--+--+--+--+--+--+--+--+--+--+--+--+
TTAATGTTTGTCCTTAGCTTACGTTGGCCGCGTCCTTGTGACGGTCGCGTAGTTGTTATAAAAGTGGACTTAGTCCTATA
< ▭▭▭▭▭▭▭▭▭▭▭▭▭▭▭▭▭▭▭▭▭▭▭▭▭▭ KanR ▭▭▭▭▭▭▭▭▭▭▭▭▭▭▭▭▭▭▭▭▭▭▭▭▭▭▭▭
```
                                                                                    1040

```
                XmaI
                TspMI  SmaI
TCTTCTAATACCTGGAATGCTGTTTTCCCGGGGATCGCAGTGGTGAGTAACCATGCATCATCAGGAGTACGGATAAAATG
+-+--+--+--+--+--+--+--+--+--+--+--+--+--+--+--+--+--+--+--+--+--+--+--+--+--+--+
AGAAGATTATGGACCTTACGACAAAAGGGCCCCTAGCGTCACCACTCATTGGTACGTAGTAGTCCTCATGCCTATTTTAC
< ▭▭▭▭▭▭▭▭▭▭▭▭▭▭▭▭▭▭▭▭▭▭▭▭▭▭ KanR ▭▭▭▭▭▭▭▭▭▭▭▭▭▭▭▭▭▭▭▭▭▭▭▭▭▭▭▭
```
                                                                                    1120

```
CTTGATGGTCGGAAGAGGCATAAATTCCGTCAGCCAGTTTAGTCTGACCATCTCATCTGTAACATCATTGGCAACGCTAC
+-+--+--+--+--+--+--+--+--+--+--+--+--+--+--+--+--+--+--+--+--+--+--+--+--+--+--+
GAACTACCAGCCTTCTCCGTATTTAAGGCAGTCGGTCAAATCAGACTGGTAGAGTAGACATTGTAGTAACCGTTGCGATG
< ▭▭▭▭▭▭▭▭▭▭▭▭▭▭▭▭▭▭▭▭▭▭▭▭▭▭ KanR ▭▭▭▭▭▭▭▭▭▭▭▭▭▭▭▭▭▭▭▭▭▭▭▭▭▭▭▭
```
                                                                                    1200

```
                                                       ClaI
                                                       BspDI
CTTTGCCATGTTTCAGAAACAACTCTGGCGCATCGGGCTTCCCATACAATCGATAGATTGTCGCACCTGATTGCCCGACA
+-+--+--+--+--+--+--+--+--+--+--+--+--+--+--+--+--+--+--+--+--+--+--+--+--+--+--+
GAAACGGTACAAAGTCTTTGTTGAGACCGCGTAGCCCGAAGGGTATGTTAGCTATCTAACAGCGTGGACTAACGGGCTGT
< ▭▭▭▭▭▭▭▭▭▭▭▭▭▭▭▭▭▭▭▭▭▭▭▭▭▭ KanR ▭▭▭▭▭▭▭▭▭▭▭▭▭▭▭▭▭▭▭▭▭▭▭▭▭▭▭▭
```
                                                                                    1280

```
     NruI
TTATCGCGAGCCCATTTATACCCATATAAATCAGCATCCATGTTGGAATTTAATCGCGGCCTAGAGCAAGACGTTTCCCG
+-+--+--+--+--+--+--+--+--+--+--+--+--+--+--+--+--+--+--+--+--+--+--+--+--+--+--+
AATAGCGCTCGGGTAAATATGGGTATATTTAGTCGTAGGTACAACCTTAAATTAGCGCCGGATCTCGTTCTGCAAAGGGC
< ▭▭▭▭▭▭▭▭▭▭▭▭▭▭▭▭▭▭▭▭▭▭▭▭▭▭ KanR ▭▭▭▭▭▭▭▭▭▭▭▭▭▭▭▭▭▭▭▭▭▭▭▭▭▭▭▭
```
                                                                                    1360

```
TTGAATATGGCTCATAACACCCCTTGTATTACTGTTTATGTAAGCAGACAGTTTTATTGTTCATGACCAAAATCCCTTAA
+-+--+--+--+--+--+--+--+--+--+--+--+--+--+--+--+--+--+--+--+--+--+--+--+--+--+--+
AACTTATACCGAGTATTGTGGGGAACATAATGACAAATACATTCGTCTGTCAAAATAACAAGTACTGGTTTTAGGGAATT
< ▭▭▭▭▭▭ KanR ▭▭▭▭▭
```
                                                                                    1440

```
CGTGAGTTTTCGTTCCACTGAGCGTCAGACCCCGTAGAAAAGATCAAAGGATCTTCTTGAGATCCTTTTTTTCTGCGCGT
+-+--+--+--+--+--+--+--+--+--+--+--+--+--+--+--+--+--+--+--+--+--+--+--+--+--+--+
GCACTCAAAAGCAAGGTGACTCGCAGTCTGGGGCATCTTTTCTAGTTTCCTAGAAGAACTCTAGGAAAAAAAGACGCGCA
                          ▭▭▭▭▭▭▭▭▭▭▭▭▭▭ ColE1 origin ▭▭▭▭▭▭▭▭▭▭▭▭▭ >
```
                                                                                    1520

```
                                                                          AcuI
AATCTGCTGCTTGCAAACAAAAAAACCACCGCTACCAGCGGTGGTTTGTTTGCCGGATCAAGAGCTACCAACTCTTTTTC
+-+--+--+--+--+--+--+--+--+--+--+--+--+--+--+--+--+--+--+--+--+--+--+--+--+--+--+
TTAGACGACGAACGTTTGTTTTTTTGGTGGCGATGGTCGCCACCAAACAAACGGCCTAGTTCTCGATGGTTGAGAAAAAG
▭▭▭▭▭▭▭▭▭▭▭▭▭▭▭▭▭▭▭▭ ColE1 origin ▭▭▭▭▭▭▭▭▭▭▭▭▭▭▭▭▭▭▭▭▭▭▭▭ >
```
                                                                                    1600

```
CGAAGGTAACTGGCTTCAGCAGAGCGCAGATACCAAATACTGTCCTTCTAGTGTAGCCGTAGTTAGGCCACCACTTCAAG
+-+--+--+--+--+--+--+--+--+--+--+--+--+--+--+--+--+--+--+--+--+--+--+--+--+--+--+
GCTTCCATTGACCGAAGTCGTCTCGCGTCTATGGTTTATGACAGGAAGATCACATCGGCATCAATCCGGTGGTGAAGTTC
▭▭▭▭▭▭▭▭▭▭▭▭▭▭▭▭▭▭▭▭ ColE1 origin ▭▭▭▭▭▭▭▭▭▭▭▭▭▭▭▭▭▭▭▭▭▭▭▭ >
```
                                                                                    1680

```
AACTCTGTAGCACCGCCTACATACCTCGCTCTGCTAATCCTGTTACCAGTGGCTGCTGCCAGTGGCGATAAGTCGTGTCT
+-+--+--+--+--+--+--+--+--+--+--+--+--+--+--+--+--+--+--+--+--+--+--+--+--+--+--+
TTGAGACATCGTGGCGGATGTATGGAGCGAGACGATTAGGACAATGGTCACCGACGACGGTCACCGCTATTCAGCACAGA
▭▭▭▭▭▭▭▭▭▭▭▭▭▭▭▭▭▭▭▭ ColE1 origin ▭▭▭▭▭▭▭▭▭▭▭▭▭▭▭▭▭▭▭▭▭▭▭▭ >
```
                                                                                    1760

```
TACCGGGTTGGACTCAAGACGATAGTTACCGGATAAGGCGCAGCGGTCGGGCTGAACGGGGGGTTCGTGCACACAGCCCA
+-+--+--+--+--+--+--+--+--+--+--+--+--+--+--+--+--+--+--+--+--+--+--+--+--+--+--+
ATGGCCCAACCTGAGTTCTGCTATCAATGGCCTATTCCGCGTCGCCAGCCCGACTTGCCCCCCAAGCACGTGTGTCGGGT
▭▭▭▭▭▭▭▭▭▭▭▭▭▭▭▭▭▭▭▭ ColE1 origin ▭▭▭▭▭▭▭▭▭▭▭▭▭▭▭▭▭▭▭▭▭▭▭▭ >
```
                                                                                    1840

```
GCTTGGAGCGAACGACCTACACCGAACTGAGATACCTACAGCGTGAGCTATGAGAAAGCGCCACGCTTCCCGAAGGGAGA
+-+--+--+--+--+--+--+--+--+--+--+--+--+--+--+--+--+--+--+--+--+--+--+--+--+--+--+
CGAACCTCGCTTGCTGGATGTGGCTTGACTCTATGGATGTCGCACTCGATACTCTTTCGCGGTGCGAAGGGCTTCCCTCT
▭▭▭▭▭▭▭▭▭▭▭▭▭▭▭▭▭▭▭▭ ColE1 origin ▭▭▭▭▭▭▭▭▭▭▭▭▭▭▭▭▭▭▭▭▭▭▭▭ >
```
                                                                                    1920

Fig 19 - continued on next page

35

BssSαI

```
AAGGCGGACAGGTATCCGGTAAGCGGCAGGGTCGGAACAGGAGAGCGCACGAGGGAGCTTCCAGGGGGAAACGCCTGGTA
TTCCGCCTGTCCATAGGCCATTCGCCGTCCCAGCCTTGTCCTCTCGCGTGCTCCCTCGAAGGTCCCCCTTTGCGGACCAT   2000
```
ColE1 origin

```
TCTTTATAGTCCTGTCGGGTTTCGCCACCTCTGACTTGAGCGTCGATTTTTGTGATGCTCGTCAGGGGGGCGGAGCCTAT
AGAAATATCAGGACAGCCCAAAGCGGTGGAGACTGAACTCGCAGCTAAAAACACTACGAGCAGTCCCCCCGCCTCGGATA   2080
```
ColE1 origin

PciI

```
GGAAAAACGCCAGCAACGCGGCCTTTTTACGGTTCCTGGCCTTTTGCTGGCCTTTTGCTCACATGTTCTTTCCTGCGTTA
CCTTTTTGCGGTCGTTGCGCCGGAAAAATGCCAAGGACCGGAAAACGACCGGAAAACGAGTGTACAAGAAAGGACGCAAT   2160
```
ColE1 origin

```
TCCCCTGATTCTGTGGATAACCGTATTACCGCCTTTGAGTGAGCTGATACCGCTCGCCGCAGCCGAACGACCGAGCGCAG
AGGGGACTAAGACACCTATTGGCATAATGGCGGAAACTCACTCGACTATGGCGAGCGGCGTCGGCTTGCTGGCTCGCGTC   2240
```

BseRI

```
CGAGTCAGTGAGCGAGGAAGCGGAGGAGCGCCTGATGCGGTATTTTCTCCTTACGCATCTGTGCGGTATTTCACACCGCA
GCTCAGTCACTCGCTCCTTCGCCTCCTCGCGGACTACGCCATAAAAGAGGAATGCGTAGACACGCCATAAAGTGTGGCGT   2320
```

TatI                    BstZ17I                    PflFI
                                                   Tth111I

```
TATATGGTGCACTCTCAGTACAATCTGCTCTGATGCCGCATAGTTAAGCCAGTATACACTCCGCTATCGCTACGTGACTG
ATATACCACGTGAGAGTCATGTTAGACGAGACTACGGCGTATCAATTCGGTCATATGTGAGGCGATAGCGATGCACTGAC   2400
```

```
GGTCATGGCTGCGCCCCGACACCCGCCAACACCCGCTGACGCGCCCTGACGGGCTTGTCTGCTCCCGGCATCCGCTTACA
CCAGTACCGACGCGGGGCTGTGGGCGGTTGTGGGCGACTGCGCGGGACTGCCCGAACAGACGAGGGCCGTAGGCGAATGT   2480
```

```
GACAAGCTGTGACCGTCTCCGGGAGCTGCATGTGTCAGAGGTTTTCACCGTCATCACCGAAACGCGCGAGGCAGCTGCGG
CTGTTCGACACTGGCAGAGGCCCTCGACGTACACAGTCTCCAAAAGTGGCAGTAGTGGCTTTGCGCGCTCCGTCGACGCC   2560
```

```
TAAAGCTCATCAGCGTGGTCGTGAAGCGATTCACAGATGTCTGCCTGTTCATCCGCGTCCAGCTCGTTGAGTTTCTCCAG
ATTTCGAGTAGTCGCACCAGCACTTCGCTAAGTGTCTACAGACGGACAAGTAGGCGCAGGTCGAGCAACTCAAAGAGGTC   2640
```

```
AAGCGTTAATGTCTGGCTTCTGATAAAGCGGGCCATGTTAAGGGCGGTTTTTTCCTGTTTGGTCACTGATGCCTCCGTGT
TTCGCAATTACAGACCGAAGACTATTTCGCCCGGTACAATTCCCGCCAAAAAAGGACAAACCAGTGACTACGGAGGCACA   2720
```

```
AAGGGGGATTTCTGTTCATGGGGGTAATGATACCGATGAAACGAGAGAGGATGCTCACGATACGGGTTACTGATGATGAA
TTCCCCCTAAAGACAAGTACCCCCATTACTATGGCTACTTTGCTCTCTCCTACGAGTGCTATGCCCAATGACTACTACTT   2800
```

```
CATGCCCGGTTACTGGAACGTTGTGAGGGTAAACAACTGGCGGTATGGATGCGGCGGGACCAGAGAAAAATCACTCAGGG
GTACGGGCCAATGACCTTGCAACACTCCCATTTGTTGACCGCCATACCTACGCCGCCCTGGTCTCTTTTTAGTGAGTCCC   2880
```

```
TCAATGCCAGCGCTTCGTTAATACAGATGTAGGTGTTCCACAGGGTAGCCAGCAGCATCCTGCGATGCAGATCCGGAACA
AGTTACGGTCGCGAAGCAATTATGTCTACATCCACAAGGTGTCCCATCGGTCGTCGTAGGACGCTACGTCTAGGCCTTGT   2960
```

```
TAATGGTGCAGGGCGCTGACTTCCGCGTTTCCAGACTTTACGAAACACGGAAACCGAAGACCATTCATGTTGTTGCTCAG
ATTACCACGTCCCGCGACTGAAGGCGCAAAGGTCTGAAATGCTTTGTGCCTTTGGCTTCTGGTAAGTACAACAACGAGTC   3040
```

```
GTCGCAGACGTTTTGCAGCAGCAGTCGCTTCACGTTCGCTCGCGTATCGGTGATTCATTCTGCTAACCAGTAAGGCAACC
CAGCGTCTGCAAAACGTCGTCGTCAGCGAAGTGCAAGCGAGCGCATAGCCACTAAGTAAGACGATTGGTCATTCCGTTGG   3120
```

Fig 19 - continued on next page

```
                                         FspI
PpuMI                                    FspAI                    BglI

CCGCCAGCCTAGCCGGGTCCTCAACGACAGGAGCACGATCATGCGCACCCGTGGGGCCGCCATGCCGGCGATAATGGCCT
GGCGGTCGGATCGGCCCAGGAGTTGCTGTCCTCGTGCTAGTACGCGTGGGCACCCCGGCGGTACGGCCGCTATTACCGGA   3200

GCTTCTCGCCGAAACGTTTGGTGGCGGGACCAGTGACGAAGGCTTGAGCGAGGGCGTGCAAGATTCCGAATACCGCAAGC
CGAAGAGCGGCTTTGCAAACCACCGCCCTGGTCACTGCTTCCGAACTCGCTCCCGCACGTTCTAAGGCTTATGGCGTTCG   3280

GACAGGCCGATCATCGTCGCGCTCCAGCGAAAGCGGTCCTCGCCGAAAATGACCCAGAGCGCTGCCGGCACCTGTCCTAC
CTGTCCGGCTAGTAGCAGCGCGAGGTCGCTTTCGCCAGGAGCGGCTTTTACTGGGTCTCGCGACGGCCGTGGACAGGATG   3360

                        PshAI

GAGTTGCATGATAAAGAAGACAGTCATAAGTGCGGCGACGATAGTCATGCCCCGCGCCCACCGGAAGGAGCTGACTGGGT
CTCAACGTACTATTTCTTCTGTCAGTATTCACGCCGCTGCTATCAGTACGGGGCGCGGGTGGCCTTCCTCGACTGACCCA   3440

TGAAGGCTCTCAAGGGCATCGGTCGAGATCCCGGTGCCTAATGAGTGAGCTAACTTACATTAATTGCGTTGCGCTCACTG
ACTTCCGAGAGTTCCCGTAGCCAGCTCTAGGGCCACGGATTACTCACTCGATTGAATGTAATTAACGCAACGCGAGTGAC   3520
                                                                        lacI

CCCGCTTTCCAGTCGGGAAACCTGTCGTGCCAGCTGCATTAATGAATCGGCCAACGCGCGGGGAGAGGCGGTTTGCGTAT
GGGCGAAAGGTCAGCCCTTTGGACAGCACGGTCGACGTAATTACTTAGCCGGTTGCGCGCCCCTCTCCGCCAAACGCATA   3600
                                      lacI

TGGGCGCCAGGGTGGTTTTTCTTTTCACCAGTGAGACGGGCAACAGCTGATTGCCCTTCACCGCCTGGCCCTGAGAGAGT
ACCCGCGGTCCCACCAAAAAGAAAAGTGGTCACTCTGCCCGTTGTCGACTAACGGGAAGTGGCGGACCGGGACTCTCTCA   3680
                                      lacI

                                                  HpaI

TGCAGCAAGCGGTCCACGCTGGTTTGCCCCAGCAGGCGAAAATCCTGTTTGATGGTGGTTAACGGCGGGATATAACATGA
ACGTCGTTCGCCAGGTGCGACCAAACGGGGTCGTCCGCTTTTAGGACAAACTACCACCAATTGCCGCCCTATATTGTACT   3760
                                      lacI

                        EcoRV                             BssHII

GCTGTCTTCGGTATCGTCGTATCCCACTACCGAGATATCCGCACCAACGCGCAGCCCGGACTCGGTAATGGCGCGCATTG
CGACAGAAGCCATAGCAGCATAGGGTGATGGCTCTATAGGCGTGGTTGCGCGTCGGGCCTGAGCCATTACCGCGCGTAAC   3840
                                      lacI

CGCCCAGCGCCATCTGATCGTTGGCAACCAGCATCGCAGTGGGAACGATGCCCTCATTCAGCATTTGCATGGTTTGTTGA
GCGGGTCGCGGTAGACTAGCAACCGTTGGTCGTAGCGTCACCCTTGCTACGGGAGTAAGTCGTAAACGTACCAAACAACT   3920
                                      lacI

AAACCGGACATGGCACTCCAGTCGCCTTCCCGTTCCGCTATCGGCTGAATTTGATTGCGAGTGAGATATTTATGCCAGCC
TTTGGCCTGTACCGTGAGGTCAGCGGAAGGGCAAGGCGATAGCCGACTTAAACTAACGCTCACTCTATAAATACGGTCGG   4000
                                      lacI

                PspOMI   ApaI    NmeAIII

AGCCAGACGCAGACGCGCCGAGACAGAACTTAATGGGCCCGCTAACAGCGCGATTTGCTGGTGACCCAATGCGACCAGAT
TCGGTCTGCGTCTGCGCGGCTCTGTCTTGAATTACCCGGGCGATTGTCGCGCTAAACGACCACTGGGTTACGCTGGTCTA   4080
                                      lacI
```

Fig 19 - continued on next page

```
GCTCCACGCCCAGTCGCGTACCGTCTTCATGGGAGAAAATAATACTGTTGATGGGTGTCTGGTCAGAGACATCAAGAAAT
+-+-+-+++-+|+-+-+-++-+|++-+-+-++-+|+-+-+-++-+|-+-+-+++-+|-+-+-+++-+|++-+-+-++-+|++
CGAGGTGCGGGTCAGCGCATGGCAGAAGTACCCTCTTTTATTATGACAACTACCCACAGACCAGTCTCTGTAGTTCTTTA
<         lacI
```
4160

                                                                          BclI*
```
AACGCCGGAACATTAGTGCAGGCAGCTTCCACAGCAATGGCATCCTGGTCATCCAGCGGATAGTTAATGATCAGCCCACT
+-+-+-+++-+|+-+-+-++-+|++-+-+-++-+|+-+-+-++-+|-+-+-+++-+|-+-+-+++-+|++-+-+-++-+|++
TTGCGGCCTTGTAATCACGTCCGTCGAAGGTGTCGTTACCGTAGGACCAGTAGGTCGCCTATCAATTACTAGTCGGGTGA
<         lacI
```
4240

```
MluI
 |
GACGCGTTGCGCGAGAAGATTGTGCACCGCCGCTTTACAGGCTTCGACGCCGCTTCGTTCTACCATCGACACCACCACGC
+-+-+-+++-+|+-+-+-++-+|++-+-+-++-+|+-+-+-++-+|-+-+-+++-+|-+-+-+++-+|++-+-+-++-+|++
CTGCGCAACGCGCTCTTCTAACACGTGGCGGCGAAATGTCCGAAGCTGCGGCGAAGCAAGATGGTAGCTGTGGTGGTGCG
<         lacI
```
4320

```
TGGCACCCAGTTGATCGGCGCGAGATTTAATCGCCGCGACAATTTGCGACGGCGCGTGCAGGGCCAGACTGGAGGTGGCA
+-+-+-+++-+|+-+-+-++-+|++-+-+-++-+|+-+-+-++-+|-+-+-+++-+|-+-+-+++-+|++-+-+-++-+|++
ACCGTGGGTCAACTAGCCGCGCTCTAAATTAGCGGCGCTGTTAAACGCTGCCGCGCACGTCCCGGTCTGACCTCCACCGT
<         lacI
```
4400

```
ACGCCAATCAGCAACGACTGTTTGCCCGCCAGTTGTTGTGCCACGCGGTTGGGAATGTAATTCAGCTCCGCCATCGCCGC
+-+-+-+++-+|+-+-+-++-+|++-+-+-++-+|+-+-+-++-+|-+-+-+++-+|-+-+-+++-+|++-+-+-++-+|++
TGCGGTTAGTCGTTGCTGACAAACGGGCGGTCAACAACACGGTGCGCCAACCCTTACATTAAGTCGAGGCGGTAGCGGCG
<         lacI
```
4480

```
TTCCACTTTTTCCCGCGTTTTCGCAGAAACGTGGCTGGCCTGGTTCACCACGCGGGAAACGGTCTGATAAGAGACACCGG
+-+-+-+++-+|+-+-+-++-+|++-+-+-++-+|+-+-+-++-+|-+-+-+++-+|-+-+-+++-+|++-+-+-++-+|++
AAGGTGAAAAAGGGCGCAAAAGCGTCTTTGCACCGACCGGACCAAGTGGTGCGCCCTTTGCCAGACTATTCTCTGTGGCC
<         lacI
```
4560

```
CATACTCTGCGACATCGTATAACGTTACTGGTTTCACATTCACCACCCTGAATTGACTCTCTTCCGGGCGCTATCATGCC
+-+-+-+++-+|+-+-+-++-+|++-+-+-++-+|+-+-+-++-+|-+-+-+++-+|-+-+-+++-+|++-+-+-++-+|++
GTATGAGACGCTGTAGCATATTGCAATGACCAAAGTGTAAGTGGTGGGACTTAACTGAGAGAAGGCCCGCGATAGTACGG
<      lacI
```
4640

```
ATACCGCGAAAGGTTTTGCGCCATTCGATGGTGTCCGGGATCTCGACGCTCTCCCTTATGCGACTCCTGCATTAGGAAGC
+-+-+-+++-+|+-+-+-++-+|++-+-+-++-+|+-+-+-++-+|-+-+-+++-+|-+-+-+++-+|++-+-+-++-+|++
TATGGCGCTTTCCAAAACGCGGTAAGCTACCACAGGCCCTAGAGCTGCGAGAGGGAATACGCTGAGGACGTAATCCTTCG
```
4720

                                                      SphI
                                                       |
```
AGCCCAGTAGTAGGTTGAGGCCGTTGAGCACCGCCGCCGCAAGGAATGGTGCATGCAAGGAGATGGCGCCCAACAGTCCC
+-+-+-+++-+|+-+-+-++-+|++-+-+-++-+|+-+-+-++-+|-+-+-+++-+|-+-+-+++-+|++-+-+-++-+|++
TCGGGTCATCATCCAACTCCGGCAACTCGTGGCGGCGGCGTTCCTTACCACGTACGTTCCTCTACCGCGGGTTGTCAGGG
```
4800

```
CCGGCCACGGGGCCTGCCACCATACCCACGCCGAAACAAGCGCTCATGAGCCCGAAGTGGCGAGCCCGATCTTCCCCATC
+-+-+-+++-+|+-+-+-++-+|++-+-+-++-+|+-+-+-++-+|-+-+-+++-+|-+-+-+++-+|++-+-+-++-+|++
GGCCGGTGCCCCGGACGGTGGTATGGGTGCGGCTTTGTTCGCGAGTACTCGGGCTTCACCGCTCGGGCTAGAAGGGGTAG
```
4880

                                                SgrAI
                                                 |
```
GGTGATGTCGGCGATATAGGCGCCAGCAACCGCACCTGTGGCGCCGGTGATGCCGGCCACGATGCGTCCGGCGTAGAGGA
+-+-+-+++-+|+-+-+-++-+|++-+-+-++-+|+-+-+-++-+|-+-+-+++-+|-+-+-+++-+|++-+-+-++-+|++
CCACTACAGCCGCTATATCCGCGGTCGTTGGCGTGGACACCGCGGCCACTACGGCCGGTGCTACGCAGGCCGCATCTCCT
```
4960

```
BglII                                                                     XbaI
 |                                                                         |
TCGAGATCTCGATCCCGCGAAATTAATACGACTCACTATAGGGGAATTGTGAGCGGATAACAATTCCCCTCTAGAAATAA
+-+-+-+++-+|+-+-+-++-+|++-+-+-++-+|+-+-+-++-+|-+-+-+++-+|-+-+-+++-+|++-+-+-++-+|++
AGCTCTAGAGCTAGGGCGCTTTAATTATGCTGAGTGATATCCCCTTAACACTCGCCTATTGTTAAGGGGAGATCTTTATT
                                              LacO >
                T7 >
```
5040

Fig 19 - continued on next page

Fig. 19

**pET28amutSapIKASETARGDGG**
5719 bp

**Fig. 20**

5′ TGGCGAATGGGACGCGCCCTGTAGCGGCGCATTAAGCGCGGCGGGTGTGGTGGTTACGCGCAGCGTGACCGCTACACTTG
3′ ACCGCTTACCCTGCGCGGGACATCGCCGCGTAATTCGCGCCGCCCACACCACCAATGCGCGTCGCACTGGCGATGTGAAC  80
M13 origin
F1 ori

CCAGCGCCCTAGCGCCCGCTCCTTTCGCTTTCTTCCCTTCCTTTCTCGCCACGTTCGCCGGCTTTCCCCGTCAAGCTCTA
GGTCGCGGGATCGCGGGCGAGGAAAGCGAAAGAAGGGAAGGAAAGAGCGGTGCAAGCGGCCGAAAGGGGCAGTTCGAGAT  160
M13 origin
F1 ori

AATCGGGGGCTCCCTTTAGGGTTCCGATTTAGTGCTTTACGGCACCTCGACCCCAAAAAACTTGATTAGGGTGATGGTTC
TTAGCCCCCGAGGGAAATCCCAAGGCTAAATCACGAAATGCCGTGGAGCTGGGGTTTTTTGAACTAATCCCACTACCAAG  240
M13 origin
F1 ori

ACGTAGTGGGCCATCGCCCTGATAGACGGTTTTTCGCCCTTTGACGTTGGAGTCCACGTTCTTTAATAGTGGACTCTTGT
TGCATCACCCGGTAGCGGGACTATCTGCCAAAAAGCGGGAAACTGCAACCTCAGGTGCAAGAAATTATCACCTGAGAACA  320
M13 origin
F1 ori

TCCAAACTGGAACAACACTCAACCCTATCTCGGTCTATTCTTTTGATTTATAAGGGATTTTGCCGATTTCGGCCTATTGG
AGGTTTGACCTTGTTGTGAGTTGGGATAGAGCCAGATAAGAAACTAAATATTCCCTAAAACGGCTAAAGCCGGATAACC  400
M13 origin
F1 ori

TTAAAAAATGAGCTGATTTAACAAAAATTTAACGCGAATTTTAACAAAATATTAACGTTTACAATTTCAGGTGGCACTTT
AATTTTTTACTCGACTAAATTGTTTTTAAATTGCGCTTAAAATTGTTTTTATAATTGCAAATGTTAAAGTCCACCGTGAAA  480
M13 origin
F1 ori

TCGGGGAAATGTGCGCGGAACCCCTATTTGTTTATTTTTCTAAATACATTCAAATATGTATCCGCTCATGAATTAATTCT
AGCCCCTTTACACGCGCCTTGGGGATAAACAAATAAAAAGATTTATGTAAGTTTATACATAGGCGAGTACTTAATTAAGA  560

TAGAAAAACTCATCGAGCATCAAATGAAACTGCAATTTATTCATATCAGGATTATCAATACCATATTTTTGAAAAAGCCG
ATCTTTTTGAGTAGCTCGTAGTTTACTTTGACGTTAAATAAGTATAGTCCTAATAGTTATGGTATAAAAACTTTTTCGGC  640
KanR

TTTCTGTAATGAAGGAGAAAACTCACCGAGGCAGTTCCATAGGATGGCAAGATCCTGGTATCGGTCTGCGATTCCGACTC
AAAGACATTACTTCCTCTTTTGAGTGGCTCCGTCAAGGTATCCTACCGTTCTAGGACCATAGCCAGACGCTAAGGCTGAG  720
KanR

GTCCAACATCAATACAACCTATTAATTTCCCCTCGTCAAAAATAAGGTTATCAAGTGAGAAATCACCATGAGTGACGACT
CAGGTTGTAGTTATGTTGGATAATTAAAGGGGAGCAGTTTTTATTCCAATAGTTCACTCTTTAGTGGTACTCACTGCTGA  800
KanR

GAATCCGGTGAGAATGGCAAAAGTTTATGCATTTCTTTCCAGACTTGTTCAACAGGCCAGCCATTACGCTCGTCATCAAA
CTTAGGCCACTCTTACCGTTTTTCAAATACGTAAAGAAAGGTCTGAACAAGTTGTCCGGTCGGTAATGCGAGCAGTAGTTT  880
KanR

Fig 21 - continued on netx page

41

PvuI
AsiSI

```
ATCACTCGCATCAACCAAACCGTTATTCATTCGTGATTGCGCCTGAGCGAGACGAAATACGCGATCGCTGTTAAAAGGAC
++++++++++|++++++++++|++++++++++|++++++++++|++++++++++|++++++++++|++++++++++|++++++++++|    960
TAGTGAGCGTAGTTGGTTTGGCAATAAGTAAGCACTAACGCGGACTCGCTCTGCTTTATGCGCTAGCGACAATTTTCCTG
                                   KanR
```

```
AATTACAAACAGGAATCGAATGCAACCGGCGCAGGAACACTGCCAGCGCATCAACAATATTTTCACCTGAATCAGGATAT
++++++++++|++++++++++|++++++++++|++++++++++|++++++++++|++++++++++|++++++++++|++++++++++|    1040
TTAATGTTTGTCCTTAGCTTACGTTGGCCGCGTCCTTGTGACGGTCGCGTAGTTGTTATAAAAGTGGACTTAGTCCTATA
                                   KanR
```

XmaI
TspMI    SmaI

```
TCTTCTAATACCTGGAATGCTGTTTTCCCGGGGATCGCAGTGGTGAGTAACCATGCATCATCAGGAGTACGGATAAAATG
++++++++++|++++++++++|++++++++++|++++++++++|++++++++++|++++++++++|++++++++++|++++++++++|    1120
AGAAGATTATGGACCTTACGACAAAAGGGCCCCTAGCGTCACCACTCATTGGTACGTAGTAGTCCTCATGCCTATTTTAC
                                   KanR
```

```
CTTGATGGTCGGAAGAGGCATAAATTCCGTCAGCCAGTTTAGTCTGACCATCTCATCTGTAACATCATTGGCAACGCTAC
++++++++++|++++++++++|++++++++++|++++++++++|++++++++++|++++++++++|++++++++++|++++++++++|    1200
GAACTACCAGCCTTCTCCGTATTTAAGGCAGTCGGTCAAATCAGACTGGTAGAGTAGACATTGTAGTAACCGTTGCGATG
                                   KanR
```

ClaI
BspDI

```
CTTTGCCATGTTTCAGAAACAACTCTGGCGCATCGGGCTTCCCATACAATCGATAGATTGTCGCACCTGATTGCCCGACA
++++++++++|++++++++++|++++++++++|++++++++++|++++++++++|++++++++++|++++++++++|++++++++++|    1280
GAAACGGTACAAAGTCTTTGTTGAGACCGCGTAGCCCGAAGGGTATGTTAGCTATCTAACAGCGTGGACTAACGGGCTGT
                                   KanR
```

NruI

```
TTATCGCGAGCCCATTTATACCCATATAAATCAGCATCCATGTTGGAATTTAATCGCGGCCTAGAGCAAGACGTTTCCCG
++++++++++|++++++++++|++++++++++|++++++++++|++++++++++|++++++++++|++++++++++|++++++++++|    1360
AATAGCGCTCGGGTAAATATGGGTATATTTAGTCGTAGGTACAACCTTAAATTAGCGCCGGATCTCGTTCTGCAAAGGGC
                                   KanR
```

```
TTGAATATGGCTCATAACACCCCTTGTATTACTGTTTATGTAAGCAGACAGTTTTATTGTTCATGACCAAAATCCCTTAA
++++++++++|++++++++++|++++++++++|++++++++++|++++++++++|++++++++++|++++++++++|++++++++++|    1440
AACTTATACCGAGTATTGTGGGGAACATAATGACAAATACATTCGTCTGTCAAAATAACAAGTACTGGTTTTAGGGAATT
     KanR
```

```
CGTGAGTTTTCGTTCCACTGAGCGTCAGACCCCGTAGAAAAGATCAAAGGATCTTCTTGAGATCCTTTTTTTCTGCGCGT
++++++++++|++++++++++|++++++++++|++++++++++|++++++++++|++++++++++|++++++++++|++++++++++|    1520
GCACTCAAAAGCAAGGTGACTCGCAGTCTGGGGCATCTTTTCTAGTTTCCTAGAAGAACTCTAGGAAAAAAAGACGCGCA
                                   ColE1 origin                                    >
```

AcuI

```
AATCTGCTGCTTGCAAACAAAAAAACCACCGCTACCAGCGGTGGTTTGTTTGCCGGATCAAGAGCTACCAACTCTTTTTC
++++++++++|++++++++++|++++++++++|++++++++++|++++++++++|++++++++++|++++++++++|++++++++++|    1600
TTAGACGACGAACGTTTGTTTTTTTGGTGGCGATGGTCGCCACCAAACAAACGGCCTAGTTCTCGATGGTTGAGAAAAAG
                    ColE1 origin                                                  >
```

```
CGAAGGTAACTGGCTTCAGCAGAGCGCAGATACCAAATACTGTCCTTCTAGTGTAGCCGTAGTTAGGCCACCACTTCAAG
++++++++++|++++++++++|++++++++++|++++++++++|++++++++++|++++++++++|++++++++++|++++++++++|    1680
GCTTCCATTGACCGAAGTCGTCTCGCGTCTATGGTTTATGACAGGAAGATCACATCGGCATCAATCCGGTGGTGAAGTTC
                    ColE1 origin                                                  >
```

```
AACTCTGTAGCACCGCCTACATACCTCGCTCTGCTAATCCTGTTACCAGTGGCTGCTGCCAGTGGCGATAAGTCGTGTCT
++++++++++|++++++++++|++++++++++|++++++++++|++++++++++|++++++++++|++++++++++|++++++++++|    1760
TTGAGACATCGTGGCGGATGTATGGAGCGAGACGATTAGGACAATGGTCACCGACGACGGTCACCGCTATTCAGCACAGA
                    ColE1 origin                                                  >
```

Fig 21 - continued on netx page

```
TACCGGGTTGGACTCAAGACGATAGTTACCGGATAAGGCGCAGCGGTCGGGCTGAACGGGGGGGTTCGTGCACACAGCCCA
+-+-+-+-+-+-+|+-+-+-+-+-+-+|+-+-+-+-+-+-+|+-+-+-+-+-+-+|+-+-+-+-+-+-+|+-+-+-+-+-+-+|     1840
ATGGCCCAACCTGAGTTCTGCTATCAATGGCCTATTCCGCGTCGCCAGCCCGACTTGCCCCCCAAGCACGTGTGTCGGGT
```
ColE1 origin >

```
GCTTGGAGCGAACGACCTACACCGAACTGAGATACCTACAGCGTGAGCTATGAGAAAGCGCCACGCTTCCCGAAGGGAGA
+-+-+-+-+-+-+|+-+-+-+-+-+-+|+-+-+-+-+-+-+|+-+-+-+-+-+-+|+-+-+-+-+-+-+|+-+-+-+-+-+-+|     1920
CGAACCTCGCTTGCTGGATGTGGCTTGACTCTATGGATGTCGCACTCGATACTCTTTCGCGGTGCGAAGGGCTTCCCTCT
```
ColE1 origin >

BsssSaI
```
AAGGCGGACAGGTATCCGGTAAGCGGCAGGGTCGGAACAGGAGAGCGCACGAGGGAGCTTCCAGGGGGAAACGCCTGGTA
+-+-+-+-+-+-+|+-+-+-+-+-+-+|+-+-+-+-+-+-+|+-+-+-+-+-+-+|+-+-+-+-+-+-+|+-+-+-+-+-+-+|     2000
TTCCGCCTGTCCATAGGCCATTCGCCGTCCCAGCCTTGTCCTCTCGCGTGCTCCCTCGAAGGTCCCCCTTTGCGGACCAT
```
ColE1 origin >

```
TCTTTATAGTCCTGTCGGGTTTCGCCACCTCTGACTTGAGCGTCGATTTTTGTGATGCTCGTCAGGGGGGCGGAGCCTAT
+-+-+-+-+-+-+|+-+-+-+-+-+-+|+-+-+-+-+-+-+|+-+-+-+-+-+-+|+-+-+-+-+-+-+|+-+-+-+-+-+-+|     2080
AGAAATATCAGGACAGCCCAAAGCGGTGGAGACTGAACTCGCAGCTAAAAACACTACGAGCAGTCCCCCCGCCTCGGATA
```
ColE1 origin >

PciI
```
GGAAAAACGCCAGCAACGCGGCCTTTTTACGGTTCCTGGCCTTTTGCTGGCCTTTTGCTCACATGTTCTTTCCTGCGTTA
+-+-+-+-+-+-+|+-+-+-+-+-+-+|+-+-+-+-+-+-+|+-+-+-+-+-+-+|+-+-+-+-+-+-+|+-+-+-+-+-+-+|     2160
CCTTTTTGCGGTCGTTGCGCCGGAAAAATGCCAAGGACCGGAAAACGACCGGAAAACGAGTGTACAAGAAAGGACGCAAT
```
ColE1 origin >

```
TCCCCTGATTCTGTGGATAACCGTATTACCGCCTTTGAGTGAGCTGATACCGCTCGCCGCAGCCGAACGACCGAGCGCAG
+-+-+-+-+-+-+|+-+-+-+-+-+-+|+-+-+-+-+-+-+|+-+-+-+-+-+-+|+-+-+-+-+-+-+|+-+-+-+-+-+-+|     2240
AGGGGACTAAGACACCTATTGGCATAATGGCGGAAACTCACTCGACTATGGCGAGCGGCGTCGGCTTGCTGGCTCGCGTC
```

BseRI
```
CGAGTCAGTGAGCGAGGAAGCGGAGGAGCGCCTGATGCGGTATTTTCTCCTTACGCATCTGTGCGGTATTTCACACCGCA
+-+-+-+-+-+-+|+-+-+-+-+-+-+|+-+-+-+-+-+-+|+-+-+-+-+-+-+|+-+-+-+-+-+-+|+-+-+-+-+-+-+|     2320
GCTCAGTCACTCGCTCCTTCGCCTCCTCGCGGACTACGCCATAAAAGAGGAATGCGTAGACACGCCATAAAGTGTGGCGT
```

TatI                                                      BstZ17I                        PflFI
                                                                                         Tth111I
```
TATATGGTGCACTCTCAGTACAATCTGCTCTGATGCCGCATAGTTAAGCCAGTATACACTCCGCTATCGCTACGTGACTG
+-+-+-+-+-+-+|+-+-+-+-+-+-+|+-+-+-+-+-+-+|+-+-+-+-+-+-+|+-+-+-+-+-+-+|+-+-+-+-+-+-+|     2400
ATATACCACGTGAGAGTCATGTTAGACGAGACTACGGCGTATCAATTCGGTCATATGTGAGGCGATAGCGATGCACTGAC
```

```
GGTCATGGCTGCGCCCCGACACCCGCCAACACCCGCTGACGCGCCCTGACGGGCTTGTCTGCTCCCGGCATCCGCTTACA
+-+-+-+-+-+-+|+-+-+-+-+-+-+|+-+-+-+-+-+-+|+-+-+-+-+-+-+|+-+-+-+-+-+-+|+-+-+-+-+-+-+|     2480
CCAGTACCGACGCGGGGCTGTGGGCGGTTGTGGGCGACTGCGCGGGACTGCCCGAACAGACGAGGGCCGTAGGCGAATGT
```

```
GACAAGCTGTGACCGTCTCCGGGAGCTGCATGTGTCAGAGGTTTTCACCGTCATCACCGAAACGCGCGAGGCAGCTGCGG
+-+-+-+-+-+-+|+-+-+-+-+-+-+|+-+-+-+-+-+-+|+-+-+-+-+-+-+|+-+-+-+-+-+-+|+-+-+-+-+-+-+|     2560
CTGTTCGACACTGGCAGAGGCCCTCGACGTACACAGTCTCCAAAAGTGGCAGTAGTGGCTTTGCGCGCTCCGTCGACGCC
```

```
TAAAGCTCATCAGCGTGGTCGTGAAGCGATTCACAGATGTCTGCCTGTTCATCCGCGTCCAGCTCGTTGAGTTTCTCCAG
+-+-+-+-+-+-+|+-+-+-+-+-+-+|+-+-+-+-+-+-+|+-+-+-+-+-+-+|+-+-+-+-+-+-+|+-+-+-+-+-+-+|     2640
ATTTCGAGTAGTCGCACCAGCACTTCGCTAAGTGTCTACAGACGGACAAGTAGGCGCAGGTCGAGCAACTCAAAGAGGTC
```

```
AAGCGTTAATGTCTGGCTTCTGATAAAGCGGGCCATGTTAAGGGCGGTTTTTTCCTGTTTGGTCACTGATGCCTCCGTGT
+-+-+-+-+-+-+|+-+-+-+-+-+-+|+-+-+-+-+-+-+|+-+-+-+-+-+-+|+-+-+-+-+-+-+|+-+-+-+-+-+-+|     2720
TTCGCAATTACAGACCGAAGACTATTTCGCCCGGTACAATTCCCGCCAAAAAAGGACAAACCAGTGACTACGGAGGCACA
```

```
AAGGGGGATTTCTGTTCATGGGGGTAATGATACCGATGAAACGAGAGAGGATGCTCACGATACGGGTTACTGATGATGAA
+-+-+-+-+-+-+|+-+-+-+-+-+-+|+-+-+-+-+-+-+|+-+-+-+-+-+-+|+-+-+-+-+-+-+|+-+-+-+-+-+-+|     2800
TTCCCCCTAAAGACAAGTACCCCCATTACTATGGCTACTTTGCTCTCTCCTACGAGTGCTATGCCCAATGACTACTACTT
```

```
CATGCCCGGTTACTGGAACGTTGTGAGGGTAAACAACTGGCGGTATGGATGCGGCGGGACCAGAGAAAAATCACTCAGGG
+-+-+-+-+-+-+|+-+-+-+-+-+-+|+-+-+-+-+-+-+|+-+-+-+-+-+-+|+-+-+-+-+-+-+|+-+-+-+-+-+-+|     2880
GTACGGGCCAATGACCTTGCAACACTCCCATTTGTTGACCGCCATACCTACGCCGCCCTGGTCTCTTTTTAGTGAGTCCC
```

Fig 21 - continued on netx page

```
TCAATGCCAGCGCTTCGTTAATACAGATGTAGGTGTTCCACAGGGTAGCCAGCAGCATCCTGCGATGCAGATCCGGAACA
+--+-+-+-+--+-+--+-+--+-+--+-+--+-+--+-+--+-+--+-+--+-+--+-+--+-+--+-+--+-+--+-+--+
AGTTACGGTCGCGAAGCAATTATGTCTACATCCACAAGGTGTCCCATCGGTCGTCGTAGGACGCTACGTCTAGGCCTTGT    2960

TAATGGTGCAGGGCGCTGACTTCCGCGTTTCCAGACTTTACGAAACACGGAAACCGAAGACCATTCATGTTGTTGCTCAG
+--+-+-+-+--+-+--+-+--+-+--+-+--+-+--+-+--+-+--+-+--+-+--+-+--+-+--+-+--+-+--+-+--+
ATTACCACGTCCCGCGACTGAAGGCGCAAAGGTCTGAAATGCTTTGTGCCTTTGGCTTCTGGTAAGTACAACAACGAGTC    3040

GTCGCAGACGTTTTGCAGCAGCAGTCGCTTCACGTTCGCTCGCGTATCGGTGATTCATTCTGCTAACCAGTAAGGCAACC
+--+-+-+-+--+-+--+-+--+-+--+-+--+-+--+-+--+-+--+-+--+-+--+-+--+-+--+-+--+-+--+-+--+
CAGCGTCTGCAAAACGTCGTCGTCAGCGAAGTGCAAGCGAGCGCATAGCCACTAAGTAAGACGATTGGTCATTCCGTTGG    3120
```

```
                                           FspI
         PpuMI                             FspAI                    BglI
          |                                  |                        |
CCGCCAGCCTAGCCGGGTCCTCAACGACAGGAGCACGATCATGCGCACCCGTGGGGCCGCCATGCCGGCGATAATGGCCT
+--+-+-+-+--+-+--+-+--+-+--+-+--+-+--+-+--+-+--+-+--+-+--+-+--+-+--+-+--+-+--+-+--+
GGCGGTCGGATCGGCCCAGGAGTTGCTGTCCTCGTGCTAGTACGCGTGGGCACCCCGGCGGTACGGCCGCTATTACCGGA    3200

GCTTCTCGCCGAAACGTTTGGTGGCGGGACCAGTGACGAAGGCTTGAGCGAGGGCGTGCAAGATTCCGAATACCGCAAGC
+--+-+-+-+--+-+--+-+--+-+--+-+--+-+--+-+--+-+--+-+--+-+--+-+--+-+--+-+--+-+--+-+--+
CGAAGAGCGGCTTTGCAAACCACCGCCCTGGTCACTGCTTCCGAACTCGCTCCCGCACGTTCTAAGGCTTATGGCGTTCG    3280

GACAGGCCGATCATCGTCGCGCTCCAGCGAAAGCGGTCCTCGCCGAAAATGACCCAGAGCGCTGCCGGCACCTGTCCTAC
+--+-+-+-+--+-+--+-+--+-+--+-+--+-+--+-+--+-+--+-+--+-+--+-+--+-+--+-+--+-+--+-+--+
CTGTCCGGCTAGTAGCAGCGCGAGGTCGCTTTCGCCAGGAGCGGCTTTTACTGGGTCTCGCGACGGCCGTGGACAGGATG    3360
```

```
                                PshAI
                                  |
GAGTTGCATGATAAAGAAGACAGTCATAAGTGCGGCGACGATAGTCATGCCCCGCGCCCACCGGAAGGAGCTGACTGGGT
+--+-+-+-+--+-+--+-+--+-+--+-+--+-+--+-+--+-+--+-+--+-+--+-+--+-+--+-+--+-+--+-+--+
CTCAACGTACTATTTCTTCTGTCAGTATTCACGCCGCTGCTATCAGTACGGGGCGCGGGTGGCCTTCCTCGACTGACCCA    3440

TGAAGGCTCTCAAGGGCATCGGTCGAGATCCCGGTGCCTAATGAGTGAGCTAACTTACATTAATTGCGTTGCGCTCACTG
+--+-+-+-+--+-+--+-+--+-+--+-+--+-+--+-+--+-+--+-+--+-+--+-+--+-+--+-+--+-+--+-+--+
ACTTCCGAGAGTTCCCGTAGCCAGCTCTAGGGCCACGGATTACTCACTCGATTGAATGTAATTAACGCAACGCGAGTGAC    3520
                                                                            [lacI]
```

```
CCCGCTTTCCAGTCGGGAAACCTGTCGTGCCAGCTGCATTAATGAATCGGCCAACGCGCGGGGAGAGGCGGTTTGCGTAT
+--+-+-+-+--+-+--+-+--+-+--+-+--+-+--+-+--+-+--+-+--+-+--+-+--+-+--+-+--+-+--+-+--+
GGGCGAAAGGTCAGCCCTTTGGACAGCACGGTCGACGTAATTACTTAGCCGGTTGCGCGCCCCTCTCCGCCAAACGCATA    3600
   [<          lacI          ]

TGGGCGCCAGGGTGGTTTTTCTTTTCACCAGTGAGACGGGCAACAGCTGATTGCCCTTCACCGCCTGGCCCTGAGAGAGT
+--+-+-+-+--+-+--+-+--+-+--+-+--+-+--+-+--+-+--+-+--+-+--+-+--+-+--+-+--+-+--+-+--+
ACCCGCGGTCCCACCAAAAAGAAAAGTGGTCACTCTGCCCGTTGTCGACTAACGGGAAGTGGCGGACCGGGACTCTCTCA    3680
   [<          lacI          ]
```

```
                                        HpaI
                                          |
TGCAGCAAGCGGTCCACGCTGGTTTGCCCCAGCAGGCGAAAATCCTGTTTGATGGTGGTTAACGGCGGGATATAACATGA
+--+-+-+-+--+-+--+-+--+-+--+-+--+-+--+-+--+-+--+-+--+-+--+-+--+-+--+-+--+-+--+-+--+
ACGTCGTTCGCCAGGTGCGACCAAACGGGGTCGTCCGCTTTTAGGACAAACTACCACCAATTGCCGCCCTATATTGTACT    3760
   [<          lacI          ]
```

```
                              EcoRV                              BssHII
                                |                                  |
GCTGTCTTCGGTATCGTCGTATCCCACTACCGAGATATCCGCACCAACGCGCAGCCCGGACTCGGTAATGGCGCGCATTG
+--+-+-+-+--+-+--+-+--+-+--+-+--+-+--+-+--+-+--+-+--+-+--+-+--+-+--+-+--+-+--+-+--+
CGACAGAAGCCATAGCAGCATAGGGTGATGGCTCTATAGGCGTGGTTGCGCGTCGGGCCTGAGCCATTACCGCGCGTAAC    3840
   [<          lacI          ]

CGCCCAGCGCCATCTGATCGTTGGCAACCAGCATCGCAGTGGGAACGATGCCCTCATTCAGCATTTGCATGGTTTGTTGA
+--+-+-+-+--+-+--+-+--+-+--+-+--+-+--+-+--+-+--+-+--+-+--+-+--+-+--+-+--+-+--+-+--+
GCGGGTCGCGGTAGACTAGCAACCGTTGGTCGTAGCGTCACCCTTGCTACGGGAGTAAGTCGTAAACGTACCAAACAACT    3920
   [<          lacI          ]

AAACCGGACATGGCACTCCAGTCGCCTTCCCGTTCCGCTATCGGCTGAATTTGATTGCGAGTGAGATATTTATGCCAGCC
+--+-+-+-+--+-+--+-+--+-+--+-+--+-+--+-+--+-+--+-+--+-+--+-+--+-+--+-+--+-+--+-+--+
TTTGGCCTGTACCGTGAGGTCAGCGGAAGGGCAAGGCGATAGCCGACTTAAACTAACGCTCACTCTATAAATACGGTCGG    4000
   [<          lacI          ]
```

Fig 21 - continued on netx page

PspOMI  ApaI  NmeAIII  BstEII

```
AGCCAGACGCAGACGCGCCGAGACAGAACTTAATGGGCCCGCTAACAGCGCGATTTGCTGGTGACCCAATGCGACCAGAT
+++++++++|+++++++++|+++++++++|+++++++++|+++++++++|+++++++++|+++++++++|+++++++++|
TCGGTCTGCGTCTGCGCGGCTCTGTCTTGAATTACCCGGGCGATTGTCGCGCTAAACGACCACTGGGTTACGCTGGTCTA
                            lacI

GCTCCACGCCCAGTCGCGTACCGTCTTCATGGGAGAAAATAATACTGTTGATGGGTGTCTGGTCAGAGACATCAAGAAAT
+++++++++|+++++++++|+++++++++|+++++++++|+++++++++|+++++++++|+++++++++|+++++++++|
CGAGGTGCGGGTCAGCGCATGGCAGAAGTACCCTCTTTTATTATGACAACTACCCACAGACCAGTCTCTGTAGTTCTTTA
                            lacI
```

BcfI*

```
AACGCCGGAACATTAGTGCAGGCAGCTTCCACAGCAATGGCATCCTGGTCATCCAGCGGATAGTTAATGATCAGCCCACT
+++++++++|+++++++++|+++++++++|+++++++++|+++++++++|+++++++++|+++++++++|+++++++++|
TTGCGGCCTTGTAATCACGTCCGTCGAAGGTGTCGTTACCGTAGGACCAGTAGGTCGCCTATCAATTACTAGTCGGGTGA
                            lacI
```

MluI

```
GACGCGTTGCGCGAGAAGATTGTGCACCGCCGCTTTACAGGCTTCGACGCCGCTTCGTTCTACCATCGACACCACCACGC
+++++++++|+++++++++|+++++++++|+++++++++|+++++++++|+++++++++|+++++++++|+++++++++|
CTGCGCAACGCGCTCTTCTAACACGTGGCGGCGAAATGTCCGAAGCTGCGGCGAAGCAAGATGGTAGCTGTGGTGGTGCG
                            lacI

TGGCACCCAGTTGATCGGCGCGAGATTTAATCGCCGCGACAATTTGCGACGGCGCGTGCAGGGCCAGACTGGAGGTGGCA
+++++++++|+++++++++|+++++++++|+++++++++|+++++++++|+++++++++|+++++++++|+++++++++|
ACCGTGGGTCAACTAGCCGCGCTCTAAATTAGCGGCGCTGTTAAACGCTGCCGCGCACGTCCCGGTCTGACCTCCACCGT
                            lacI

ACGCCAATCAGCAACGACTGTTTGCCCGCCAGTTGTTGTGCCACGCGGTTGGGAATGTAATTCAGCTCCGCCATCGCCGC
+++++++++|+++++++++|+++++++++|+++++++++|+++++++++|+++++++++|+++++++++|+++++++++|
TGCGGTTAGTCGTTGCTGACAAACGGGCGGTCAACAACACGGTGCGCCAACCCTTACATTAAGTCGAGGCGGTAGCGGCG
                            lacI

TTCCACTTTTTCCCGCGTTTTCGCAGAAACGTGGCTGGCCTGGTTCACCACGCGGGAAACGGTCTGATAAGAGACACCGG
+++++++++|+++++++++|+++++++++|+++++++++|+++++++++|+++++++++|+++++++++|+++++++++|
AAGGTGAAAAAGGGCGCAAAAGCGTCTTTGCACCGACCGGACCAAGTGGTGCGCCCTTTGCCAGACTATTCTCTGTGGCC
                            lacI

CATACTCTGCGACATCGTATAACGTTACTGGTTTCACATTCACCACCCTGAATTGACTCTCTTCCGGGCGCTATCATGCC
+++++++++|+++++++++|+++++++++|+++++++++|+++++++++|+++++++++|+++++++++|+++++++++|
GTATGAGACGCTGTAGCATATTGCAATGACCAAAGTGTAAGTGGTGGGACTTAACTGAGAGAAGGCCCGCGATAGTACGG
                    lacI

ATACCGCGAAAGGTTTTGCGCCATTCGATGGTGTCCGGGATCTCGACGCTCTCCCTTATGCGACTCCTGCATTAGGAAGC
+++++++++|+++++++++|+++++++++|+++++++++|+++++++++|+++++++++|+++++++++|+++++++++|
TATGGCGCTTTCCAAAACGCGGTAAGCTACCACAGGCCCTAGAGCTGCGAGAGGGAATACGCTGAGGACGTAATCCTTCG
```

SphI

```
AGCCCAGTAGTAGGTTGAGGCCGTTGAGCACCGCCGCCGCAAGGAATGGTGCATGCAAGGAGATGGCGCCCAACAGTCCC
+++++++++|+++++++++|+++++++++|+++++++++|+++++++++|+++++++++|+++++++++|+++++++++|
TCGGGTCATCATCCAACTCCGGCAACTCGTGGCGGCGGCGTTCCTTACCACGTACGTTCCTCTACCGCGGGTTGTCAGGG

CCGGCCACGGGGCCTGCCACCATACCCACGCCGAAACAAGCGCTCATGAGCCCGAAGTGGCGAGCCCGATCTTCCCCATC
+++++++++|+++++++++|+++++++++|+++++++++|+++++++++|+++++++++|+++++++++|+++++++++|
GGCCGGTGCCCCGGACGGTGGTATGGGTGCGGCTTTGTTCGCGAGTACTCGGGCTTCACCGCTCGGGCTAGAAGGGGTAG
```

SgrAI

```
GGTGATGTCGGCGATATAGGCGCCAGCAACCGCACCTGTGGCGCCGGTGATGCCGGCCACGATGCGTCCGGCGTAGAGGA
+++++++++|+++++++++|+++++++++|+++++++++|+++++++++|+++++++++|+++++++++|+++++++++|
CCACTACAGCCGCTATATCCGCGGTCGTTGGCGTGGACACCGCGGCCACTACGGCCGGTGCTACGCAGGCCGCATCTCCT
```

Fig 21 - continued on netx page

45

**Fig. 21**

**Fig. 22**

46

**Fig. 23**

**Fig. 24**

**Fig. 25**

**SEC** Superdex peptide PC

**C18** rgdggr21.DATA - Prostar 325 Absorbance Channel 1 EL07119035

## Peak results:

| Index | Name | Time [Min] | Quantity [% Area] | Height [mAU] | Area [mAU.Min] | Area % [%] |
|-------|---------|------------|-------------------|--------------|----------------|------------|
| 1 | UNKNOWN | 3,46 | 58,66 | 2476,4 | 521,5 | 58,659 |
| 2 | UNKNOWN | 3,72 | 9,67 | 931,3 | 85,9 | 9,666 |
| 3 | UNKNOWN | 17,72 | 25,15 | 1936,9 | 223,6 | 25,152 |
| 4 | UNKNOWN | 23,58 | 6,52 | 601,4 | 58,0 | 6,524 |
| | | | | | | |
| Total | | | 100.00 | 5946,0 | 889,1 | 100,00 |

24

Fig. 27

Fig. 28

Fig. 29

Fig. 30

Fig. 31

Fig. 32

Fig. 33

**Fig. 34**

Fig. 35

Fig. 36

Fig. 37

SEQ. 1
CCATGGATCACCATCACCACCACCACTGGTATTACCCGGAACAGAAACTCATCTCAGAAGAGGATC
TGGTCGACATGCAGATTTTTGTGAAAACCCTGACCGGCAAAACCATTACCCTGGAAGTGGAACCGA
GCGATACCATTGAAAATGTGAAAGCGAAAATTCAGGATAAAGAAGGCATTCCGCCGGATCAGCAG
CGTCTGATTTTTGCGGGCAAACAGCTGGAAGATGGCCGTACCCTGAGCGATTATAATATTCAGAAA
GAAAGCACCCTGCATCTGGTGCTGCGTCTGCGTGGCGGCATCATGAGTCGCTCTTCacccCGTGGTGA
CCGTGGCGATCGTGGTGACCGTGGCGATCGTGGTGACCGTGGCGATCGTGGTGACCGTGGCGATCG
TGGTGACCGTGGCGATcccaGAAGAGCTTGACTCGAG

SEQ. 2
CCATGGATCACCATCACCACCACCACTGGTATTACCCGGAACAGAAACTCATCTCAGAAGAGGATC
TGGTCGACATGCAGATTTTTGTGAAAACCCTGACCGGCAAAACCATTACCCTGGAAGTGGAACCGA
GCGATACCATTGAAAATGTGAAAGCGAAAATTCAGGATAAAGAAGGCATTCCGCCGGATCAGCAG
CGTCTGATTTTTGCGGGCAAACAGCTGGAAGATGGCCGTACCCTGAGCGATTATAATATTCAGAAA
GAAAGCACCCTGCATCTGGTGCTGCGTCTGCGTGGCGGCATCATGAGTCGCTCTTCacccCGTGGTGA
CGGTGGCCGTGGCGATGGTGGCCGTGGTGACGGTGGCCGTGGCGATGGTGGCCGTGGTGACGGTG
GCCGTGGCGATGGTGGCCGTGGTGACGGTGGCCGTGGCGATGGTGGCCGTGGTGACGGTGGCCGT
GGTGATGGTGGCcccaGAAGAGCTTGACTCGAG

SEQ.3
MDHHHHHHWYYPEQKLISEEDLVDMQIFVKTLTGKTITLEVEPSDTIENVKAKIQDKEGIPPDQQRLIFA
GKQLEDGRTLSDYNIQKESTLHLVLRLRGGIMSRSSPRGDRGDRGDRGDRGDRGDRGDRGDRGD
PRRA

SEQ. 4
MDHHHHHHWYYPEQKLISEEDLVDMQIFVKTLTGKTITLEVEPSDTIENVKAKIQDKEGIPPDQQRLIFA
GKQLEDGRTLSDYNIQKESTLHLVLRLRGGIMSRSSPRGDGGRGDGGRGDGGRGDGGRGDGGRGDGG
RGDGGRGDGGRGDGGRGDGGPRRA

**Fig. 38**

(A1) **GFAP**  (A2) **DCX**  (A3) **Hoechst**

(B1) **GFAP**  (B2) **Ki67**  (B3) **Hoechst**

(C1) **SOX2**  (C2) **Ki67**  (C3) **Hoechst**

**Fig. 39**

GFAP  NeuN

Ki67  Kaspaza 3

**Fig. 40**

Fig. 41

Fig. 42

Fig. 43

Fig. 44

Fig. 45

Fig. 46

Fig. 47

Fig. 48

Fig. 49

Fig. 50

Fig. 51

Fig. 52

EP 3 549 947 B1

Fig. 53

Fig. 54

65

**Fig. 55**

**Fig. 56**

**Fig. 57**

**Fig. 58**

**Fig. 59**

**Fig. 60**

Fig. 61

Fig. 62

Fig. 63

Control

Damage
(100 µM NMDA)

Damage+RGD1
(100 µM NMDA
+100 µM RGD1)

Fig. 64

Fig. 65

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- PL 228341 B

**Non-patent literature cited in the description**

- **BREWER GJ ; COTMAN CW.** Survival and growth of hippocampal neurons in defined medium at low density: advantages of a sandwich culture technique or low oxygen. *Brain Res.,* 1989, vol. 494 (1), 65-74
- **BREWER GJ ; TORRICELLI JR ; EVEGE EK ; PRICE PJ.** Optimized survival of hippocampal neurons in B27-supplemented Neurobasal, a new serum-free medium combination. *J Neurosci Res.,* 1993, vol. 35 (5), 567-76
- **MILLER G.** Neuroscience. New guidelines aim to improve studies of traumatic brain injury. *Science,* 2010, vol. 328 (5976), 297
- **CHEN CC ; HUNG TH ; LEE CY ; WANG LF ; WU CH ; KE CH et al.** Berberine protects against neuronal damage via suppression of glia-mediated inflammation in traumatic brain injury. *PloS one.,* 2014, vol. 9 (12), e 115694
- **MILLER DM ; SINGH IN ; WANG JA ; HALL ED.** Nrf2-ARE activator carnosic acid decreases mitochondrial dysfunction, oxidative damage and neuronal cytoskeletal degradation following traumatic brain injury in mice. *Experimental neurology.,* 2015, vol. 264, 103-10
- **RUSSO MV ; MCGAVERN DB.** Inflammatory neuroprotection following traumatic brain injury. *Science,* 2016, vol. 353 (6301), 783-5
- **JACKSON A.** Spinal-cord injury: Neural interfaces take another step forward. *Nature,* 2016, vol. 539 (7628), 177-8
- **OWENS B.** Stroke. *Nature,* 2014, vol. 510 (7506), S1
- **VENKAT P ; SHEN Y ; CHOPP M ; CHEN J.** Cell-based and pharmacological neurorestorative therapies for ischemic stroke. *Neuropharmacology,* 2017
- **WANG Q ; YU S ; SIMONYI A ; SUN GY ; SUN AY.** Kainic acid-mediated excitotoxicity as a model for neurodegeneration. *Molecular neurobiology.,* 2005, vol. 31 (1-3), 3-16

- **MELONI BP ; CRAIG AJ ; MILECH N ; HOPKINS RM ; WATT PM ; KNUCKEY NW.** The neuroprotective efficacy of cell-penetrating peptides TAT, penetratin, Arg-9, and Pep-1 in glutamic acid, kainic acid, and in vitro ischemia injury models using primary cortical neuronal cultures. *Cell Mol Neurobiol.,* 2014, vol. 34 (2), 173-81
- **MELONI BP ; BROOKES LM ; CLARK VW ; CROSS JL ; EDWARDS AB ; ANDERTON RS et al.** Poly-arginine and arginine-rich peptides are neuroprotective in stroke models. *Journal of cerebral blood flow and metabolism : official journal of the International Society of Cerebral Blood Flow and Metabolism.,* 2015, vol. 35 (6), 993-1004
- **PATEL S ; TSANG J ; HARBERS GM ; HEALY KE ; LI S.** Regulation of endothelial cell function by GRGDSP peptide grafted on interpenetrating polymers. *Journal of biomedical materials research Part A.,* 2007, vol. 83 (2), 423-33
- **MIHARDJA SS ; GAO D ; SIEVERS RE ; FANG Q ; FENG J ; WANG J et al.** Targeted in vivo extracellular matrix formation promotes neovascularization in a rodent model of myocardial infarction. *PloS one,* 2010, vol. 5 (4), e10384
- **RUBTSOV MA ; SYRKINA MS ; ALIEV G.** RGD-based Therapy: Principles of Selectivity. *Current pharmaceutical design.,* 2016, vol. 22 (7), 932-52
- **AOTA S ; NOMIZU M ; YAMADA KM.** The short amino acid sequence Pro-His-Ser-Arg-Asn in human fibronectin enhances cell-adhesive function. *The Journal of biological chemistry,* 1994, vol. 269 (40), 24756-61
- **WANG F ; LI Y ; SHEN Y ; WANG A ; WANG S ; XIE T.** The functions and applications of RGD in tumor therapy and tissue engineering. *International journal of molecular sciences.,* 2013, vol. 14 (7), 13447-62
- **ISENBERG WM ; MCEVER RP ; PHILLIPS DR ; SHUMAN MA ; BAINTON DF.** The platelet fibrinogen receptor: an immunogold-surface replica study of agonist-induced ligand binding and receptor clustering. *The Journal of cell biology,* 1987, vol. 104 (6), 1655-63

- **KATOH D ; NAGAHARU K ; SHIMOJO N ; HANAMURA N ; YAMASHITA M ; KOZUKA Y et al.** Binding of alphavbeta1 and alphavbeta6 integrins to tenascin-C induces epithelial-mesenchymal transition-like change of breast cancer cells. *Oncogenesis,* 2013, vol. 2, e65
- **DENHARDT DT ; GIACHELLI CM ; RITTLING SR.** Role of osteopontin in cellular signaling and toxicant injury. *Annual review of pharmacology and toxicology.,* 2001, vol. 41, 723-49
- **STIPP CS.** Laminin-binding integrins and their tetraspanin partners as potential antimetastatic targets. *Expert reviews in molecular medicine,* 2010, vol. 12, e3
- **MASSIA SP ; HUBBELL JA.** An RGD spacing of 440 nm is sufficient for integrin alpha V beta 3-mediated fibroblast spreading and 140 nm for focal contact and stress fiber formation. *The Journal of cell biology,* 1991, vol. 114 (5), 1089-100
- **CLARK EA ; BRUGGE JS.** Integrins and signal transduction pathways: the road take. *Science,* 1995, vol. 268 (5208), 233-9
- **ALBELDA SM ; METTE SA ; ELDER DE ; STEWART R ; DAMJANOVICH L ; HERLYN M et al.** Integrin distribution in malignant melanoma: association of the beta 3 subunit with tumor progression. *Cancer research,* 1990, vol. 50 (20), 6757-64
- **CHAN BM ; MATSUURA N ; TAKADA Y ; ZETTER BR ; HEMLER ME.** In vitro and in vivo consequences of VLA-2 expression on rhabdomyosarcoma cells. *Science,* 1991, vol. 251 (5001), 1600-2
- **FERRIS DM ; MOODIE GD ; DIMOND PM ; GIORANNI CW ; EHRLICH MG ; VALENTINI RF.** RGD-coated titanium implants stimulate increased bone formation in vivo. *Biomaterials,* 1999, vol. 20 (23-24), 2323-31
- **ALKHODARY MA.** Laser micro-grooved, Arginine-Glycine-Apspartic acid (RGD) coated dental implants, a 5 years radiographic follow-up. *International journal of health sciences.,* 2014, vol. 8 (4), 361-9
- **UZUNALLI G ; SORAN Z ; ERKAL TS ; DAGDAS YS ; DINE E ; HONDUR AM et al.** Bioactive self-assembled peptide nanofibers for corneal stroma regeneration. *Acta biomaterialia.,* 2014, vol. 10 (3), 1156-66
- **TWEDEN KS ; HARASAKI H ; JONES M ; BLEVITT JM ; CRAIG WS ; PIERSCHBACHER M et al.** Accelerated healing of cardiovascular textiles promoted by an RGD peptide. *The Journal of heart valve disease,* 1995, vol. 4 (1), 90-7
- **WU XAR, D.** Integrins as receptor targets for neurological disorders. *Pharmacology & Therapeutics.,* 2012, vol. 134 (1), 68-81
- **BECCHETTI A ; ARCANGELI A.** Integrins and ion channels in cell migration: implications for neuronal development, wound healing and metastatic spread. *Adv Exp Med Biol.,* 2010, vol. 674, 107-23
- **BURKIN DJ ; KIM JE ; GU M ; KAUFMAN SJ.** Laminin and alpha7beta1 integrin regulate agrin-induced clustering of acetylcholine receptors. *Journal of cell science,* 2000, vol. 113, 2877-86
- **BERNARD-TRIFILO JA ; KRAMAR EA ; TORP R ; LIN CY ; PINEDA EA ; LYNCH G et al.** Integrin signaling cascades are operational in adult hippocampal synapses and modulate NMDA receptor physiology. *Journal of neurochemistry.,* 2005, vol. 93 (4), 834-49
- **VAN DER LAAN LJ ; VAN DER GOES A ; WAUBEN MH ; RUULS SR ; DOPP EA ; DE GROOT CJ et al.** Beneficial effect of modified peptide inhibitor of alpha4 integrins on experimental allergic encephalomyelitis in Lewis rats. *Journal of neuroscience research.,* 2002, vol. 67 (2), 191-9
- **GLADSON CL ; STEWART JE ; OLMAN MA ; CHANG PL ; SCHNAPP LM ; GRAMMER JR et al.** Attachment of primary neonatal rat astrocytes to vitronectin is mediated by integrins alphavbeta5 and alpha8beta1: modulation by the type 1 plasminogen activator inhibitor. *Neuroscience letters,* 2000, vol. 283 (2), 157-61
- **SILLETTI S ; KESSLER T ; GOLDBERG J ; BOGER DL ; CHERESH DA.** Disruption of matrix metalloproteinase 2 binding to integrin alpha vbeta 3 by an organic molecule inhibits angiogenesis and tumor growth in vivo. *Proceedings of the National Academy of Sciences of the United States of America,* 2001, vol. 98 (1), 119-24
- **AKIYAMA H ; KAWAMATA T ; DEDHAR S ; MCGEER PL.** Immunohistochemical localization of vitronectin, its receptor and beta-3 integrin in Alzheimer brain tissue. *Journal of neuroimmunology.,* 1991, vol. 32 (1), 19-28
- **CLEGG DO ; WINGERD KL ; HIKITA ST ; TOLHURST EC.** Integrins in the development, function and dysfunction of the nervous system. *Frontiers in bioscience : a journal and virtual library.,* 2003, vol. 8, d723-50
- **DITYATEV A ; FELLIN T.** Extracellular matrix in plasticity and epileptogenesis. *Neuron glia biology.,* 2008, vol. 4 (3), 235-47
- **WAGNER S ; TAGAYA M ; KOZIOL JA ; QUARANTA V ; DEL ZOPPO GJ.** Rapid disruption of an astrocyte interaction with the extracellular matrix mediated by integrin alpha 6 beta 4 during focal cerebral ischemia/reperfusion. *Stroke.,* 1997, vol. 28 (4), 858-65
- **RELTON JK ; SLOAN KE ; FREW EM ; WHALLEY ET ; ADAMS SP ; LOBB RR.** Inhibition of alpha4 integrin protects against transient focal cerebral ischemia in normotensive and hypertensive rats. *Stroke.,* 2001, vol. 32 (1), 199-205

- **MABON PJ ; WEAVER LC ; DEKABAN GA.** Inhibition of monocyte/macrophage migration to a spinal cord injury site by an antibody to the integrin alphaD: a potential new antiinflammatory treatment. *Experimental neurology.,* 2000, vol. 166 (1), 52-64
- **WEAVER LC ; BAO F ; DEKABAN GA ; HRYCIW T ; SHULTZ SR ; CAIN DP et al.** CD11d integrin blockade reduces the systemic inflammatory response syndrome after traumatic brain injury in rats. *Experimental neurology.,* 2015, vol. 271, 409-22
- **VOGELEZANG MG ; LIU Z ; RELVAS JB ; RAIVICH G ; SCHERER SS ; FFRENCH-CONSTANT C.** Alpha4 integrin is expressed during peripheral nerve regeneration and enhances neurite outgrowth. *The Journal of neuroscience : the official journal of the Society for Neuroscience.,* 2001, vol. 21 (17), 6732-44
- **WERNER A ; WILLEM M ; JONES LL ; KREUTZBERG GW ; MAYER U ; RAIVICH G.** Impaired axonal regeneration in alpha7 integrin-deficient mice. *The Journal of neuroscience : the official journal of the Society for Neuroscience.,* 2000, vol. 20 (5), 1822-30
- **XIAO T ; TAKAGI J ; COLLER BS ; WANG JH ; SPRINGER TA.** Structural basis for allostery in integrins and binding to fibrinogen-mimetic therapeutics. *Nature,* 2004, vol. 432 (7013), 59-67
- **WANG G.** Post-translational Modifications of Natural Antimicrobial Peptides and Strategies for Peptide Engineering. *Curr Biotechnol.,* 2012, vol. 1 (1), 72-9
- **SOUNDARA MANICKAM D ; BISHT HS ; WAN L ; MAO G ; OUPICKY D.** Influence of TAT-peptide polymerization on properties and transfection activity of TAT/DNA polyplexes. *J Control Release.,* 2005, vol. 102 (1), 293-306
- **MORINI R ; BECCHETTI A.** Integrin receptors and ligand-gated channels. *Advances in experimental medicine and biology.,* 2010, vol. 674, 95-105
- **MAIER M ; SEABROOK TJ ; LAZO ND ; JIANG L ; DAS P ; JANUS C et al.** Short amyloid-beta (Abeta) immunogens reduce cerebral Abeta load and learning deficits in an Alzheimer's disease mouse model in the absence of an Abeta-specific cellular immune response. *The Journal of neuroscience : the official journal of the Society for Neuroscience.,* 2006, vol. 26 (18), 4717-28
- **WU DY ; WANG LC ; MASON CA ; GOLDBERG DJ.** Association of beta 1 integrin with phosphotyrosine in growth cone filopodia. *The Journal of neuroscience : the official journal of the Society for Neuroscience.,* 1996, vol. 16 (4), 1470-8
- **YIP PM ; ZHAO X ; MONTGOMERY AM ; SIU CH.** The Arg-Gly-Asp motif in the cell adhesion molecule L1 promotes neurite outgrowth via interaction with the alphavbeta3 integrin. *Molecular biology of the cell,* 1998, vol. 9 (2), 277-90
- **RAFIUDDIN AHMED M ; JAYAKUMAR R.** Peripheral nerve regeneration in RGD peptide incorporated collagen tubes. *Brain research,* 2003, vol. 993 (1-2), 208-16
- **WOERLY S ; PINET E ; DE ROBERTIS L ; VAN DIEP D ; BOUSMINA M.** Spinal cord repair with PHPMA hydrogel containing RGD peptides (NeuroGel). *Biomaterials,* 2001, vol. 22 (10), 1095-111
- **YANG H ; YANG H ; LU Y ; MA S ; LIU Y ; JIA G et al.** Targeted delivery of extracellular matrix protected against neurologic defects after focal ischemia reperfusion in rats. *Journal of stroke and cerebrovascular diseases : the official journal of National Stroke Association.,* 2015, vol. 24 (1), 154-62
- **ANANTHANARAYANAN B ; LITTLE L ; SCHAFFER DV ; HEALY KE ; TIRRELL M.** Neural stem cell adhesion and proliferation on phospholipid bilayers functionalized with RGD peptides. *Biomaterials,* 2010, vol. 31 (33), 8706-15
- **YANG K ; LEE JS ; KIM J ; LEE YB ; SHIN H ; UM SH et al.** Poly dopamine-mediated surface modification of scaffold materials for human neural stem cell engineering. *Biomaterials,* 2012, vol. 33 (29), 6952-64
- **CIPRIANI R ; CHARA JC ; RODRIGUEZ-ANTIGUEDAD A ; MATUTE C.** FTY720 attenuates excitotoxicity and neuroinflammation. *Journal of neuroinflammation,* 2015, vol. 12, 86
- **MELONI BP ; MAJDA BT ; KNUCKEY NW.** Establishment of neuronal in vitro models of ischemia in 96-well microtiter strip-plates that result in acute, progressive and delayed neuronal death. *Neuroscience.,* 2001, vol. 108 (1), 17-26
- **LIN CY ; HILGENBERG LG ; SMITH MA ; LYNCH G ; GALL CM.** Integrin regulation of cytoplasmic calcium in excitatory neurons depends upon glutamate receptors and release from intracellular stores. *Molecular and cellular neurosciences.,* 2008, vol. 37 (4), 770-80
- **WATSON PM ; HUMPHRIES MJ ; RELTON J ; ROTHWELL NJ ; VERKHRATSKY A ; GIBSON RM.** Integrin-binding RGD peptides induce rapid intracellular calcium increases and MAPK signaling in cortical neurons. *Molecular and cellular neurosciences.,* 2007, vol. 34 (2), 147-54
- **MILNER R ; HUNG S ; WANG X ; BERG GI ; SPATZ M ; DEL ZOPPO GJ.** Responses of endothelial cell and astrocyte matrix-integrin receptors to ischemia mimic those observed in the neurovascular unit. *Stroke.,* 2008, vol. 39 (1), 191-7
- **CHEN FY ; LEE TJ.** Arginine synthesis from citrulline in perivascular nerves of cerebral artery. *J Pharmacol Exp Ther.,* 1995, vol. 273 (2), 895-901
- **DE HEMPTINNE A ; MARRANNES R ; VANHEEL B.** Influence of organic acids on intracellular pH. *The American journal of physiology,* 1983, vol. 245 (3), C178-83

- **GOLDMAN SA ; PULSINELLI WA ; CLARKE WY ; KRAIG RP ; PLUM F.** The effects of extracellular acidosis on neurons and glia in vitro. *Journal of cerebral blood flow and metabolism : official journal of the International Society of Cerebral Blood Flow and Metabolism.,* 1989, vol. 9 (4), 471-7
- **BAUMANN F ; LEUKEL P ; DOERFELT A ; BEIER CP ; DETTMER K ; OEFNER PJ et al.** Lactate promotes glioma migration by TGF-beta2-dependent regulation of matrix metalloproteinase-2. *Neuro-oncology.,* 2009, vol. 11 (4), 368-80
- **LI X ; YU X ; DAI D ; SONG X ; XU W.** The altered glucose metabolism in tumor and a tumor acidic microenvironment associated with extracellular matrix metalloproteinase inducer and monocarboxylate transporters. *Oncotarget.,* 2016, vol. 7 (17), 23141-55
- **SAITO A ; HAYASHI T ; OKUNO S ; NISHI T ; CHAN PH.** Oxidative stress affects the integrin-linked kinase signaling pathway after transient focal cerebral ischemia. *Stroke.,* 2004, vol. 35 (11), 2560-5
- **GARY DS ; MATTSON MP.** Integrin signaling via the PI3-kinase-Akt pathway increases neuronal resistance to glutamate-induced apoptosis. *Journal of neurochemistry.,* 2001, vol. 76 (5), 1485-96
- **SAAVEDRA A ; BALTAZAR G ; CARVALHO CM ; DUARTE EP.** GDNF modulates HO-1 expression in substantia nigra postnatal cell cultures. *Free radical biology & medicine.,* 2005, vol. 39 (12), 1611-9
- **SHI Y ; ETHELL IM.** Integrins control dendritic spine plasticity in hippocampal neurons through NMDA receptor and Ca2+/calmodulin-dependent protein kinase II-mediated actin reorganization. *The Journal of neuroscience : the official journal of the Society for Neuroscience.,* 2006, vol. 26 (6), 1813-22
- **WANG Q ; YU S ; SIMONYI A ; SUN GY ; SUN AY.** Kainic acid-mediated excitotoxicity as a model for neurodegeneration. *Molecular neurobiology,* 2005, vol. 31 (1-3), 3-16
- **MELONI BP ; CRAIG AJ ; MILECH N ; HOPKINS RM ; WATT PM ; KNUCKEY NW.** The neuroprotective efficacy of cell-penetrating peptides TAT, penetratin, Arg-9, and Pep-1 in glutamic acid, kainic acid, and in vitro ischemia injury models using primary cortical neuronal cultures. *Cellular and molecular neurobiology.,* 2014, vol. 34 (2), 173-81
- **CIPRIANI R ; CHARA JC ; RODRIGUEZ-ANTIGUEDAD A ; MATUTE C.** FTY720 attenuates excitotoxicity and neuroinflammation. *Journal of neuroinflammation,* 2015, vol. 12, 86